(19) **Europäisches Patentamt / European Patent Office / Office européen des brevets**

(11) **EP 4 293 038 A1**

(12) **EUROPEAN PATENT APPLICATION**
published in accordance with Art. 153(4) EPC

(43) Date of publication:
**20.12.2023 Bulletin 2023/51**

(21) Application number: **22752191.1**

(22) Date of filing: **29.01.2022**

(51) International Patent Classification (IPC):
**C07K 14/05** (2006.01)  **C07K 16/08** (2006.01)
**A61K 39/395** (2006.01)  **A61K 39/245** (2006.01)

(52) Cooperative Patent Classification (CPC):
**A61K 39/245; A61K 39/395; A61K 39/42;
A61P 31/22; A61P 35/00; C07K 14/03;
C07K 14/05; C07K 16/00; C07K 16/08;
C07K 19/00; C12N 15/62; G01N 33/569;
G01N 33/577**

(86) International application number:
**PCT/CN2022/074985**

(87) International publication number:
**WO 2022/171030 (18.08.2022 Gazette 2022/33)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **10.02.2021 CN 202110182104**

(71) Applicants:
• **Xiamen University
Xiamen, Fujian 361005 (CN)**
• **Sun Yat-Sen University Cancer Center
(Sun Yat-Sen University Cancer Hospital,
Sun Yat-Sen University Cancer Institute)
Guangzhou, Guangdong 510060 (CN)**
• **Sun Yat-Sen University
Guangzhou, Guangdong 510275 (CN)**

(72) Inventors:
• **CHEN, Yixin
Xiamen, Fujian 361005 (CN)**

• **ZHANG, Xiao
Guangzhou, Guangdong 510060 (CN)**
• **HONG, Junping
Xiamen, Fujian 361005 (CN)**
• **XU, Miao
Guangzhou, Guangdong 510060 (CN)**
• **WU, Qian
Xiamen, Fujian 361005 (CN)**
• **ZHONG, Ling
Guangzhou, Guangdong 510060 (CN)**
• **XIA, Ningshao
Xiamen, Fujian 361005 (CN)**

(74) Representative: **Lavoix
Bayerstraße 83
80335 München (DE)**

Remarks:
The complete document including Reference
Table(s) and the Sequence Listing(s) can be
downloaded from the EPO website

(54) **EPITOPE PEPTIDE AND ANTIBODY FOR PREVENTING AND TREATING EB VIRUS INFECTION AND RELATED DISEASES**

(57) Provided are an epitope peptide (or a variant thereof) that can be used for preventing or treating an EBV infection, a recombinant protein containing the epitope peptide (or variant thereof) and a carrier protein, and the use of the epitope peptide (or variant thereof) and the recombinant protein. Further provided are an antibody against the epitope peptide, and the use thereof in the detection, prevention and/or treatment of an EBV infection and/or diseases caused by the infection.

EP 4 293 038 A1

**FIG. 3**

**Description**

**Technical Field**

The present invention relates to the fields of immunology and molecular virology, especially the field of prevention and treatment of EBV. Specifically, the present invention relates to an epitope peptide (or variant thereof) that can be used to prevent or treat an EBV infection, a recombinant protein comprising the epitope peptide (or variant thereof) and a carrier protein, and use of the epitope peptide (or variant thereof) and recombinant protein. The present invention also relates to an antibody against the epitope peptide, and use thereof for detection, prevention and/or treatment of an EBV infection and/or a disease caused by the infection.

**Background Art**

Epstein-Barr virus (EBV), a member of the γ-herpesvirus subfamily, is associated with a lymphoproliferative disorder (LPD) in an immunocompromised host and associated with many different types of human B cell malignancies and epithelial cells malignancies. In immunocompetent people, most patients with EBV infection have no clinical symptoms or only subclinical manifestations, but in immunocompromised patients, there may be higher morbidity and mortality. At present, the treatment methods for EBV infection are very limited, mainly including therapy with CD20 monoclonal antibody or therapy with chemical drugs. However, the therapy with CD20 monoclonal antibody not only can kill EBV-infected B cells, but also lead to the death of normal B lymphocytes, which further weaken the patients' immune function. Conventional therapy with chemical drugs may lead to a decline in the overall immune function of patients and cause other serious side effects.

Therefore, it is urgent and necessary to develop innovative and more effective methods and drugs for the treatment of EBV infection.

**Contents of the present invention**

Although there may be multiple B cell response (antibody response) epitopes on various proteins of Epstein-Barr virus, not all antibody against any epitope can be applied to the treatment of EBV infection. Therefore, the key to the development of immunotherapeutic drugs/methods that can effectively treat EBV infection is to identify targets (epitopes) that can induce antibody responses for effectively eliminating the virus and virus-infected cells in the body, and to obtain antibody against the targets (epitopes).

The present invention identifies such a target (epitope), and based on this, provides an epitope peptide (or variant thereof) that can be used to treat an EBV infection, a recombinant protein comprising the epitope peptide (or variant thereof) and a carrier protein, and a use of the epitope peptide (or variant thereof) and recombinant protein. The present invention also provides a monoclonal antibody with excellent properties against such epitope peptide/epitope, which can efficiently neutralize EBV and block or inhibit the fusion of EBV with a cell. The present invention thus provides the following aspects.

Epitope peptide recognized by monoclonal antibody 3A3 and derivative antibody thereof

Epitope peptide

In a first aspect, the present invention provides an isolated epitope peptide or variant thereof, wherein the epitope peptide comprises an epitope located within the amino acid residues at the positions 341-362 of EBV gB protein, and the epitope comprises at least the amino acid residues at the positions 352, 356 and 360 of EBV gB protein;
the variant differs from the epitope peptide from which it is derived only by a mutation (e.g., substitution, addition or deletion) of one or several (e.g., 1, 2 or 3) amino acid residues, and does not comprise a mutation at positions corresponding to the amino acid positions 352, 356 and 360 of EBV gB protein, and retains a biological function of the epitope peptide from which it is derived.

The biological function of the epitope peptide described in the first aspect of the present invention includes but is not limited to one or more selected from the following:

1) specifically binding to monoclonal antibody 3A3 (e.g., that has heavy chain CDRs set forth in SEQ ID NOs: 3-5 and light chain CDRs set forth in SEQ ID NOs: 6-8);

2) an ability to induce in a subject an antiserum capable of neutralizing EBV, and/or blocking or inhibiting the fusion of EBV with a cell (optionally, after the fusion of the epitope peptide with a carrier protein);

3) an ability to induce an antibody response for effectively clearing EBV and an EBV-infected cell in vivo (optionally, after the fusion of the epitope peptide with a carrier protein); and

4) an ability to prevent and/or treat an EBV infection or a disease associated with EBV infection in a subject (optionally, after the fusion of the epitope peptide with a carrier protein).

In certain embodiments, the EBV is an M81 strain (e.g., Genbank ID: KF373730.1). In certain embodiments, the EBV gB protein has a sequence set forth in SEQ ID NO: 17.

In certain embodiments, the amino acid residues at positions 341-362 of the EBV gB protein are set forth in SEQ ID NO: 18.

In certain embodiments, the epitope is a linear epitope, which consists of at least 3, at least 4, at least 5, at least 6, at least 7, at least 8, at least 9, at least 10, at least 15 or at least 20 consecutive amino acid residues located within the amino acid residues at the positions 341-362 of EBV gB protein.

In certain embodiments, the linear epitope consists of amino acid residues at the positions 341-362 of EBV gB protein.

In certain embodiments, the epitope peptide consists of 5-50 (e.g., 10-50, 10-40, 20-40; for example 20-25 or 35-40; for example 20, 21, 22, 23, 24, 25, 26, 27, 28, 30, 31, 32, 33, 34, 35, 36, 37, 38, 39, or 40) consecutive amino acid residues of the EBV gB protein, and comprises the linear epitope.

In certain embodiments, the epitope peptide consists of amino acid residues at the positions 341-362 or the amino acid residues at the positions 331-367 of EBV gB protein.

In certain embodiments, the epitope peptide consists of the sequence set forth in SEQ ID NO: 18 or 19.

In certain embodiments, the epitope is a conformational epitope, which consists of at least 3, at least 4, at least 5, at least 6, at least 7, at least 8, at least 9, at least 10, at least 15 or at least 20 non-consecutive amino acid residues of EBV gB protein, and comprises at least the amino acid residues at the positions 352, 356 and 360 of EBV gB protein.

In certain embodiments, the epitope peptide consists of 5-50 (e.g., 10-50, 10-40, 20-40; for example 20-25 or 35-40; for example 20, 21, 22, 23, 24, 25, 26, 27, 28, 30, 31, 32, 33, 34, 35, 36, 37, 38, 39, or 40) consecutive amino acid residues of EBV gB protein, and comprises the conformational epitope.

In particular, the epitope peptide or variant thereof of the present invention can be fused with a carrier protein to enhance the immunogenicity of the epitope peptide or variant thereof, so that the epitope peptide or variant thereof can be recognized by the body's immune system, and can induce an antibody response that effectively clears the virus and virus-infected cells in the body.

Therefore, in the second aspect, the present invention also provides a recombinant protein, which comprises the isolated epitope peptide or variant thereof described in the first aspect, and a carrier protein, and the recombinant protein is not a naturally occurring protein or fragment thereof. In the recombinant protein, the epitope peptide or variant thereof can be ligated to the N-terminal or C-terminal of the carrier protein, inserted into the interior of the carrier protein, or replace a part of the amino acid sequence of the carrier protein, depending on the specific carrier protein used. In addition, optionally, the epitope peptide or variant thereof is connected to the carrier protein via a linker. The recombinant protein of the present invention is not limited by its production method, for example, it can be produced by genetic engineering method (recombinant technology), or by chemical synthesis method.

In certain embodiments, the recombinant protein is capable of displaying the isolated epitope peptide or variant thereof described in the first aspect, and the epitope peptide or variant thereof can be specifically bound by a monoclonal antibody 3A3 or derivative antibody thereof.

In certain embodiments, the carrier protein is selected from HBcAg or fragment thereof, for example, one or more amino acid residues (e.g., amino acids at the positions 79-81) of HBcAg are substituted with the epitope peptide of the present invention. In certain embodiments, the epitope peptide is connected to HBcAg or fragment thereof via a linker (e.g., a glycine-rich flexible linker). In certain embodiments, the fragment of HBcAg comprises aa 1-149 of HBcAg or consists of aa 1-149 of HBcAg.

When HBcAg is used as the carrier protein, a full-length HBcAg protein or fragment thereof (e.g., aa 1-149 at the N-terminal of HBcAg protein) can be used (see, Yang Haijie et al., Construction of Peptide Display Vector Based on HBV Core Protein, Journal of Xiamen University (Natural Science) 2004.05 Vol. 43.No. 4). For example, when a fragment (aa 1-149) of HBcAg protein is used as the carrier protein, the sequence encoding HBcAg aa79-81 in the nucleotide sequence encoding the HBcAg protein fragment (aa 1-149) can be deleted, and a linker can be introduced at each of the two ends of the deletion, and a BamH I/EcoR I restriction site can be designed between the two linkers, thereby obtaining the coding sequence of the carrier protein HBc149/mut (the amino acid sequence of HBc149/mut can be shown as SEQ ID NO: 21), and the coding sequence is cloned into an expression vector. Afterwards, using the BamH I/EcoR I restriction site, the coding sequence of the epitope peptide of interest is cloned between the linkers, thereby obtaining the HBc149 carrier protein chimerized with the epitope peptide.

In some embodiments, the recombinant protein comprises the sequence set forth in SEQ ID NO: 22.

In the third aspect, the present invention also provides an isolated nucleic acid molecule, which comprises a nucleotide sequence encoding the epitope peptide or variant thereof of the present invention or the recombinant protein of the present invention. In a fourth aspect, the present invention also provides a vector, which comprises the isolated nucleic acid molecule as described above. The vector of the present invention can be a cloning vector or an expression vector. In a preferred embodiment, the vector of the present invention is, for example, plasmid, cosmid, phage, cosmid and the like.

In a fifth aspect, it is also provided a host cell comprising the isolated nucleic acid molecule or vector of the present invention. Such host cells include, but are not limited to, prokaryotic cell such as *E. coli* cell, and eukaryotic cell such as yeast cell, insect cell, plant cell, and animal cell (e.g., mammalian cell, such as mouse cell, human cell, etc.). The cell of the present invention may also be a cell line, such as 293T cell. In certain embodiments, the host cell is a microorganism.

In another aspect, it is also provided a method for preparing the recombinant protein of the present invention, which comprises culturing the host cell of the present invention under suitable conditions, and recovering the recombinant protein of the present invention from the cell culture.

Display platform

The epitope peptide or variant thereof according to the first aspect of the present invention can be displayed on a particle, such as liposome, virus-like particle (VLP), nanoparticle, virosome or exosome to enhance the efficacy of antigen presentation in vivo.

Therefore, in a sixth aspect, the present invention provides a particle, on which surface the epitope peptide or variant thereof of the first aspect is displayed. In certain embodiments, the particle is a virus-like particle (VLP). In certain embodiments, the epitope peptide or variant thereof according to the first aspect of the present invention is fused to and/or displayed on a virus-like particle (VLP).

Antibody

In the seventh aspect, the present invention provides an antibody or antigen-binding fragment thereof, which specifically binds the epitope peptide or variant thereof as described in the first aspect. The antibody or antigen-binding fragment thereof has one or more of the following characteristics: (a) neutralizing EBV in vitro or in a subject (e.g., a human); (b) blocking or inhibiting EBV from fusing to a cell in vitro or in a subject (e.g., a human); (c) preventing and/or treating EBV infection or a disease associated with EBV infection; (d) specifically binding to a 70KDa subunit of EBV gB protein.

In certain embodiments, the antibody or antigen-binding fragment thereof comprises:

(a) a heavy chain variable region (VH) comprising the following 3 complementarity determining regions (CDRs):

(i) a VH CDR1, which consists of the following sequence: SEQ ID NO: 3, or a sequence having a substitution, deletion or addition of one or several amino acids (e.g., a substitution, deletion or addition of 1, 2 or 3 amino acids) as compared thereto,

(ii) a VH CDR2, which consists of the following sequence: SEQ ID NO: 4, or a sequence having a substitution, deletion or addition of one or several amino acids (e.g., a substitution, deletion or addition of 1, 2 or 3 amino acids) as compared thereto, and

(iii) a VH CDR3, which consists of the following sequence: SEQ ID NO: 5, or a sequence having a substitution, deletion or addition of one or several amino acids (e.g., a substitution, deletion or addition of 1, 2 or 3 amino acids) as compared thereto;
and/or,

(b) a light chain variable region (VL) comprising the following 3 complementarity determining regions (CDRs):

(iv) a VL CDR1, which consists of the following sequence: SEQ ID NO: 6, or a sequence having a substitution, deletion or addition of one or several amino acids (e.g., a substitution, deletion or addition of 1, 2 or 3 amino acids) as compared thereto,

(v) a VL CDR2, which consists of the following sequence: SEQ ID NO: 7, or a sequence having a substitution, deletion or addition of one or several amino acids (e.g., a substitution, deletion or addition of 1, 2 or 3 amino acids) as compared thereto, and

(vi) a VL CDR3, which consists of the following sequence: SEQ ID NO: 8, or a sequence having a substitution, deletion or addition of one or several amino acids (e.g., a substitution, deletion or addition of 1, 2 or 3 amino acids) as compared thereto.

In certain embodiments, the CDR described in any one of (i) to (vi) is defined according to the IMGT numbering system.

In certain embodiments, the substitution described in any one of (i) to (vi) is a conservative substitution.

In certain embodiments, the antibody or antigen-binding fragment thereof comprises:

the following 3 heavy chain CDRs: a VH CDR1 having a sequence as set forth in SEQ ID NO: 3, a VH CDR2 having a sequence as set forth in SEQ ID NO: 4, a VH CDR3 having a sequence as set forth in SEQ ID NO: 5; and/or,

the following 3 light chain CDRs: a VL CDR1 having a sequence as set forth in SEQ ID NO: 6, a VL CDR2 having a sequence as set forth in SEQ ID NO: 7, and a VL CDR3 having a sequence as set forth in SEQ ID NO: 8.

In certain embodiments, the antibody or antigen-binding fragment thereof comprises:

the 3 CDRs comprised in the heavy chain variable region (VH) as set forth in SEQ ID NO: 1; and/or,

the 3 CDRs comprised in the light chain variable region (VL) as set forth in SEQ ID NO:2.

In certain implementations, the 3 CDRs comprised in the VH and/or the 3 CDRs comprised in the VL are defined by the Kabat, IMGT or Chothia numbering system.

In certain embodiments, the antibody or antigen-binding fragment thereof comprises:

(a) a heavy chain variable region (VH), which comprises an amino acid sequence selected from the group consisting of:

(i) a sequence set forth in SEQ ID NO: 1;

(ii) a sequence having a substitution, deletion or addition of one or several amino acids (e.g., a substitution, deletion or addition of 1, 2, 3, 4 or 5 amino acids) as compared to the sequence as set forth in SEQ ID NO: 1; or

(iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence as set forth in SEQ ID NO: 1; and

(b) a light chain variable region (VL), which comprises an amino acid sequence selected from the group consisting of:

(iv) a sequence as set forth in SEQ ID NO: 2;

(v) a sequence having a substitution, deletion or addition of one or several amino acids (e.g., a substitution, deletion or addition of 1, 2, 3, 4 or 5 amino acids) as compared to the sequence as set forth in SEQ ID NO: 2; or

(vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence as set forth in SEQ ID NO: 2.

In certain embodiments, the substitution described in (ii) or (v) is a conservative substitution.

In certain embodiments, the antibody or antigen-binding fragment thereof comprises: a VH comprising the sequence set forth in SEQ ID NO: 1 and a VL comprising the sequence set forth in SEQ ID NO: 2.

In certain embodiments, the antibody or antigen-binding fragment thereof is humanized.

In certain embodiments, the antibody or antigen-binding fragment thereof comprises a framework region sequence derived from a human immunoglobulin.

In certain embodiments, the antibody or antigen-binding fragment thereof comprises: a heavy chain framework region sequence derived from a human heavy chain germline sequence, and a light chain framework region sequence derived from a human light chain germline sequence.

In certain embodiments, the antibody or antigen-binding fragment thereof further comprises a constant region derived from a rabbit or human immunoglobulin.

In certain embodiments, the heavy chain of the antibody or antigen-binding fragment thereof comprises a heavy chain constant region derived from a human immunoglobulin (e.g., IgG1, IgG2, IgG3, or IgG4), and the light chain of the antibody or antigen-binding fragment thereof comprises a light chain constant region derived from a human immunoglobulin (e.g., κ or λ).

In certain embodiments, the heavy chain of the antibody or antigen-binding fragment thereof comprises a heavy chain constant region as set forth in SEQ ID NO: 30, and the light chain of the antibody or antigen-binding fragment thereof comprises a light chain constant region as set forth in SEQ ID NO: 31.

In certain embodiments, the antigen-binding fragment is selected from the group consisting of Fab, Fab', (Fab')$_2$, Fv, disulfide-linked Fv, scFv, diabody, and single domain antibody (sdAb); and/or, the antibody is a rabbit-derived antibody, a chimeric antibody, a humanized antibody, a bispecific antibody or a multispecific antibody.

The antibody of the present invention is not limited by its production method, for example, it can be produced by a genetic engineering method (recombinant technology), or by a chemical synthesis method. The antigen-binding fragment of the present invention can be obtained by hydrolyzing an intact antibody molecule (see, Morimoto et al., J. Biochem. Biophys. Methods 24:107-117 (1992), and Brennan et al., Science 229:81 (1985)). Additionally, these antigen-binding fragments can also be produced directly by recombinant host cells (reviewed in Hudson, Curr. Opin. Immunol. 11: 548-557 (1999); Little et al., Immunol. Today, 21: 364-370 (2000)). For example, Fab' fragments can be obtained directly from host cells; Fab' fragments can be chemically coupled to form F(ab')$_2$ fragments (Carter et al., Bio/Technology, 10: 163-167 (1992)). In addition, Fv, Fab or F(ab')$_2$ fragment can also be directly isolated from a culture medium of recombinant host cells. Other techniques for preparing these antigen-binding fragments are well known to those of ordinary skill in the art.

Accordingly, in an eighth aspect, the present invention provides an isolated nucleic acid molecule, which comprises a nucleotide sequence encoding the antibody or antigen-binding fragment thereof of the present invention, or a heavy chain variable region and/or a light chain variable region thereof. In certain embodiments, the isolated nucleic acid molecule encodes the antibody or antigen-binding fragment thereof of the present invention, or a heavy chain variable region and/or a light chain variable region thereof.

In a ninth aspect, the present invention provides a vector (e.g., a cloning vector or an expression vector), which comprises the isolated nucleic acid molecule as described above. In certain embodiments, the vector of the present invention is, for example, plasmid, cosmid, phage, and the like.

In certain embodiments, the vector comprises a first nucleotide sequence encoding a heavy chain variable region of the antibody or antigen-binding fragment thereof of the present invention, and/or a second nucleotide sequence encoding a light chain variable region of the antibody or antigen-binding fragment thereof of the present invention, wherein the first nucleotide sequence and the second nucleotide sequence are provided on the same or different vectors.

In certain embodiments, the vector comprises a first nucleotide sequence encoding a heavy chain of the antibody or antigen-binding fragment thereof of the present invention, and/or a second nucleotide sequence encoding a light chain of the antibody or antigen-binding fragment thereof of the present invention; wherein the first nucleotide sequence and the second nucleotide sequence are provided on the same or different vectors.

In a tenth aspect, the present invention provides a host cell, which comprises the isolated nucleic acid molecule or the vector as described above. Such host cells include, but are not limited to, prokaryotic cell such as *E. coli* cells, and eukaryotic cell such as yeast cell, insect cell, plant cell, and animal cell (e.g., mammalian cell, such as mouse cell, human cell, etc.). In certain preferred embodiments, the host cell of the present invention is a mammalian cell, such as a CHO (e.g., CHO-K1, CHO-S, CHO DG44). In certain embodiments, the host cell is a microorganism.

In another aspect, there is provided a method for producing the antibody or antigen-binding fragment thereof of the present invention, which comprises culturing the host cell as described above under conditions that allow the expression of the antibody or antigen-binding fragment thereof, and recovering the antibody or antigen-binding fragment thereof from a cell culture of the host cell.

Conjugate

The antibody or antigen-binding fragment thereof of the present invention can be derivatized, for example, linked to another molecule (e.g., another polypeptide or protein). Typically, the derivatization (e.g., labeling) of the antibody or antigen-binding fragment thereof will not adversely affect its binding to EBV. Accordingly, the antibody or antigen-binding fragment thereof of the present invention is also intended to include such derivatized forms. For example, the antibody or antigen-binding fragment thereof of the present invention can be functionally linked (by chemical coupling, genetic fusion, non-covalent linkage, or other way) to one or more other molecular moieties, such as another antibody (e.g., forming a bispecific antibody), detection reagent, pharmaceutical reagent, and/or protein or polypeptide (e.g., avidin or polyhistidine tag) capable of mediating the binding of the antibody or antigen-binding fragment to another molecule. In addition, the antibody or antigen-binding fragment thereof of the present invention may also be derivatized with a chemical group, such as polyethylene glycol (PEG), methyl or ethyl, or saccharide group. These groups can be used to improve the biological properties of antibody, such as increasing serum half-life thereof.

Therefore, in the eleventh aspect, the present invention also provides a conjugate, which comprises the antibody or antigen-binding fragment thereof of the present invention and a detectable label linked thereto.

In certain embodiments, the detectable label can be adapted for use in immunological detection (e.g., enzyme-linked immunoassay, radioimmunoassay, fluorescence immunoassay, chemiluminescence immunoassay, etc.). In certain embodiments, the detectable label may be selected from the group consisting of enzyme (e.g., horseradish peroxidase, alkaline phosphatase, or β-galactosidase), chemiluminescent reagent (e.g., acridinium ester, Luminol and derivative thereof, or ruthenium derivative), fluorescent dye (e.g., fluorescein or fluorescent protein such as FITC, TRITC, or PE), radionuclide, or biotin.

In certain embodiments, the detectable label as described above can be linked to the antibody or antigen-binding fragment thereof of the present invention via a linker of various lengths to reduce potential steric hindrance.

Epitope peptide recognized by monoclonal antibody 3A5 and derivative antibody thereof

Epitope peptide

In a twelfth aspect, the present invention provides an isolated epitope peptide or variant thereof, the epitope peptide comprises an epitope located within the amino acid residues at positions 528-616 of the EBV gB protein, and the epitope comprises at least the amino acid residues at positions 540, 567, 610 and 613 of the EBV gB protein;

the variant differs from the epitope peptide from which it is derived only by a mutation (e.g., substitution, addition or deletion) of one or several (e.g., 1, 2 or 3) amino acid residues, and does not comprise a mutation at positions corresponding to the amino acid positions 540, 567, 610 and 613 of EBV gB protein, and retains a biological function of the epitope peptide from which it is derived.

The biological function of the epitope peptide described in the twelfth aspect of the present invention includes but is not limited to one or more selected from the following:

1) specifically binding to monoclonal antibody 3A5 (e.g., that has heavy chain CDRs set forth in SEQ ID NOs: 11-13 and light chain CDRs set forth in SEQ ID NOs: 14-16);

2) an ability to induce in a subject an antiserum capable of neutralizing EBV, and/or blocking or inhibiting the fusion of EBV with a cell (optionally, after the fusion of the epitope peptide with a carrier protein);

3) an ability to induce an antibody response for effectively clearing EBV and an EBV-infected cell in vivo (optionally, after the fusion of the epitope peptide with a carrier protein); and

4) an ability to prevent and/or treat an EBV infection or a disease associated with EBV infection in a subject (optionally, after the fusion of the epitope peptide with a carrier protein).

In certain embodiments, the EBV is an M81 strain (e.g., Genbank ID: KF373730.1). In certain embodiments, the EBV gB protein has a sequence set forth in SEQ ID NO: 17.

In certain embodiments, the amino acid residues at positions 528-616 of EBV gB protein are set forth in SEQ ID NO: 20.

In certain embodiments, the epitope is a linear epitope, which consists of at least 3, at least 4, at least 5, at least 6, at least 7, at least 8, at least 9, at least 10, at least 15 or at least 20 consecutive amino acid residues located within the amino acid residues at the positions 528-616 of EBV gB protein.

In certain embodiments, the linear epitope consists of the amino acid residues at positions 528-616 of EBV gB protein or a fragment thereof.

In some embodiments, the epitope peptide consists of 5-100 (e.g., 10-100, 10-90, 20-90; for example, 5-10, 10-20, 20 -30, 30-40, 40-50, 50-60, 60-70, 70-80, 80-90) consecutive amino acid residues of EBV gB protein, and comprises the linear epitope.

In certain embodiments, the epitope peptide consists of the amino acid residues at positions 528-616 of EBV gB protein or a fragment thereof.

In certain embodiments, the epitope peptide consists of the sequence set forth in SEQ ID NO: 20 or a fragment thereof.

In certain embodiments, the epitope is a conformational epitope, which consists of at least 3, at least 4, at least 5, at least 6, at least 7, at least 8, at least 9, at least 10, at least 15 or at least 20 non-consecutive amino acid residues located within the amino acid residues at positions 528-616 of EBV gB protein, and comprises at least the amino acid residues at positions 540, 567, 610 and 613 of EBV gB protein.

In some embodiments, the epitope peptide consists of 5-100 (e.g., 10-100, 10-90, 20-90; for example, 5-10, 10-20, 20-30, 30-40, 40-50, 50-60, 60-70, 70-80, 80-90) consecutive amino acid residues of EBV gB protein, and comprises the conformational epitope.

In particular, the epitope peptide or variant thereof of the present invention can be fused with a carrier protein to enhance the immunogenicity of the epitope peptide or variant thereof, so that the epitope peptide or variant thereof can be recognized by the body's immune system, and can induce an antibody response that effectively clears the virus and virus-infected cells in the body.

Therefore, in the thirteenth aspect, the present invention also provides a recombinant protein, which comprises the isolated epitope peptide or variant thereof described in the twelfth aspect, and a carrier protein, and the recombinant protein is not a naturally occurring protein or fragment thereof. In the recombinant protein, the epitope peptide or variant thereof can be ligated to the N-terminal or C-terminal of the carrier protein, inserted into the interior of the carrier protein, or replace a part of the amino acid sequence of the carrier protein, depending on the specific carrier protein used. In addition, optionally, the epitope peptide or variant thereof is connected to the carrier protein via a linker. The recombinant protein of the present invention is not limited by its production method, for example, it can be produced by genetic engineering method (recombinant technology), or by chemical synthesis method.

In certain embodiments, the recombinant protein is capable of displaying the isolated epitope peptide or variant thereof described in the twelfth aspect, and the epitope peptide or variant thereof can be specifically bound by a monoclonal antibody 3A5 or derivative antibody thereof.

In certain embodiments, the carrier protein is selected from HBcAg or fragment thereof, for example, one or more amino acid residues (e.g., amino acids at the positions 79-81) of HBcAg are substituted with the epitope peptide of the present invention. In certain embodiments, the epitope peptide is connected to HBcAg or fragment thereof via a linker (e.g., a glycine-rich flexible linker). In certain embodiments, the fragment of HBcAg comprises aa 1-149 of HBcAg or consists of aa 1-149 of HBcAg.

When HBcAg is used as the carrier protein, a full-length HBcAg protein or fragment thereof (e.g., aa 1-149 at the N-terminal of HBcAg protein) can be used (see, Yang Haijie et al., Construction of Peptide Display Vector Based on HBV Core Protein, Journal of Xiamen University (Natural Science) 2004.05 Vol. 43.No. 4). For example, when a fragment (aa 1-149) of HBcAg protein is used as the carrier protein, the sequence encoding HBcAg aa79-81 in the nucleotide sequence encoding the HBcAg protein fragment (aa 1-149) can be deleted, and a linker can be introduced at each of the two ends of the deletion, and a BamH I/EcoR I restriction site can be designed between the two linkers, thereby obtaining the coding sequence of the carrier protein HBc149/mut (the amino acid sequence of HBc149/mut can be shown as SEQ ID NO: 21), and the coding sequence is cloned into an expression vector. Afterwards, using the BamH I/EcoR I restriction site, the coding sequence of the epitope peptide of interest is cloned between the linkers, thereby obtaining the HBc149 carrier protein chimerized with the epitope peptide.

In some embodiments, the recombinant protein comprises the sequence set forth in SEQ ID NO: 22.

In the fourteenth aspect, the present invention also provides an isolated nucleic acid molecule, which comprises a nucleotide sequence encoding the epitope peptide or variant thereof of the present invention or the recombinant protein of the present invention. In a fifteenth aspect, the present invention also provides a vector, which comprises the isolated nucleic acid molecule as described above. The vector of the present invention can be a cloning vector or an expression vector. In a preferred embodiment, the vector of the present invention is, for example plasmid, cosmid, phage, cosmid and the like.

In a sixteenth aspect, a host cell is also provided, which comprises the isolated nucleic acid molecule or the vector of the present invention. Such host cells include, but are not limited to, prokaryotic cell such as *E. coli* cell, and eukaryotic cell such as yeast cell, insect cell, plant cell, and animal cell (e.g., mammalian cell, such as mouse cell, human cell, etc.). The cell of the present invention may also be a cell line, such as 293T cell. In certain embodiments, the host cell is a microorganism.

In another aspect, a method for preparing the recombinant protein of the present invention is also provided, which comprises culturing the host cell of the present invention under suitable conditions, and recovering the recombinant protein of the present invention from a cell culture.

Display platform

The epitope peptide or variant thereof according to the twelfth aspect of the present invention can be displayed on a particle such as liposome, virus-like particle (VLP), nanoparticle, virosome or exosome to enhance the efficacy of antigen presentation in vivo.

Accordingly, in a seventeenth aspect, the present invention provides a particle, displaying on its surface the epitope peptide or variant thereof of the twelfth aspect. In certain embodiments, the particle is a virus-like particle (VLP). In certain embodiments, the epitope peptide or variant thereof according to the twelfth aspect of the present invention is fused to and/or displayed on a virus-like particle (VLP).

Antibody

In an eighteenth aspect, the present invention provides an antibody or antigen-binding fragment thereof, which specifically binds the epitope peptide or variant thereof of the twelfth aspect. The antibody or antigen-binding fragment thereof has one or more of the following characteristics: (a) neutralizing EBV in vitro or in a subject (e.g., a human); (b) blocking or inhibiting EBV from fusing to a cell in vitro or in a subject (e.g., a human); (c) preventing and/or treating EBV infection or a disease associated with EBV infection; (d) specifically binding to a 40KDa subunit of EBV gB protein.

In certain embodiments, the antibody or antigen-binding fragment thereof comprises:

(a) a heavy chain variable region (VH) comprising the following 3 complementarity determining regions (CDRs):

(i) a VH CDR1, which consists of the following sequence: SEQ ID NO: 11, or a sequence having a substitution, deletion or addition of one or several amino acids (e.g., a substitution, deletion or addition of 1, 2 or 3 amino acids) as compared thereto,

(ii) a VH CDR2, which consists of the following sequence: SEQ ID NO: 12, or a sequence having a substitution, deletion or addition of one or several amino acids (e.g., a substitution, deletion or addition of 1, 2 or 3 amino acids) as compared thereto, and

(iii) a VH CDR3, which consists of the following sequence: SEQ ID NO: 13, or a sequence having a substitution, deletion or addition of one or several amino acids (e.g., a substitution, deletion or addition of 1, 2 or 3 amino acids) as compared thereto;
and/or,

(b) a light chain variable region (VL) comprising the following 3 complementarity determining regions (CDRs):

(iv) a VL CDR1, which consists of the following sequence: SEQ ID NO: 14, or a sequence having a substitution, deletion or addition of one or several amino acids (e.g., a substitution, deletion or addition of 1, 2 or 3 amino acids) as compared thereto,

(v) a VL CDR2, which consists of the following sequence: SEQ ID NO: 15, or a sequence having a substitution, deletion or addition of one or several amino acids (e.g., a substitution, deletion or addition of 1, 2 or 3 amino acids) as compared thereto, and

(vi) a VL CDR3, which consists of the following sequence: SEQ ID NO: 16, or a sequence having a substitution, deletion or addition of one or several amino acids (e.g., a substitution, deletion or addition of 1, 2 or 3 amino acids) as compared thereto.

In certain embodiments, the CDR described in any one of (i) to (vi) is defined according to the IMGT numbering system.

In certain embodiments, the substitution described in any one of (i) to (vi) is a conservative substitution.

In certain embodiments, the antibody or antigen-binding fragment thereof comprises:

the following 3 heavy chain CDRs: a VH CDR1 having a sequence as set forth in SEQ ID NO: 11, a VH CDR2 having a sequence as set forth in SEQ ID NO: 12, and a VH CDR3 having a sequence as set forth in SEQ ID NO: 13; and/or,

the following 3 light chain CDRs: a VL CDR1 having a sequence as set forth in SEQ ID NO: 14, a VL CDR2 having a sequence as set forth in SEQ ID NO: 15, and a VL CDR3 having a sequence as set forth in SEQ ID NO: 16.

In certain embodiments, the antibody or antigen-binding fragment thereof comprises:

the 3 CDRs comprised in the heavy chain variable region (VH) as set forth in SEQ ID NO: 9; and/or,

the 3 CDRs comprised in the light chain variable region (VL) as set forth in SEQ ID NO: 10.

In certain embodiments, the 3 CDRs comprised in the VH and/or the 3 CDRs comprised in the VL are defined by the Kabat, IMGT or Chothia numbering system.

In certain embodiments, the antibody or antigen-binding fragment thereof comprises:

(a) a heavy chain variable region (VH) comprising an amino acid sequence selected from the group consisting of:

(i) a sequence set forth in SEQ ID NO: 9;

(ii) a sequence having a substitution, deletion or addition of one or several amino acids (e.g., a substitution, deletion or addition of 1, 2, 3, 4 or 5 amino acids) as compared to the sequence as set forth in SEQ ID NO: 9; or

(iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence as set forth in SEQ ID NO: 9;
and

(b) a light chain variable region (VL) comprising an amino acid sequence selected from the group consisting of:

(iv) a sequence set forth in SEQ ID NO: 10;

(v) a sequence having a substitution, deletion or addition of one or several amino acids (e.g., a substitution, deletion or addition of 1, 2, 3, 4 or 5 amino acids) as compared to the sequence as set forth in SEQ ID NO: 10; or

(vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence as set forth in SEQ ID NO: 10.

In certain embodiments, the substitution described in (ii) or (v) is a conservative substitution.

In certain embodiments, the antibody or antigen-binding fragment thereof comprises: a VH comprising a sequence set forth in SEQ ID NO: 9 and a VL comprising a sequence set forth in SEQ ID NO: 10.

In certain embodiments, the antibody or antigen-binding fragment thereof is humanized.

In certain embodiments, the antibody or antigen-binding fragment thereof comprises a framework region sequence derived from a human immunoglobulin.

In certain embodiments, the antibody or antigen-binding fragment thereof comprises: a heavy chain framework region sequence derived from a human heavy chain germline sequence, and a light chain framework region sequence derived from a human light chain germline sequence.

In certain embodiments, the antibody or antigen-binding fragment thereof further comprises a constant region derived from a rabbit or human immunoglobulin.

In certain embodiments, the heavy chain of the antibody or antigen-binding fragment thereof comprises a heavy chain constant region derived from a human immunoglobulin (e.g., IgG1, IgG2, IgG3, or IgG4), and the light chain of the antibody or antigen-binding fragment thereof comprises a light chain constant region derived from a human immunoglobulin (e.g., $\kappa$ or $\lambda$).

In certain embodiments, the heavy chain of the antibody or antigen-binding fragment thereof comprises a heavy chain constant region as set forth in SEQ ID NO: 30, and the light chain of the antibody or antigen-binding fragment thereof comprises a light chain constant region as set forth in SEQ ID NO: 31.

In certain embodiments, the antigen-binding fragment is selected from the group consisting of Fab, Fab', (Fab')$_2$, Fv, disulfide-linked Fv, scFv, diabody, and single domain antibody (sdAb); and/or, the antibody is a rabbit-derived antibody, a chimeric antibody, a humanized antibody, a bispecific antibody or a multispecific antibody.

The antibody of the present invention is not limited by its production method, for example, it can be produced by a genetic engineering method (recombinant technology), or by a chemical synthesis method. The antigen-binding fragment of the present invention can be obtained by hydrolyzing an intact antibody molecule (see, Morimoto et al., J. Biochem. Biophys. Methods 24:107-117 (1992), and Brennan et al., Science 229:81 (1985)). Additionally, these antigen-binding fragments can also be produced directly by recombinant host cells (reviewed in Hudson, Curr. Opin. Immunol. 11: 548-557 (1999); Little et al., Immunol. Today, 21: 364-370 (2000)). For example, Fab' fragments can be obtained directly from host cells; Fab' fragments can be chemically coupled to form F(ab')$_2$ fragments (Carter et al., Bio/Technology, 10: 163-167 (1992)). In addition, Fv, Fab or F(ab')$_2$ fragment can also be directly isolated from a culture medium of recombinant host cells. Other techniques for preparing these antigen-binding fragments are well known to those of ordinary skill in the art.

Accordingly, in a nineteenth aspect, the present invention provides an isolated nucleic acid molecule, which comprises a nucleotide sequence encoding the antibody or antigen-binding fragment thereof of the present invention, or a heavy chain variable region and/or a light chain variable region thereof. In certain embodiments, the isolated nucleic acid molecule encodes the antibody or antigen-binding fragment thereof of the present invention, or a heavy chain variable region and/or a light chain variable region thereof.

In a twentieth aspect, the present invention provides a vector (e.g., a cloning vector or an expression vector), which comprises the isolated nucleic acid molecule as described above. In certain embodiments, the vector of the present invention is, for example, plasmid, cosmid, phage, and the like.

In certain embodiments, the vector comprises a first nucleotide sequence encoding a heavy chain variable region of the antibody or antigen-binding fragment thereof of the present invention, and/or a second nucleotide sequence encoding a light chain variable region of the antibody or antigen-binding fragment thereof of the present invention; wherein the first nucleotide sequence and the second nucleotide sequence are provided on the same or different vectors.

In certain embodiments, the vector comprises a first nucleotide sequence encoding a heavy chain of the antibody or antigen-binding fragment thereof of the present invention, and/or a second nucleotide sequence encoding a light chain of the antibody or antigen-binding fragment thereof of the present invention; wherein the first nucleotide sequence and the second nucleotide sequence are provided on the same or different vectors.

In a twenty-first aspect, the present invention provides a host cell, which comprises the isolated nucleic acid molecule or the vector as described above. Such host cells include, but are not limited to, prokaryotic cell such as *E. coli* cells, and eukaryotic cell such as yeast cell, insect cell, plant cell, and animal cell (e.g., mammalian cell, such as mouse cell, human cell, etc.). In certain preferred embodiments, the host cell of the present invention is a mammalian cell, such as a CHO (e.g., CHO-K1, CHO-S, CHO DG44). In certain embodiments, the host cell is a microorganism.

In another aspect, there is provided a method for producing the antibody or antigen-binding fragment thereof of the present invention, which comprises culturing the host cell as described above under conditions that allow the expression of the antibody or antigen-binding fragment thereof, and recovering the antibody or antigen-binding fragment thereof from a cell culture of the host cell.

Conjugate

The antibody or antigen-binding fragment thereof of the present invention can be derivatized, for example, linked to another molecule (e.g., another polypeptide or protein). Typically, the derivatization (e.g., labeling) of the antibody or antigen-binding fragment thereof will not adversely affect its binding to EBV. Accordingly, the antibody or antigen-binding fragment thereof of the present invention is also intended to include such derivatized forms. For example, the antibody or antigen-binding fragment thereof of the present invention can be functionally linked (by chemical coupling, genetic fusion, non-covalent linkage, or other way) to one or more other molecular moieties, such as another antibody (e.g., forming a bispecific antibody), detection reagent, pharmaceutical reagent, and/or protein or polypeptide (e.g., avidin or polyhistidine tag) capable of mediating the binding of the antibody or antigen-binding fragment to another molecule. In addition, the antibody or antigen-binding fragment thereof of the present invention may also be derivatized with a chemical group, such as polyethylene glycol (PEG), methyl or ethyl, or saccharide group. These groups can be used to improve the biological properties of antibody, such as increasing serum half-life thereof.

Accordingly, in a twenty-second aspect, the present invention also provides a conjugate, which comprises the antibody or antigen-binding fragment thereof of the present invention and a detectable label linked thereto.

In certain embodiments, the detectable label can be adapted for use in immunological detection (e.g., enzyme-linked immunoassay, radioimmunoassay, fluorescence immunoassay, chemiluminescence immunoassay, etc.). In certain embodiments, the detectable label may be selected from the group consisting of enzyme (e.g., horseradish peroxidase, alkaline phosphatase, or β-galactosidase), chemiluminescent reagent (e.g., acridinium ester, Luminol and derivative thereof, or ruthenium derivative), fluorescent dye (e.g., fluorescein or fluorescent protein such as FITC, TRITC, or PE), radionuclide, or biotin.

In certain embodiments, the detectable label as described above can be linked to the antibody or antigen-binding fragment thereof of the present invention via a linker of various lengths to reduce potential steric hindrance.

Epitope peptide based therapeutic or prophylactic applications

The epitope peptide or recombinant protein of the present invention can be readily used in various therapeutic or prophylactic applications by inducing an immune response to EBV. For example, it can be administered to a subject to induce an immune response to EBV, for example, to induce the production of a neutralizing antibody against EBV. For a subject at risk of developing EBV infection, the epitope peptide or recombinant protein of the present invention can be administered to provide a prophylactic protection against viral infection.

Therefore, in the twenty-third aspect, the present invention provides an immunogenic composition, which comprises: (i) the epitope peptide or variant thereof described in the first aspect, the recombinant protein described in the second aspect, or the particle described in the sixth aspect, or (ii) the epitope peptide or variant thereof described in the twelfth aspect, the recombinant protein described in the thirteenth aspect, or the particle described in the seventeenth aspect.

In certain embodiments, the immunogenic composition further comprises a pharmaceutically acceptable carrier and/or excipient.

In certain embodiments, the immunogenic composition is capable of inducing, stimulating or enhancing an immune response against EBV.

In certain embodiments, the immunogenic composition is a vaccine.

In certain embodiments, the pharmaceutically acceptable carrier and/or excipient comprises an adjuvant. Adjuvants for co-administration or inclusion in the immunogenic composition according to the present invention should preferably be those that are potentially safe, well tolerated and effective in humans. Such adjuvants are well known to those skilled in the art.

The immunogenic composition of the present invention can also be used in combination with an additional agent known in the art for the treatment or prevention of EBV infection.

The immunogenic composition of the present invention may be formulated into any dosage form known in the medical art, for example, tablet, pill, suspension, emulsion, solution, gel, capsule, powder, granule, elixir, lozenge, suppository, injection (including solution for injection, sterile powder for injection, and concentrated solution for injection), inhalant, spray, etc. The preferred dosage form depends on the intended mode of administration and therapeutic use. The immunogenic composition of the present invention should be sterile and stable under the conditions of manufacture and storage. A preferred dosage form is injection. Such injection can be sterile solution for injection. In addition, the sterile solution for injection can be prepared as sterile lyophilized powder (e.g., by vacuum drying or freeze-drying) for ease of storage and use. Such sterile lyophilized powder can be dispersed in a suitable vehicle before use, such as water for injection (WFI), bacteriostatic water for injection (BWFI), sodium chloride solution (e.g., 0.9% (w/v) NaCl), dextrose solution (e.g., 5% dextrose), surfactant-containing solution (e.g., 0.01% polysorbate 20), pH buffered solution (e.g., phosphate buffered saline), Ringer's solution and any combination thereof.

The immunogenic composition of the present invention may be administered via any suitable method known in the art, including, but not limited to, oral, buccal, sublingual, ophthalmic, local, parenteral, rectal, intrathecal, intracisterna, inguinal, intravesical, topical (e.g., powder, ointment, or drop), or nasal route. However, for many therapeutic uses, the preferred route/mode of administration is parenteral (e.g., intravenous or bolus injection, subcutaneous injection, intraperitoneal injection, intramuscular injection). The skilled artisan will understand that the route and/or mode of administration will vary depending on the intended purpose.

The immunogenic composition of the present invention should be administered in an amount sufficient to induce an immune response against EBV. The appropriate amount of immunogen can be determined according to the particular disease or condition to be treated or prevented, its severity, the age of the subject, and other personal attributes of the particular subject (e.g., the general state of the subject's health and the robustness of the subject's immune system). Determination of effective doses is also guided by animal model studies followed by human clinical trials, and by administration regimens that significantly reduce the occurrence or severity of the target disease symptom or condition in subjects.

In another aspect, the present invention provides a use of the epitope peptide or variant thereof described in the first aspect or the twelfth aspect, or the recombinant protein described in the second aspect or the thirteenth aspect, or the isolated nucleic acid molecule described in the third aspect or the fourteenth aspect, or the vector described in the fourth aspect or the fifteenth aspect, or the host cell described in the fifth aspect or the sixteenth aspect, or the particle described in the sixth aspect or the seventeenth aspect in the manufacture of an immunogenic composition for preventing and/or treating an EBV infection or a disease associated with EBV infection in a subject. Also provided is a method for preventing and/or treating an EBV infection or a disease associated with EBV infection in a subject (e.g., a human), which comprises: administering to a subject in need thereof an effective amount of the epitope peptide or variant thereof described in the first aspect or the twelfth aspect, or the recombinant protein described in the second aspect or the thirteenth aspect, or the isolated nucleic acid molecule described in the third aspect or the fourteenth aspect, or the vector described in the fourth aspect or the fifteenth aspect, or the host cell described in the fifth aspect or the sixteenth aspect, or the particle described in the sixth aspect or the seventeenth aspect, or the immunogenic composition described in the twenty-third aspect.

In certain embodiments, the EBV infection is a chronic active EBV (CAEBV) infection or a primary EBV infection.

In certain embodiments, the disease associated with EBV infection is a mononucleosis or an EBV-associated cancer.

In certain embodiments, the EBV-associated cancer is selected from the group consisting of lymphoproliferative disorder (LPD) such as B-cell lymphoma, including Burkitt's lymphoma (BL), Hodgkin's lymphoma (HL), diffuse large B cellular lymphoma (DLBCL) or post-transplantation lymphoproliferative disorder (PTLD), or epithelial (nasopharyngeal, lung, breast) carcinoma, lymphoepithelioma, carcinoma with lymphoid stroma (GCLS, e.g. gastric cancer) or neuroglioma.

In certain embodiments, the subject is a mammal, such as a human.

For prophylactic applications, the epitope peptide, recombinant protein, particle or immunogenic composition of the present invention is provided prior to any symptom, for example prior to infection. Prophylactic administration of the immunogenic composition is used for the prevention or amelioration of any subsequent infection, to attenuate the expected severity, duration or extent of infection and/or associated disease symptoms after exposure or suspected exposure to the virus or after actual initiation of infection. Thus, in some embodiments, the subject to be treated is a subject who has or is at risk of developing an EBV infection, for example, due to being or likely to be exposed to EBV.

For therapeutic applications, the epitope peptide, recombinant protein, particle or immunogenic composition of the present invention is provided at or after the onset of symptoms of a disease or infection, for example after the onset of symptoms of EBV infection or after diagnosis of EBV infection.

Antibody-based therapeutic or prophylactic applications

The antibody of the present invention can be easily used in various therapeutic or prophylactic applications related to EBV infection. For example, it can be administered to a subject to neutralize virus, block or inhibit the fusion of virus with cells, thereby clearing the virus. For a subject at risk of developing EBV infection, the antibody of the present invention can be administered to provide prophylactic protection against viral infection.

In certain embodiments, it is beneficial to use the antibody or antigen-binding fragment thereof described in the seventh aspect of the present invention in combination with the antibody or antigen-binding fragment thereof described in the eighteenth aspect.

Accordingly, in a twenty-fourth aspect, the present invention provides a composition, which comprises:

(i) the antibody or antigen-binding fragment thereof described in the seventh aspect, the isolated nucleic acid molecule described in the eighth aspect, the vector described in the ninth aspect or the host cell described in the tenth aspect; and,

(ii) the antibody or antigen-binding fragment thereof described in the eighteenth aspect, the isolated nucleic acid molecule described in the nineteenth aspect, the vector described in the twentieth aspect or the host cell described in the twenty-first aspect;

In certain embodiments, the composition comprises the antibody or antigen-binding fragment thereof described in the seventh aspect, and the antibody or antigen-binding fragment thereof described in the eighteenth aspect.

In some embodiments, for the composition, the antibody of (i) is a chimeric antibody, and/or, the antibody of (ii) is a chimeric antibody.

It will be readily understood that (i) and (ii) of the composition may be provided as separate components or as a mixed combination.

When using the composition, (i) and (ii) of the composition may be used simultaneously or sequentially. For example, when the composition is administered to a subject, (i) and (ii) of the composition may be administered to the subject simultaneously or sequentially.

In the twenty-fifth aspect, the present invention provides a pharmaceutical composition, which comprises the antibody or antigen-binding fragment thereof described in the seventh aspect or the eighteenth aspect or the composition described in the twenty-fourth aspect, and optionally a pharmaceutically acceptable carrier and/or excipient.

In certain embodiments, the pharmaceutical composition may also comprise an additional pharmaceutically active agent.

In certain exemplary embodiments, the pharmaceutically acceptable carrier and/or excipient comprises a sterile injectable liquid (e.g., aqueous or non-aqueous suspension or solution). In certain exemplary embodiments, such sterile injectable liquid is selected from the group consisting of water for injection (WFI), bacteriostatic water for injection (BWFI), sodium chloride solution (e.g., 0.9% (w/v) NaCl), dextrose solution (e.g., 5% dextrose), surfactant-contained solution (e.g., 0.01% polysorbate 20), pH buffered solution (e.g., phosphate buffered saline), Ringer's solution, and any combination thereof.

In another aspect, the present invention provides a method for neutralizing EBV, which comprises using the antibody or antigen-binding fragment thereof or the pharmaceutical composition of the present invention. The method can be used to neutralize EBV in vitro or in a subject (e.g., a human).

In certain embodiments, the method is used to neutralize the virulence of EBV in a sample. In certain embodiments, the method comprises: contacting a sample comprising EBV with the antibody or antigen-binding fragment thereof or the pharmaceutical composition of the present invention.

In another aspect, the present invention provides a use of the antibody or antigen-binding fragment thereof described in the seventh or eighteenth aspect, or the isolated nucleic acid molecule described in the eighth or nineteenth aspect, or the vector described in the ninth or twentieth aspect, or the host cell described in the tenth or twenty-first aspect, or the composition described in the twenty-fourth aspect, or the pharmaceutical composition described in the twenty-fifth aspect in the manufacture of a medicament for neutralizing the virulence of EBV, or for inhibiting or blocking the fusion of EBV with cells, or for preventing and/or treating an EBV infection or a disease associated with EBV infection in a subject. Also provided is a method for preventing or treating an EBV infection or a disease associated with EBV infection in a subject, which comprises: administering to the subject in need thereof an effective amount of the antibody or antigen-binding fragment thereof of the present invention, the isolated nucleic acid molecule or vector or host cell encoding the antibody or antigen-binding fragment thereof, the composition or pharmaceutical composition.

In certain embodiments, the EBV infection is a chronic active EBV (CAEBV) infection or a primary EBV infection.

In certain embodiments, the disease associated with EBV infection is a mononucleosis or an EBV-associated cancer.

In certain embodiments, the EBV-associated cancer is selected from the group consisting of lymphoproliferative disorder (LPD) such as B-cell lymphoma, including Burkitt's lymphoma (BL), Hodgkin's lymphoma (HL), diffuse large B cellular lymphoma (DLBCL) or post-transplantation lymphoproliferative disorder (PTLD), or epithelial (nasopharyngeal, lung, breast) carcinoma, lymphoepithelioma, carcinoma with lymphoid stroma (GCLS, e.g. gastric cancer) or neuroglioma.

In certain embodiments, the subject is a mammal, such as a human.

In certain embodiments, the antibody or antigen-binding fragment thereof is used alone, or in combination with an additional pharmaceutically active agent (e.g., an additional antiviral agent). The antibody or antigen-binding fragment thereof of the present invention and the additional pharmaceutically active agent may be administered simultaneously, separately or sequentially. In addition, the antibody described in the seventh aspect and the antibody described in the eighteenth aspect of the present invention may also be administered in combination.

The antibody or antigen-binding fragment thereof of the present invention, or the pharmaceutical composition of the present invention can be formulated into any dosage form known in the medical field, for example, tablet, pill, suspension, emulsion, solution, gel, capsule, powder, granule, elixir, lozenge, suppository, injection (including solution for injection, sterile powder for injection, and concentrated solution for injection), inhalant, spray, etc. The preferred dosage form depends on the intended mode of administration and therapeutic use. The antibody or antigen-binding fragment thereof or the pharmaceutical composition of the present invention should be sterile and stable under the conditions of manufacture and storage. A preferred dosage form is injection. Such injection can be a sterile solution for injection. For example, the sterile solution for injection can be prepared by incorporating in an appropriate solvent a necessary dose of the antibody or antigen-binding fragment thereof of the present invention and, optionally, concomitantly incorporating other desired ingredients (including, but not limited to, pH adjuster, surfactant, adjuvant, ionic strength enhancer, isotonic agent, preservative, diluent, or any combination thereof), followed by filter sterilization. In addition, the sterile solution for injection can be prepared as sterile lyophilized powder (e.g., by vacuum drying or freeze-drying) for ease of storage and use. Such sterile lyophilized powder can be dispersed in a suitable vehicle before use, such as water for injection (WFI), bacteriostatic water for injection (BWFI), sodium chloride solution (e.g., 0.9% (w/v) NaCl), dextrose solution (e.g., 5% dextrose), surfactant-contained solution (e.g., 0.01% polysorbate 20), pH buffered solution (e.g., phosphate buffered saline), Ringer's solution and any combination thereof.

The antibody or antigen-binding fragment thereof of the present invention, or the pharmaceutical composition of the present invention may be administered by any suitable method known in the art, including, but not limited to, oral, buccal, sublingual, ophthalmic, local, parenteral, rectal, intrathecal, intra-cisterna, inguinal, intravesical, topical (e.g., powder, ointment, or drop), or nasal route. However, for many therapeutic uses, the preferred route/mode of administration is parenteral (e.g., intravenous or bolus injection, subcutaneous injection, intraperitoneal injection, intramuscular injection). The skilled artisan will understand that the route and/or mode of administration will vary depending on the intended purpose. In certain embodiments, the antibody or antigen-binding fragment thereof or the pharmaceutical composition of the present invention is administered by intravenous injection or bolus injection.

Antibody-based detection applications

The antibody or antigen-binding fragment thereof of the present invention can specifically bind to the gB protein (especially the ectodomain) of EBV, so that it can be used to detect EBV or gB protein thereof, and optionally diagnose whether the subject is infected with EBV.

Therefore, in another aspect, the present invention provides a kit, which comprises the antibody or antigen-binding fragment thereof of the present invention, or the conjugate of the present invention.

In some embodiments, the kit comprises the conjugate of the present invention.

In other embodiments, the kit comprises the antibody or antigen-binding fragment thereof of the present invention. In certain embodiments, the antibody or antigen-binding fragment thereof does not comprise a detectable label. In certain embodiments, the kit further comprises a second antibody that specifically recognizes the antibody or antigen-binding fragment thereof of the present invention; optionally, the second antibody further comprises a detectable label, such as an enzyme (e.g., horseradish peroxidase or alkaline phosphatase), chemiluminescent reagent (e.g., acridinium ester, luminol and derivative thereof, or ruthenium derivative), fluorescent dye (e.g., fluorescein or fluorescent protein), radionuclide or biotin.

In certain embodiments, the second antibody is specific for an antibody of the species (e.g., rabbit or human) from which the constant region comprised by the antibody or antigen-binding fragment thereof of the present invention is derived.

In certain embodiments, the second antibody is an anti-immunoglobulin (e.g., human or rabbit immunoglobulin) antibody, for example, an anti-IgG antibody. In certain embodiments, the second antibody is an anti-rabbit IgG antibody or an anti-human IgG antibody.

In certain embodiments, the kits of the present invention may further comprise a reagent for allowing the corresponding detectable label to be detected. For example, when the detectable label is an enzyme, the kit may also include a chromogenic substrate for the corresponding enzyme, such as o-phenylenediamine (OPD), tetramethylbenzidine (TMB), ABTS, or luminol compound for horseradish peroxidase, or p-nitrophenyl phosphate (p-NPP) or AMPPD for alkaline phosphatase. For example, when the detectable label is a chemiluminescence reagent (e.g., an acridinium ester compound), the kit may further comprise a pre-excitation solution and/or an excitation solution for chemiluminescence.

In another aspect, the present invention provides a method of detecting the presence or level of EBV or gB protein thereof, or an EBV-infected cell in a sample, which comprises using the antibody or antigen-binding fragment thereof of the present invention. In certain embodiments, the gB protein is expressed on the cell surface.

In certain embodiments, the method is an immunological assay, such as a western blot, an enzyme immunoassay (e.g., ELISA), a chemiluminescence immunoassay, a fluorescence immunoassay, or a radioimmunoassay.

In some embodiments, the method comprises using the conjugate of the present invention.

In other embodiments, the method comprises using the antibody or antigen-binding fragment thereof of the present invention. In certain embodiments, the antibody or antigen-binding fragment thereof does not comprise a detectable label. In some embodiments, the method further comprises using a second antibody bearing a detectable label (e.g., enzyme (e.g., horseradish peroxidase or alkaline phosphatase), chemiluminescence reagent (e.g., acridinium ester, luminol or ruthenium derivative), fluorescent dye (e.g., fluorescein or fluorescent protein), radionuclide or biotin) to detect the antibody or antigen-binding fragment thereof.

In certain embodiments, the second antibody is specific for an antibody of the species (e.g., rabbit or human) from which the constant region comprised by the antibody or antigen-binding fragment thereof of the present invention is derived.

In certain embodiments, the second antibody is an anti-immunoglobulin (e.g., human or rabbit immunoglobulin) antibody, for example, an anti-IgG antibody. In certain embodiments, the second antibody is an anti-rabbit IgG antibody or an anti-human IgG antibody.

In certain embodiments, the method comprises: (1) contacting the sample with the antibody or antigen-binding fragment thereof of the present invention; (2) detecting the formation of an antigen-antibody immune complex or detecting an amount of the immune complex. The formation of the immune complex indicates the presence of gB protein, EBV or EBV-infected cell.

In certain embodiments, the method can be used for a diagnostic purpose, for example, whether a subject is infected with EBV can be diagnosed based on the presence or level of EBV or gB protein thereof in a sample. In such embodiments, the sample is a bodily fluid sample (e.g., whole blood, plasma, serum, salivary, excretion or urine) from a subject (e.g., a mammal, preferably a human).

In certain embodiments, the method may be used for a non-diagnostic purpose, for example, the sample is not derived from a subject, for example, is a vaccine sample.

In certain embodiments, the subject is a mammal, such as a human.

In another aspect, there is provided the use of the antibody or antigen-binding fragment thereof of the present invention or the conjugate in the preparation of a detection reagent for detecting the presence or level of EBV or its gB protein, or EBV-infected cells in a sample, and/or for diagnosing whether a subject is infected with EBV.

In certain embodiments, the method is an immunological assay, such as a western blot, an enzyme immunoassay (e.g., ELISA), a chemiluminescent immunoassay, a fluorescence immunoassay, or a radioimmunoassay.

In certain embodiments, the detection reagent detects the presence or level of EBV or gB protein thereof, or cells infected with EBV in the sample by the detection method as described above, and optionally diagnoses whether the subject is infected with EBV according to the detection result.

In certain embodiments, the sample is a bodily fluid sample (e.g., whole blood, plasma, serum, salivary excretion or urine) from a subject (e.g., a mammal, preferably a human).

Definition of terms

In the present invention, unless otherwise specified, the scientific and technical terms used herein have the meanings commonly understood by those skilled in the art. In addition, the laboratory procedures of virology, biochemistry, nucleic acid chemistry, and immunology used herein are routine procedures widely used in the corresponding fields. Meanwhile, in order to better understand the present invention, definitions and explanations of related terms are provided below.

As used herein, the term "Epstein-Barr virus (EBV)", also known as human herpesvirus type 4 (HHV-4), is a member of the herpesvirus family and is one of the most common human viruses. The genome sequence of EBV is known to those skilled in the art, and can be referred to various public databases, such as GenBank ID: KF373730.1 (M81 strain), GenBank ID: AB828190.1 (1 LGY-C666-1 strain), Genbank ID: KC617875.1 (C666-1 strain), GenBank ID: JQ009376.2 (HKNPC1 strain), etc.

As used herein, the term "gB" or "gB protein" refers to a glycoprotein of EBV, which, together with gH and gL protein, constitutes the core membrane fusion machinery that mediates EBV entry into cells. In addition to being involved in fusion, EBV gB is also essential for virus maturation and release, and gB-deficient viruses cannot be produced. The amino acid sequence of gB protein is well known to those skilled in the art (see, for example, the sequence set forth in GenBank: KF373730.1).

As used herein, when referring to the amino acid sequence of gB, it is described using the sequence set forth in SEQ ID NO:17. For example, the expression "amino acid residues at positions 341-362 of gB protein" refers to the amino acid residues at positions 341-362 of the polypeptide set forth in SEQ ID NO:17. However, those skilled in the art understand that in the amino acid sequence of gB, a mutation or variation (including but not limited to, substitution, deletion and/or addition, such as gB of different gene types or gene subtypes) can be naturally produced or artificially introduced, without affecting its biological function. Therefore, in the present invention, the term "gB" shall include all such sequences, including for example the sequence set forth in SEQ ID NO: 17 and its natural or artificial variants. And, when describing a sequence fragment of gB, it includes not only the sequence fragment of SEQ ID NO: 17, but also the corresponding sequence fragment in its natural or artificial variant. For example, the expression "amino acid residues at positions 341-362 of gB protein" includes the amino acid residues at positions 341-362 of SEQ ID NO: 17, and corresponding fragments in its (natural or artificial) variants. According to the present invention, the expression "corresponding sequence fragment" or "corresponding fragment" refers to a fragment at equivalent positions in sequences being compared, when the sequences are optimally aligned, i.e. when the sequences are aligned for the highest percentage identity.

As used herein, the term "epitope" refers to a portion of an antigen capable of being recognized and specifically bound by a particular antibody. When the antigen is a polypeptide, the epitope can be formed by consecutive amino acids or non-consecutive amino acids gathered by the tertiary folding of protein, which are called linear epitope or conformational epitope, respectively. Epitopes formed from consecutive amino acids are typically retained upon protein denaturation, whereas epitopes formed by tertiary folding are typically lost upon protein denaturation. An epitope typically comprises at least 3, 4, 5, 6, 7, 8, 9, 10, 11, 12, 13, 14 or 15 consecutive or non-consecutive amino acids in a unique spatial conformation.

As used herein, the term "epitope peptide" refers to a peptide segment on an antigen that can serve as an epitope. In some cases, an epitope peptide alone is capable of being specifically recognized/bound by an antibody against the epitope. In other cases, it may be necessary to fuse the epitope peptide to a carrier protein so that the epitope peptide can be recognized by a specific antibody. As used herein, the term "carrier protein" refers to a protein that can act as a carrier for an epitope peptide, i.e., at a specific position thereof (e.g., inside, or at N-terminal or C-terminal of the protein), an epitope peptide can be inserted, so that the epitope peptide can be presented, and the epitope peptide can be recognized by the antibody or the immune system. Such carrier proteins are well known to those skilled in the art, and include, for example, HPV L1 protein (in which, an epitope peptide may be inserted between amino acids 130-131 or between amino acids 426-427 of the protein, see, Slupetzky, K. et al., J Gen Virol,2001, 82: 2799-2804; Varsani, A. et al., J Virol, 2003, 77: 8386-8393), HBV core antigen (in which, the amino acids 79-81 of the protein can be replaced with an epitope peptide, see, Koletzki, D., et al. Biol Chem, 1999, 380: 325-333), woodchuck hepatitis virus core protein (in which the amino acids 79-81 of the protein can be replaced by an epitope peptide, see, Sabine König, J. Virol. 1998, 72(6):4997), CRM197 protein (in which, an epitope peptide can be linked to the N- or C-terminus of the protein or fragment thereof). Optionally, a linker can be used between the epitope peptide and the carrier protein to facilitate the folding of each of them.

As used herein, the term "antibody" refers to an immunoglobulin molecule, which is usually composed of two pairs of polypeptide chains, each pair having one light chain (LC) and one heavy chain (HC). Antibody light chains can be classified as $\kappa$ (kappa) and $\lambda$ (lambda) light chains. Heavy chains can be classified as $\mu, \delta, \gamma, \alpha$ or $\varepsilon$, and the antibody's isotypes are defined as IgM, IgD, IgG, IgA, and IgE, respectively. Within the light and heavy chains, the variable and constant regions are joined by a "J" region of about 12 or more amino acids, and the heavy chain also comprises a "D" region of about 3 or more amino acids. Each heavy chain is composed of a heavy chain variable region (VH) and a heavy chain constant region (CH). The heavy chain constant region consists of 3 domains (CH1, CH2 and CH3). Each light chain is composed of a light chain variable region (VL) and a light chain constant region (CL). The light chain constant region consists of one domain, CL. The constant domains are not directly involved in antibody-antigen binding, but exhibit a variety of effector functions, such as mediating the binding of immunoglobulin to host tissues or factors, including various cells of the immune system (e.g., effector cells) and the first component (C1q) of classical complement system. The VH and VL regions can also be subdivided into regions of high variability called complementarity determining regions (CDRs) interspersed with more conserved regions called framework regions (FRs). Each $V_H$ and $V_L$ consists of 3 CDRs and 4 FRs arranged in the following order: FR1, CDR1, FR2, CDR2, FR3, CDR3, FR4, from amino-terminus to carboxy-terminus. The variable regions (VH and VL) of each heavy chain/light chain pair form antigen binding site, respectively. The allocation of amino acids in each region or domain can follow the definitions of Kabat, Sequences of Proteins of Immunological Interest (National Institutes of Health, Bethesda, Md. (1987 and 1991)), or Chothia & Lesk (1987) J. Mol. Biol. 196 :901-917; as defined by Chothia et al. (1989) Nature 342:878-883.

As used herein, the term "complementarity determining region" or "CDR" refers to the amino acid residues in antibody variable region that are responsible for antigen binding. The variable regions of the heavy and light chains each comprise three CDRs, designated as CDR1, CDR2 and CDR3. The precise boundaries of these CDRs can be defined according to various numbering systems known in the art, such as the Kabat numbering system (Kabat et al., Sequences of Proteins of Immunological Interest, 5th Ed. Public Health Service, National Institutes of Health, Bethesda, Md., 1991), the Chothia numbering system (Chothia & Lesk (1987) J. Mol. Biol. 196:901-917; Chothia et al. (1989) Nature 342:878-883), or the IMGT numbering system (Lefranc et al. al., Dev. Comparat. Immunol. 27:55-77, 2003). For a given antibody, those skilled in the art will readily identify the CDRs defined by each numbering system. Also, the correspondence between different numbering systems is well known to those skilled in the art (e.g., see, Lefranc et al., Dev. Comparat. Immunol. 27:55-77, 2003).

In the present invention, the CDRs comprised in the antibody or antigen-binding fragment thereof of the present invention can be determined according to various numbering systems known in the art. In certain embodiments, the CDRs comprised in the antibody or antigen-binding fragment thereof of the present invention are preferably determined by Kabat, Chothia or IMGT numbering system. In certain embodiments, the CDRs comprised in the antibody or antigen-binding fragment thereof of the present invention are preferably determined by the IMGT numbering system.

As used herein, the term "framework region" or "FR" residues refers to those amino acid residues in an antibody variable region other than the CDR residues as defined above.

The term "antibody" is not limited to any particular method of producing antibody. For example, it includes recombinant antibody, monoclonal antibody and polyclonal antibody. The antibody can be of different isotype, for example, an IgG (e.g., IgG1, IgG2, IgG3 or IgG4 subtype), IgAl, IgA2, IgD, IgE or IgM antibody.

As used herein, the term "antigen-binding fragment" of antibody refers to a polypeptide comprising a fragment of a full-length antibody that retains the ability to specifically bind to the same antigen to which the full-length antibody binds, and/or competes with the full-length antibody for specific binding to the antigen, which is also referred to as an "antigen-binding moiety". See, generally, Fundamental Immunology, Ch. 7 (Paul, W., ed., 2nd ed., Raven Press, N.Y. (1989)), which is incorporated herein by reference in its entirety for all purposes. An antigen-binding fragment of an antibody can be obtained by recombinant DNA technique or by enzymatic or chemical cleavage of intact antibody. Non-limiting examples of antigen-binding fragment include Fab, Fab', F(ab')$_2$, Fd, Fv, complementarity determining region (CDR) fragment, scFv, diabody, single domain antibody, chimeric antibody, linear antibody, nanobody (technology from Domantis), probody, and such a polypeptide that comprises at least a portion of antibody sufficient to confer the polypeptide with antigen binding ability. Engineered antibody variants are reviewed by Holliger et al., 2005; Nat Biotechnol, 23: 1126-1136.

As used herein, the term "full-length antibody" refers to an antibody consisting of two "full-length heavy chains" and two "full-length light chains". Among them, "full-length heavy chain" refers to such a polypeptide chain, which consists of a heavy chain variable region (VH), a heavy chain constant region CH1 domain, a hinge region (HR), a heavy chain constant region CH2 domain, and a heavy chain constant region CH3 domain; and, when the full-length antibody is of IgE isotype, it optionally further comprises a heavy chain constant region CH4 domain. Preferably, the "full-length heavy chain" is a polypeptide chain consisting of VH, CH1, HR, CH2 and CH3 in the direction from N-terminal to C-terminal. The "full-length light chain" is a polypeptide chain consisting of a light chain variable region (VL) and a light chain constant region (CL) in the direction from N-terminal to C-terminal. The two pairs of full-length antibody chains are linked together by disulfide bonds between CL and CH1 and between the HRs of the two full-length heavy chains. The full-length antibody of the present invention can be derived from a single species, such as human; it can also be a chimeric antibody or a humanized antibody. The full-length antibody of the present invention comprises two antigen-binding sites respectively formed by VH and VL pairs, and these two antigen-binding sites specifically recognize/bind to the same antigen.

As used herein, the term "Fd" refers to an antibody fragment consisting of VH and CH1 domains; the term "dAb fragment" refers to an antibody fragment consisting of a VH domain (Ward et al., Nature 341:544 546 (1989)); the term "Fab fragment" refers to an antibody fragment consisting of VL, VH, CL and CH1 domains; the term "F(ab')$_2$ fragment" refers to an antibody fragment comprising two Fab fragments that are linked via disulfide bridges on hinge regions; the term "Fab' fragment" refers to a fragment that is obtained by reducing disulfide bonds linking the two heavy chain fragments in F(ab')$_2$ fragment, and consists of an intact light chain and a Fd fragment (consisting of VH and CH1 domains) of the heavy chain.

As used herein, the term "Fv" refers to an antibody fragment consisting of the VL and VH domains of a single arm of an antibody. The Fv fragment is generally considered to be the smallest antibody fragment capable of forming a complete antigen-binding site. It is generally believed that the six CDRs confer antigen-binding specificity to an antibody. However, even one variable region (e.g., Fd fragment, which comprises only three CDRs specific for antigen) is able to recognize and bind to antigen, although it perhaps has a lower affinity than that of the complete binding site.

As used herein, the term "Fc" refers to an antibody fragment formed by linking the second and third constant regions of the first heavy chain of an antibody with the second and third constant regions of the second heavy chain via disulfide bonds. The Fc fragment of an antibody has a variety of different functions, but is not involved in antigen binding.

As used herein, the term "scFv" refers to a single polypeptide chain comprising VL and VH domains, wherein the VL and VH are connected via a linker (see, for example, Bird et al., Science 242:423-426 (1988); Huston et al., Proc. Natl. Acad. Sci. USA 85:5879-5883 (1988); and Pluckthun, The Pharmacology of Monoclonal antibody, Vol. 113, Roseburg and Moore eds., Springer-Verlag, New York, pp. 269-315 (1994)). Such scFv molecules may have the general structure: NH$_2$-VL-linker-VH-COOH or NH$_2$-VH-linker-VL-COOH. A suitable prior art linker may consist of repeated amino acid sequence GGGGS or variant thereof. For example, a linker having an amino acid sequence (GGGGS)$_4$ can be used, and variant thereof can also be used (Holliger et al. (1993), Proc. Natl. Acad. Sci. USA 90: 6444-6448). Other linkers described by Alfthan et al. (1995), Protein Eng. 8:725-731, Choi et al. (2001), Eur. J. Immunol. 31: 94-106, Hu et al. (1996), Cancer Res. 56:3055-3061, described by Kipriyanov et al. (1999), J. Mol. Biol. 293:41-56 and Roovers et al. (2001), Cancer Immunol can be used in the present invention. In some cases, there may also be a disulfide bond between the VH and VL of scFv. In certain embodiments of the present invention, scFv can form di-scFv, which means that two or more single scFvs are connected in series to form an antibody. In certain embodiments of the present invention, scFv can form (scFv)$_2$, which means that two or more single scFvs are connected parallelly to form an antibody.

As used herein, the term "diabody" means that its VH and VL domains are expressed on a single polypeptide chain, but a linker that is too short to allow pairing between the two domains of the same chain is used, so that the domains are forced to pair with the complementary domains of another chain and to produce two antigen-binding sites (see, for example, Holliger P. et al., Proc. Natl. Acad. Sci. USA 90:6444-6448 (1993), and Poljak R. J. et al., Structure 2:1121-1123 (1994)).

As used herein, the term "single-domain antibody (sdAb)" has the meaning commonly understood by those skilled in the art, which refers to an antibody fragment consisting of single monomer variable antibody domain (e.g., a single heavy chain variable region), which retains the ability to specifically bind the same antigen to which the full-length antibody binds. Single-domain antibody is also called nanobody.

Each of the above antibody fragments maintains the ability to specifically bind to the same antigen to which the full-length antibody binds, and/or competes with the full-length antibody for specific binding to the antigen.

Antigen-binding fragments of antibody (e.g., the antibody fragments described above) can be obtained from a given antibody (e.g., an antibody provided herein) using conventional techniques known to those skilled in the art (e.g., recombinant DNA techniques or enzymatic or chemical cleavage methods), and antigen-binding fragments of antibody can be screened for specificity in the same manner as for the intact antibody.

Herein, unless the context clearly dictates otherwise, when referring to the term "antibody", it includes not only intact antibody but also antigen-binding fragments of antibody.

As used herein, the term "chimeric antibody" refers to an antibody, in which a part of its light chain and/or heavy chain is derived from an antibody (which may be derived from a specific species or belong to a certain specific antibody class or subclass), and the other part of its light chain or/and heavy chain is derived from another antibody (which may be derived from the same or different species or belong to the same or different antibody class or subclass), but in any case, it still retains the binding activity to the target antigen (U.S.P 4,816,567 to Cabilly et al.; Morrison et al., Proc. Natl. Acad. Sci. USA, 81:6851 6855 (1984)). In certain embodiments, the term "chimeric antibody" may include such an antibody (e.g., human-mouse chimeric antibody) in which the heavy and light chain variable regions of the antibody are derived from a first antibody (e.g., a murine antibody), and the heavy and light chain constant regions of the antibody are derived from a second antibody (e.g., a human antibody).

As used herein, the term "humanized antibody" refers to a genetically engineered non-human antibody whose amino acid sequence has been modified to increase sequence homology with a human antibody. Generally speaking, all or part of the CDR regions of a humanized antibody are derived from a non-human antibody (donor antibody), and all or part of the non-CDR regions (e.g., variable region FR and/or constant region) are derived from human immunoglobulin (receptor antibody). Typically, at least one or two, but usually all three acceptor CDRs (of heavy and/or light immunoglobulin chains) of a humanized antibody are replaced by donor CDRs. The immunoglobulin providing the CDRs is called the "donor" and the immunoglobulin providing the framework is called the "acceptor". In one embodiment, the donor immunoglobulin is a non-human (e.g., rabbit) antibody, and the acceptor framework can be a naturally occurring human framework or a sequence having an identity of about 85%, 90%, 95%, 99% or higher as compared thereto. Humanized antibody generally retains the desired properties of the donor antibody, including, but not limited to, antigen specificity, affinity, reactivity, and the like. The donor antibody can be a mouse, rat, rabbit or non-human primate antibody having desired properties (e.g., antigen specificity, affinity, reactivity, etc.).

The chimeric antibody or humanized antibody of the present invention can be prepared according to the sequence of monoclonal antibody produced by an immunized animal (e.g., rabbit). DNA encoding the heavy and light chains can be obtained from hybridomas or specific B cells of interest from the immunized animal, and engineered to comprise human immunoglobulin sequences using standard molecular biology techniques.

To prepare a chimeric antibody, the immunoglobulin variable regions of an immunized animal (e.g., rabbit) can be linked to human immunoglobulin constant regions using methods known in the art (see, for example, US Patent No. 4,816,567 to Cabilly et al.). For example, a VH-encoding DNA is operably linked to another DNA molecule encoding a heavy chain constant region to obtain a full-length heavy chain gene. The sequence of the human heavy chain constant region gene is known in the art (see, for example, Kabat, E.A. et al. (1991) Sequences of Proteins of Immunological Interest, Fifth Edition, U.S. Department of Health and Human Services, NIH Publication No. 91-3242), and DNA fragments comprising these regions can be amplified by standard PCR. The heavy chain constant region may be an IgG1, IgG2, IgG3, IgG4, IgA, IgE, IgM or IgD constant region, but is generally preferred to be an IgG1 or IgG4 constant region. For example, the VL-encoding DNA is operably linked to another DNA molecule encoding a light chain constant region CL to obtain a full-length light chain gene (as well as a Fab light chain gene). The sequence of the human light chain constant region gene is known in the art (see, for example, Kabat, E.A. et al. (1991) Sequences of Proteins of Immunological Interest, Fifth Edition, U.S. Department of Health and Human Services, NIH Publication No. 91-3242), and DNA fragments comprising these regions can be amplified by standard PCR. The light chain constant region can be a κ or λ constant region, but is generally preferred to be a κ constant region.

To prepare a humanized antibody, the CDR regions of an immunized animal (e.g., a rabbit) can be grafted into a human framework sequence using methods known in the art (see, U.S. Patent No. 5,225,539 to Winter; U.S. Patent Nos. 5,530,101 to Queen et al.; 5,585,089; 5,693,762 and 6,180,370; and Lo, Benny, K.C., editor, in Antibody Engineering: Methods and Protocols, volume 248, Humana Press, New Jersey, 2004). The human framework sequence can be derived from, for example, a germline antibody gene. The term "germline antibody gene" refers to an immunoglobulin-encoding sequence present in the genome of an organism, which has not undergone a maturing process for genetic rearrangement and mutation that can lead to the expression of a specific immunoglobulin. "Heavy chain germline gene" refers to a germline antibody gene or gene fragment encoding the heavy chain of immunoglobulin, which comprises V gene (variable), D gene (diversity), J gene (joining) and C gene (constant); similarly, the expression "light chain germline gene" refers to a germline antibody gene or gene fragment encoding the light chain of immunoglobulin, which comprises V gene (variable), J gene (joining) and C gene (constant). In the present invention, the amino acid sequence encoded by the germline antibody gene or germline antibody gene fragment is also called "germline sequence", and the amino acid sequence encoded by the heavy chain germline gene is called heavy chain germline sequence, the amino acid sequence encoded by the light chain germline gene is called light chain germline sequence. Germline antibody genes or germline antibody gene fragments and their corresponding germline sequences are well known to those skilled in the art, and can be obtained or queried from professional databases (e.g., IMGT, UNSWIg, NCBI or VBASE2).

As used herein, the term "specific binding" refers to a non-random binding reaction between two molecules, such as the reaction between an antibody and the antigen it targets. The strength or affinity of a specific binding interaction can be expressed in terms of the equilibrium dissociation constant ($K_D$) for that interaction. In the present invention, the term "$K_D$" refers to the dissociation equilibrium constant of a specific antibody-antigen interaction, which is used to describe the binding affinity between an antibody and an antigen. The smaller the equilibrium dissociation constant, the tighter the antibody-antigen binding, and the higher the affinity between the antibody and the antigen. The specific binding properties between two molecules can be determined using methods well known in the art, for example using surface plasmon resonance (SPR) in a BIACORE instrument.

As used herein, the detectable label according to the present invention can be any substance detectable by fluorescent, spectroscopic, photochemical, biochemical, immunological, electrical, optical or chemical means. Such labels are well known in the art, examples of which include, but are not limited to, enzyme (e.g., horseradish peroxidase, alkaline phosphatase, β-galactosidase, urease, glucose oxidase, etc.), radionuclide (e.g., $^3H$, $^{125}I$, $^{35}S$, $^{14}C$, or $^{32}P$), fluorescent dye (e.g., fluorescein isothiocyanate (FITC), fluorescein, tetramethylrhodamine isothiocyanate (TRITC), phycoerythrin (PE), Texas Red, rhodamine, quantum dots or cyanine dye derivative (e.g., Cy7, Alexa 750)), luminescent substance (e.g., chemiluminescent substance, such as acridinium ester compound, luminol and derivative thereof, ruthenium derivative such as ruthenium terpyridine), magnetic beads (e.g., Dynabeads®), calorimetric marker such as colloidal gold or colored glass or plastic (e.g., polystyrene, polypropylene, latex, etc.) beads, and biotin for binding to avidin (e.g., streptavidin) modified with the above markers.

As used herein, the term "vector" refers to a nucleic acid delivery vehicle into which a polynucleotide can be inserted. When the vector is capable of making the expression of the protein encoded by the inserted polynucleotide, the vector is called an expression vector. A vector can be introduced into a host cell by transformation, transduction or transfection, so that the genetic material elements it carries can be expressed in the host cell. Vectors are well known to those skilled in the art, including but not limited to: plasmid; phagemid; cosmid; artificial chromosome, such as yeast artificial chromosome (YAC), bacterial artificial chromosome (BAC) or P1-derived artificial chromosome (PAC); phage such as λ phage or M13 phage and animal virus. Animal viruses that can be used as vectors include, but are not limited to, retrovirus (including lentivirus), adenovirus, adeno-associated virus, herpesvirus (e.g., herpes simplex virus), poxvirus, baculovirus, papillomavirus, papovavirus (e.g., SV40). A vector can comprise a variety of elements that control expression, including but not limited to, promoter sequence, transcription initiation sequence, enhancer sequence, selection element, and reporter gene. In addition, the vector may also comprise an origin of replication.

As used herein, the term "host cell" refers to a cell into which a vector can be introduced, which includes, but is not limited to, prokaryotic cell such as *Escherichia coli* or *Bacillus subtilis,* fungal cell such as yeast cell or *Aspergillus,* insect cell such as *S2 Drosophila* cell or Sf9, or animal cell such as fibroblast, CHO cell, COS cell, NSO cell, HeLa cell, BHK cell, HEK 293 cell or human cell.

As used herein, the term "identity" is used to refer to the match of sequences between two polypeptides or between two nucleic acids. When a position in both sequences being compared is occupied by the same base or amino acid monomer subunit (e.g., a position in each of the two DNA molecules is occupied by an adenine, or a position in each of the two polypeptides is occupied by lysine), then the molecules are identical at that position. "Percent identity" between two sequences is a function of the number of matching positions shared by the two sequences divided by the number of positions being compared x 100. For example, two sequences have an identity of 60% if 6 out of 10 positions match. For example, the DNA sequences CTGACT and CAGGTT have an identity of 50% (3 out of a total of 6 positions match). Typically, the comparison is made when two sequences are aligned for maximum identity. Such alignment can be achieved by using, for example, the method of Needleman et al. (1970) J. Mol. Biol. 48:443-453, which can be conveniently performed by computer programs such as the Align program (DNAstar, Inc.). The algorithm of E. Meyers and W. Miller (Comput. Appl Biosci., 4:11-17 (1988)) which has been incorporated into the ALIGN program (version 2.0) can also be used to determine the percent identity between two amino acid sequences, by using the PAM120 weight residue table, a gap length penalty of 12, and a gap penalty of 4. In addition, the algorithm of Needleman and Wunsch (J Mol Biol. 48:444-453 (1970)) that has been integrated into the GAP program of GCG software package (available at www.gcg.com) can be used to determine the percent identity between two amino acid sequences, by using the Blossum 62 matrix or PAM250 matrix, a gap weight of 16, 14, 12, 10, 8, 6 or 4, and a length weight of 1, 2, 3, 4, 5 or 6.

As used herein, the term "conservative substitution" refers to an amino acid substitution that does not adversely affect or alter the expected properties of a protein/polypeptide comprising the amino acid sequence. For example, a conservative substitution can be introduced by standard techniques known in the art, such as site-directed mutagenesis and PCR-mediated mutagenesis. Conservative amino acid substitutions include substitutions for amino acid residues with amino acid residues that have similar side chains, e.g., substitutions for amino acid residues with residues that are physically or functionally similar (e.g., having similar size, shape, charge, chemical properties, including ability to form covalent or hydrogen bonds, etc.). Families of amino acid residues having similar side chains have been defined in the art. These families include amino acids with basic side chain (e.g., lysine, arginine, and histidine), acidic side chain (e.g., aspartic acid, glutamic acid), uncharged polar side chain (e.g., glycine, asparagine, glutamine, serine, threonine, tyrosine, cysteine, tryptophan), non-polar side chain (e.g., alanine, valine, leucine, isoleucine amino acid, proline, phenylalanine, methionine), β branched side chain (e.g., threonine, valine, isoleucine) and aromatic side chain (e.g., tyrosine, phenylalanine, tryptophan, histidine). Therefore, it is preferred to replace the corresponding amino acid residue with another amino acid residue from the same side chain family. Methods for identifying amino acid conservative substitution are well known in the art (see, for example, Brummell et al., Biochem. 32:1180-1187 (1993); Kobayashi et al. Protein Eng. 12(10):879-884 (1999); and Burks et al. Proc. Natl Acad. Set USA 94:412-417 (1997), which are incorporated herein by reference).

The writing of the twenty conventional amino acids referred to herein follows conventional usage. See, for example, Immunology-A Synthesis (2nd Edition, E. S. Golub and D. R. Gren, Eds., Sinauer Associates, Sunderland, Mass. (1991)), which is incorporated herein by reference. In the present invention, the terms "polypeptide" and "protein" have the same meaning and are used interchangeably. And in the present invention, amino acids are generally represented by single-letter and three-letter abbreviations known in the art. For example, alanine can be represented by A or Ala.

As used herein, the term "pharmaceutically acceptable carrier and/or excipient" refers to a carrier and/or excipient pharmacologically and/or physiologically compatible with a subject and an active ingredient, which is well known in the art (see, for example, Remington's Pharmaceutical Sciences. Edited by Gennaro AR, 19th ed. Pennsylvania: Mack Publishing Company, 1995), and includes, but is not limited to: pH adjuster, surfactant, adjuvant, ionic strength enhancer, diluent, agent for maintaining osmotic pressure, agent for delaying absorption, preservative. For example, the pH adjuster includes, but is not limited to, phosphate buffer. The surfactant includes but is not limited to cationic, anionic or nonionic surfactant such as Tween-80. The ionic strength enhancer includes, but is not limited to, sodium chloride. The preservative includes, but is not limited to, various antibacterial and antifungal agents, such as paraben, chlorobutanol, phenol, sorbic acid, and the like. The agent for maintaining osmotic pressure includes, but is not limited to, saccharide, NaCl, and the like. The agent for delaying absorption includes, but is not limited to, monostearate and gelatin. The diluent includes, but is not limited to, water, aqueous buffer (e.g., buffered saline), alcohol and polyol (e.g., glycerol), and the like. The preservative includes, but is not limited to, various antibacterial and antifungal agents, such as thimerosal, 2-phenoxyethanol, paraben, chlorobutanol, phenol, sorbic acid, and the like. The stabilizer has the meaning generally understood by those skilled in the art, and it can stabilize the desired activity of the active ingredient in the medicine, including but not limited to sodium glutamate, gelatin, SPGA, saccharide (e.g., sorbitol, mannitol, starch, sucrose, lactose, dextran, or glucose), amino acid (e.g., glutamic acid, glycine), protein (e.g., dry whey, albumin or casein) or degradation product thereof (e.g., lactalbumin hydrolyzate), etc. In certain exemplary embodiments, the pharmaceutically acceptable carrier or excipient comprises a sterile injectable liquid (e.g., an aqueous or non-aqueous suspension or solution). In certain exemplary embodiments, such sterile injectable liquid is selected from water for injection (WFI), bacteriostatic water for injection (BWFI), sodium chloride solution (e.g., 0.9% (w/v) NaCl), dextrose solution (e.g., 5% dextrose), surfactant-contained solution (e.g., 0.01% polysorbate 20), pH buffered solution (e.g., phosphate buffered saline), Ringer's solution, and any combination thereof.

As used herein, the term "prevention" refers to a method that is performed to prevent or delay the occurrence of a disease or disorder or symptom (e.g., EBV infection) in a subject. As used herein, the term "treatment" refers to a method that is performed to obtain a beneficial or desired clinical result. For the purposes of the present invention, a beneficial or desired clinical outcome includes, but is not limited to, alleviation of symptom, reduction of of disease extent, stabilization (i.e., no longer worsening) of disease state, delay or slowing of disease progression, amelioration or palliation of disease state, and relief of symptom (whether partial or total), whether detectable or undetectable. In addition, "treatment" can also refer to prolonging survival as compared to expected survival if not receiving treatment.

As used herein, the term "subject" refers to a mammal, such as a human. In certain embodiments, the subject (e.g., human) has, or is at risk of having, EBV infection or a disease associated with EBV infection.

As used herein, the term "effective amount" refers to an amount sufficient to achieve, or at least partially achieve, the desired effect. For example, an effective amount for preventing a disease (e.g., EBV infection) refers to an amount sufficient to prevent, arrest, or delay the occurrence of a disease (e.g., EBV infection); an effective amount for treating a disease refers to an amount sufficient to cure or at least partially prevent an existing disease or complication thereof in a patient who has been suffered from the disease. Determining such an effective amount is well within the capability of those skilled in the art. For example, the effective amount for therapeutic use will depend on the severity of the disease to be treated, the general state of the patient's own immune system, the general condition of the patient such as age, weight and sex, the mode of administration of drug, and other treatment administered concomitantly etc.

As used herein, the term "neutralizing activity" refers to a functional activity of the antibody or antibody fragment to bind to an antigenic protein on virus, thereby preventing the virus from infecting the cells and/or the maturation of virus progeny and/or the release of virus progeny, and the antibody or antibody fragment with neutralizing activity can prevent the amplification of the virus, thereby inhibiting or eliminating the infection of the virus.

Beneficial effects of the present invention

The epitope peptide and the recombinant protein of the present invention can induce an antibody response to effectively eliminate EBV and EBV-infected cells in vivo, and have the potential to be developed into an EBV vaccine. The present invention also provides a monoclonal antibody and antigen-binding fragment thereof capable of specifically recognizing/binding to the epitope peptides of the present invention. Such monoclonal antibody and antigen-binding fragment thereof can neutralize EBV efficiently and inhibit EBV from being fused with cells, and thus can be used for the prevention and/or treatment of EBV infection or a disease caused by EBV infection. In addition, the antibody of the present invention can also be used for specific detection of EBV or gB protein thereof, and has clinical value for the diagnosis of EBV infection.

The embodiments of the present invention will be described in detail below with reference to the drawings and examples, but those skilled in the art will understand that the following drawings and examples are only for illustrating the present invention, rather than limiting the scope of the present invention. Various objects and advantages of the present invention will become apparent to those skilled in the art from the accompanying drawings and the following detailed description of the preferred embodiment.

**Brief Description of the Drawings**

Fig. 1 shows the SDS-PAGE identification results of gB protein ectodomain after denaturation treatment and non-denaturation treatment.

Fig. 2 shows the antibody titer detection results of rabbit immune serum induced by gB protein.

Fig. 3 shows the ELISA reaction results of monoclonal antibodies 3A3, 3A5, AMMOS with gB protein.

Fig. 4 shows the Western Blot reaction results of monoclonal antibodies 3A3, 3A5, AMMOS with gB protein.

Fig. 5 shows the affinity constant determination results of monoclonal antibodies 3A3, 3A5, AMMOS with gB protein.

Fig. 6 shows the immunofluorescence results of monoclonal antibodies 3A3, 3A5, AMMOS with MDCK cells expressing gB protein.

Fig. 7 shows the neutralization results of monoclonal antibodies 3A3, 3A5, AMMOS and 1E12 in epithelial cell infection model and B cell infection model.

Fig. 8 shows the results of monoclonal antibodies 3A3, 3A5, AMMOS and control antibody 1E12 in assays for blocking cell fusion.

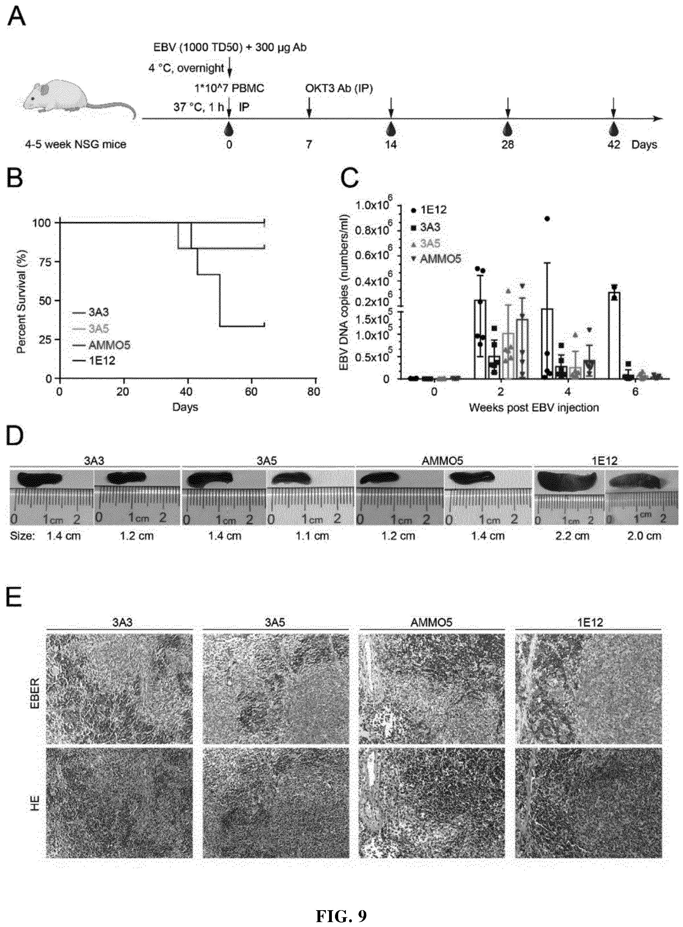

Fig. 9 shows the protective effects of monoclonal antibodies 3A3, 3A5, AMMOS and control antibody 1E12 in an animal model. Among them, Fig. 9A shows a schematic diagram of treatment process of the animal model; Fig. 9B shows the survival rate of mice in each antibody treatment group; Fig. 9C shows the EBV DNA copy number in the peripheral blood of mice in each antibody treatment group; Fig. 9D shows the spleen size after euthanasia of the mice in each antibody treatment group; Fig. 9E shows the EBER in situ hybridization detection results of the spleen tissue section of the mice in each antibody treatment group.

Fig. 10 shows the epitope competition results between each of monoclonal antibodies 3A3, 3A5, AMMOS and 72A1.

Fig. 11 shows the Western Blot reaction results of monoclonal antibodies 3A3 (Fig. 11A), 3A5 (Fig. 11B) with wild-type (WT) or each point mutant gB protein.

Fig. 12 shows the ELISA reaction results of monoclonal antibodies 3A3 (Fig. 12A), 3A5 (Fig. 12B) with wild-type (WT) or each point mutant gB protein.

Fig. 13 shows the Western Blot reaction results of monoclonal antibodies 3A3 and 3A5 with different truncated epitopes of gB protein.

Fig. 14 shows the location of epitopes recognized by monoclonal antibodies 3A3 and 3A5 in the 3D structure of gB protein.

Fig. 15 shows the Western Blot reaction results of monoclonal antibodies 3A3, 11H10 with HBc149 protein chimerized with gB-331-367aa epitope.

Fig. 16 shows the ELISA reaction results of monoclonal antibodies 3A3, 11H10 with HBc149 protein chimerized with gB-331-367aa epitope.

Fig. 17 shows the HPLC identification results of HBc149 virus-like particle chimerized with gB-331-367aa epitope (149-αB) and simple HBc149 virus-like particle (149-WT).

Fig. 18 shows the transmission electron microscopy results of HBc149 virus-like particle chimerized with gB-331-367aa epitope (149-αB) and wild-type HBc149 virus-like particle (149-WT).

Fig. 19 shows the ELISA detection results of the binding titer of rabbit sera immunized with HBc149 virus-like particle chimerized with gB-331-367aa epitope (149-αB) and wild-type HBc149 virus-like particle (149-WT).

Fig. 20 shows the detection results of the neutralization titer of rabbit sera immunized with HBc149 virus-like particle chimerized with gB-331-367aa epitope (149-αB) and wild-type HBc149 virus-like particle (149-WT).

Fig. 21 shows the neutralization results of the mixed antibody (3A3+3A5), and monoclonal antibodies 3A3, 3A5, AMMOS and 1E12 in the B cell infection model.

Fig. 22 shows the flow chart of the protection experiment of 3A3 chimeric antibody, 3A5 chimeric antibody, mixed antibody (3A3 chimeric antibody+3A5 chimeric antibody), AMMOS and control antibody VRC01 in the humanized mouse model.

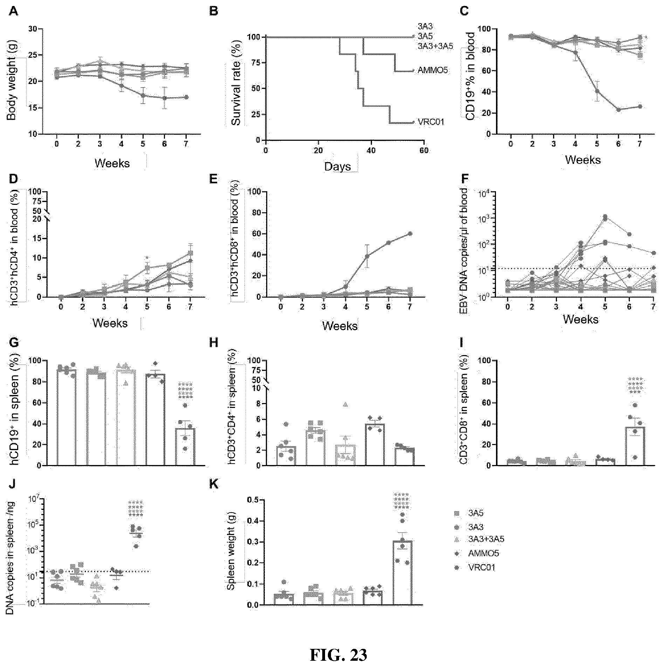

Fig. 23 shows the protective effect of 3A3 chimeric antibody, 3A5 chimeric antibody, mixed antibody (3A3 chimeric antibody+3A5 chimeric antibody), AMMOS and control antibody VRC01 in the humanized mouse model. Wherein, Fig. 23A shows the body weight change of mice in each antibody treatment group; Fig. 23B shows the survival rate of mice in each antibody treatment group; Fig. 23C shows the change of proportion of hCD19+B cells in the peripheral blood of mice in each antibody treatment group; Fig. 23D shows the change of proportion of hCD3+hCD4+ double-positive T cells in the peripheral blood of mice in each antibody treatment group; Fig. 23E shows the change of proportion of hCD3+hCD8+ double-positive T cells in the peripheral blood of mice in each antibody treatment group; Fig. 23F shows the EBV DNA copy number in the peripheral blood of mice in each antibody treatment group; Fig. 23G shows the proportion of hCD9+ B cells in the spleen after euthanasia of mice in each antibody treatment group; Fig. 23H shows the proportion of hCD3+hCD4+ double-positive T cells in the spleen after euthanasia of mice in each antibody treatment group; Fig. 23I shows the proportion of hCD3+hCD8+ double-positive T cells in the spleen after euthanasia of mice in each antibody treatment group; Fig. 23J shows the EBV DNA copy number in the spleen after euthanasia of mice in each antibody treatment group; Fig. 23K shows the comparison of spleen weights after euthanasia of mice in each antibody treatment group.

Fig. 24 shows the results of EBER in situ hybridization detection, hCD20 staining and H&E staining of spleen tissue sections of mice after euthanasia in each antibody treatment group.

Sequence information

Information on the partial sequences involved in the present invention is provided in Table 1 below.

Table 1: Description of the sequences

| SEQ ID NO | Description | Sequence information |
|---|---|---|
| 1 | 3A3 VH | QSVKESGGRLVTPGTPLTLTCTVSGFSLSSYAMSWVRQAPGKGLEYIGVIYASGSTYYASWAKGRFTISRTATTVDLKITSPTTEDTATYFCGRGVSTNMWGPGTLVTVSS |
| 2 | 3A3 VL | DLVMTQTPSSVSAAVGGTVTIKCQASQSLGGGLAWYQQKPGQRPKLLIYSASTLESGVPSRFRGSGSGTEFTLTISDLECADAATYYCQSAYGPTSNGLFNAFGGGTKVVIK |
| 3 | 3A3 CDR-H1 | GFSLSSYA |
| 4 | 3A3 CDR-H2 | IYASGST |
| 5 | 3A3 CDR-H3 | GRGVSTNM |
| 6 | 3A3 CDR-L1 | QSLGGG |
| 7 | 3A3 CDR-L2 | SAS |
| 8 | 3A3 CDR-L3 | QSAYGPTSNGLFNA |
| 9 | 3A5 VH | QSVKESGGRLVTPGTPLTLTCTVSGFSLSSYEMGWVRQAPGEGLEWIGTISTGGSSYYASWAKGRFTISRTSTTVDLKMTSLTTADTATYFCARGYGGYGIGAGYFNIWGPGTLVTVSS |
| 10 | 3A5 VL | ALVMTQTPSSVSEPVGGTVTIKCQASQSISSYLAWYQRKPGQRPKLLIYGTSTLASGVPSRFIGSGSGTDYTLTISDLECDDAATYYCQQGFSTSNVYNSFGGGTKVDIK |

(continued)

| SEQ ID NO | Description | Sequence information |
|---|---|---|
| 11 | 3A5 CDR-H1 | GFSLSSYE |
| 12 | 3A5 CDR-H2 | ISTGGSS |
| 13 | 3A5 CDR-H3 | ARGYGGYGIGAGYFNI |
| 14 | 3A5 CDR-L1 | QSISSY |
| 15 | 3A5 CDR-L2 | GTS |
| 16 | 3A5 CDR-L3 | QQGFSTSNVYNS |
| 17 | Amino acid sequence of EBV M81 strain gB protein | MTRRRVLSVVVLLAALACRLGAQTPEQPAPPATTVQPTATRQQ TSFPFRVCELSSHGDLFRFSSDIQCPSFGTRENHTEGLLMVFKDNI IPYSFKVRSYTKIVTNILIYNGWYADSVTNRHEEKFSVESYETDQ MDTIYQCYNAVKMTKDGLTRVYVDRDGVNITVNLKPTGGLAN GVRRYASQTELYDAPGWLIWTYRTRTTVNCLITDMMAKSNSPF DFFVTTTGQTVEMSPFYDGKNTETFHERADSFHVRTNYKIVDY DNRGTNPQGERRAFLDKGTYTLSWKLENRTAYCPLQHWQTFDS TIATETGKSIHFVTDEGTSSFVTNTTVGIELPDAFKCIEEQVNKT MHEKYEAVQDRYTKGQEAITYFITSGGLLLAWLPLTPRSLATVK NLTELTTPTSSPPSSPSPPAPPAARGSTSAAVLRRRRRNAGNATT PVPPAAPGKSLGTLNNPATVQIQFAYDSLRRQINRMLGDLARA WCLEQKRQNMVLRELTKINPTTVMSSIYGKAVAAKRLGDVISV SQCVPVNQATVTLRKSMRVPGSETMCYSRPLVSFSFINDTKTYE GQLGTDNEIFLTKKMTEVCQATSQYYFQSGNEIHVYNDYHHFK TIELDGIATLQTFISLNTSLIENIDFASLELYSRDEQRASNVFDLEG IFREYNFQAQNIAGLRKDLDNAVSNGRNQFVDGLGELMDSLGS VGQSITNLVSTVGGLFSSLVSGFISFFKNPFGGMLILVLVVGVVIL VISLTRRTRQMSQQPVQMLYPGIDELAQQHASGEGPGINPISKTE LQAIMLALHEQNEQKRAAQRAAGPSVASRALQAARDRFPGLR RRYHDPETAAALLGEAETEF |
| 18 | EBV gB protein aa341-362 | KCIEEQVNKTMHEKYEAVQDRY |
| 19 | EBV gB protein aa331-367 | TVGIELPDAFKCIEEQVNKTMHEKYEAVQDRYTKGQE |
| 20 | EBV gB protein aa528-616 | CVPVNQATVTLRKSMRVPGSETMCYSRPLVSFSFINDTKTYEGQLG TDNEIFLTKKMTEVCQATSQYYFQSGNEIHVYNDYHHFKTIEL |
| 21 | Carrier protein HBc149/mut | MDIDPYKEFGASVELLSFLPSDFFPSIRDLLDTASALYREALESPEHC SPHHTALRQAILCWGELMNLATWVGSNLEDGGGGSGGGGTG**SEF G**GGGSGGGGSRELVVSYVNVMGLKIRQLLWFHISCLTFGRETVL EYLVSFGVWIRTPPAYRPQNAPILSTLPETTVV |
| 22 | Recombination protein gB aa331-367+ HBc149/mut | MDIDPYKEFGASVELLSFLPSDFFPSIRDLLDTASALYREALESPEHC SPHHTALRQAILCWGELMNLATWVGSNLEDGGGGSGGGGTGSET VGIELPDAFKCIEEQVNKTMHEKYEAVQDRYTKGQEFGGGGSGGG GSRELVVSYVNVMGLKIRQLLWFHISCLTFGRETVLEYLVSFGV WIRTPPAYRPQNAPILSTLPETTVV |

(continued)

| SEQ ID NO | Description | Sequence information |
|---|---|---|
| 23 | Amino acid sequence of EBV gB protein after amino acid substitution in Example 1 | MTRRRVLSVVVLLAALACRLGAQTPEQPAPPATTVQPTATRQQ TSFPFRVCELSSHGDLFRFSSDIQCPSFGTRENHTEGLLMVFKDNI IPYSFKVRSYTKIVTNILIYNGHRADSVTNRHEEKFSVESYETDQ MDTIYQCYNAVKMTKDGLTRVYVDRDGVNITVNLKPTGGLAN GVRRYASQTELYDAPGRVEATYRTRTTVNCLITDMMAKSNSPF DFFVTTTGQTVEMSPFYDGKNTETFHERADSFHVRTNYKIVDY DNRGTNPQGERRAFLDKGTYTLSWKLENRTAYCPLQHWQTFDS TIATETGKSIHFVTDEGTSSFVTNTTVGIELPDAFKCIEEQVNKT MHEKYEAVQDRYTKGQEAITYFITSGGLLLAWLPLTPRSLATVK NLTELTTPTSSPPSSPSPPAPPAARGSTSAAVLRRRRRNAGNATT PVPPAAPGKSLGTLNNPATVQIQFAYDSLRRQINRMLGDLARA WCLEQKRQNMVLRELTKINPTTVMSSIYGKAVAAKRLGDVISV SQCVPVNQATVTLRKSMRVPGSETMCYSRPLVSFSFINDTKTYE GQLGTDNEIFLTKKMTEVCQATSQYYFQSGNEIHVYNDYHHFK TIELDGIATLQTFISLNTSLIENIDFASLELYSRDEQRASNVFDLEG IFREYNFQAQNIAGLRKDLDNAVSNGRNQFVDGLGELMDSLGS VGQSITNLVSTVGGLFSSLVSGFISFFKNPFGGMLILVLVVGVVIL VISLTRRTRQMSQQPVQMLYPGIDELAQQHASGEGPGINPISKTE LQAIMLALHEQNEQKRAAQRAAGPSVASRALQAARDRFPGLR RRRYHDPETAAALLGEAETEF |
| 24 | gB ectodomain in Example 1 (aa24-683) | TPEQPAPPATTVQPTATRQQTSFPFRVCELSSHGDLFRFSSDIQCPSF GTRENHTEGLLMVFKDNIIPYSFKVRSYTKIVTNILIYNGHRADSVT NRHEEKFSVESYETDQMDTIYQCYNAVKMTKDGLTRVYVDRDGV NITVNLKPTGGLANGVRRYASQTELYDAPGRVEATYRTRTTVNCLI TDMMAKSNSPFDFFVTTTGQTVEMSPFYDGKNTETFHERADSFHV RTNYKIVDYDNRGTNPQGERRAFLDKGTYTLSWKLENRTAYCPLQ HWQTFDSTIATETGKSIHFVTDEGTSSFVTNTTVGIELPDAFKCIEEQ VNKTMHEKYEAVQDRYTKGQEAITYFITSGGLLLAWLPLTPRSLAT VKNLTELTTPTSSPPSSPSPPAPPAARGSTSAAVLRRRRRNAGNATT PVPPAAPGKSLGTLNNPATVQIQFAYDSLRRQINRMLGDLARAWC LEQKRQNMVLRELTKINPTTVMSSIYGKAVAAKRLGDVISVSQCVP VNQATVTLRKSMRVPGSETMCYSRPLVSFSFINDTKTYEGQLGTDN EIFLTKKMTEVCQATSQYYFQSGNEIHVYNDYHHFKTIELDGIATL QTFISLNTSLIENIDFASLELYSRDEQRASNVFDLEGIFREYNFQAQN IAGLRKDLDNAVS |
| 25 | Primer | GGTCACAATCTCCACGCTGA |
| 26 | Primer | CAACGAGGCTGACCTGATCC |
| 27 | Nucleotide sequence of carrier protein HBc149/mut | ATGGACATTGACCCTTATAAAGAATTTGGAGCTTCTGTGGAGTT ACTCTCTTTTTTGCCTTCCGACTTCTTTCCTTCTATTCGAGATCTC CTCGACACCGCCTCTGCTCTGTATCGGGAGGCCTTAGAGTCTCC GGAACATTGTTCACCTCACCATACGGCACTCAGGCAAGCTATTC TGTGTTGGGGTGAGTTGATGAATCTAGCCACCTGGGTGGGAAGT AATTTGGAAGATGGTGGAGGTGGTTCTGGAGGTGGTGGTACT**GATCCGAATTC**GGTGGTGGAGGTTCAGGAGGAGGTGGTTCCAG GGAACTAGTAGTCAGCTATGTCAACGTTAATATGGGCCTAAAAA TCAGACAACTATTGTGGTTTCACATTTCCTGTCTTACTTTTGGGA GAGAAACTGTTCTTGAATATTGGTGTCTTTTGGAGTGTGGATTC GCACTCCTCCTGCATATAGACCACAAAATGCCCCTATCTTATCA ACACTTCCGGAAACTACTGTTGTT |

(continued)

| SEQ ID NO | Description | Sequence information |
|---|---|---|
| 28 | VH nucleotide sequence of control antibody 1E12 | CAGTCAGTGAAGGAGTCCGGGGGTCGCCTGGTCACGCCTGGGA CACCCCTGACACTCACCTGCACCGTCTCTGGAATCGACCTCAGT ACCTATGTAATGACTTGGGTCCGCCAGGCTCCAGGGAAGGGGCT GGAATGCATCGGAATCATTGGTTATGGTGGTAGCACATACTACG CGAGCTGGGCGACAGGCCGATTCACCATCTCCAAAACCTCGACC ACGGTGGATCTGAGAATGACCAGTCTGACAACCGAGGACACGG CCACCTATTTCTGTGCCAGAGGTGTTAGTAGTAATCTTTATAGG GGAATGAATTTGTGGGGCCAAGGCACCCTGGTCACCGTCTCTTC A |
| 29 | VL nucleotide sequence of control antibody 1E12 | GCCCTCGTGATGACCCAGACTCCATCTCCCGTGTCTGCAGCTGT GGGAGGCACAGTCAGCATCAGTTGCCAGTCCAGTCCGAGTGTTT ATAGTAATTACTTATCCTGGTATCAGCAGAAACCAGGGCAGCCT CCCAAGCTCCTGATCTACGAAACATCCAAACTGGAATCTGGGGT CCCATCGCGGTTCAGCGGCAGTGGATCTGGGACACAGTTCACTC TCACCATCAGCGGCGTGCAATGTGACGATGCTGCCACTTACTAC TGTGCAGGCGGTTATAGTGGTATTAGTGATACGTTTGCTTTCGG CGGAGGGACCAAGGTGGACATCAAA |
| 30 | Human heavy chain constant region sequence | ASTKGPSVFPLAPSSKSTSGGTAALGCLVKDYFPEPVTVSWNSGAL TSGVHTFPAVLQSSGLYSLSSVVTVPSSSLGTQTYICNVNHKPSNTK VDKKVEPKSCDKTHTCPPCPAPELLGGPSVFLFPPKPKDTLMISRTP EVTCVVVDVSHEDPEVKFNWYVDGVEVHNAKTKPREEQYNSTYR VVSVLTVLHQDWLNGKEYKCKVSNKALPAPIEKTISKAKGQPREP QVYTLPPSRDELTKNQVSLTCLVKGFYPSDIAVEWESNGQPENNYK TTPPVLDSDGSFFLYSKLTVDKSRWQQGNVFSCSVMHEALHNHYT QKSLSLSPGK |
| 31 | Human light chain constant region sequence | RTVAAPSVFIFPPSDEQLKSGTASVVCLLNNFYPREAKVQWKVDSA LQSGNSQESVTEQDSKDSTYSLSSTLTLSKADYEKHKVYACEVTHQ GLSSPVTKSFNRGEC |
| 32 | Control antibody VRC01-VH | QVQLVQSGGQMKKPGESMRISCRASGYEFIDCTLNWIRLAPGKRPE WMGWLKPRGGAVNYARPLQGRVTMTRDVYSDTAFLELRSLTVD DTAVYFCTRGKNCDYNWDFEHWGRGTPVIVSS |
| 33 | Control antibody VRC01-VL | EIVLTQSPGTLSLSPGETAIISCRTSQYGSLAWYQQRPGQAPRLVIYS GSTRAAGIPDRFSGSRWGPDYNLTISNLESGDFGVYYCQQYEFFGQ GTKVQVDIKR |

## EXAMPLES

The present invention will now be described with reference to the following examples, which are intended to illustrate the present invention, but not to limit it.

Unless otherwise specified, the molecular biology experiment methods and immunoassay methods used in the present invention are basically referred to the methods described by J. Sambrook et al., Molecular Cloning: A Laboratory Manual, 2nd Edition, Cold Spring Harbor Laboratory Press, 1989, and F. M. Ausubel et al., Molecular Biology Experimental Guide, 3rd Edition, John Wiley & Sons, Inc., 1995; the restriction enzymes were used in accordance with the conditions recommended by the product manufacturer. Those skilled in the art understand that the examples describe the present invention by way of example and are not intended to limit the scope of the present invention.

Example 1: Preparation of anti-EBV gB protein rabbit monoclonal antibody

1.1 Preparation of EBV gB protein

The WY[112-113] and WLIW[193-196] in the amino acid sequence (SEQ ID NO: 17) of gB protein of the EBV M81 strain were substituted with HR[177-178] and RVEA[258-261] in the amino acid sequence of HSV-1 gB protein, respectively, to avoid the polymorphism of the expressed protein. The amino acid sequence of the substituted gB protein was set forth in SEQ ID NO:23, and its ectodomain (24-683aa) was set forth in SEQ ID NO:24.

The substituted gB ectodomain (24-683aa) coding sequence was constructed on the pCDNA3.1 expression vector that could be used for eukaryotic expression by using the Gibson assembly method, and the C-terminal of the protein had a 6xHis tag protein sequence for affinity chromatography purification. The successfully constructed recombinant plasmid was transfected into 293F cells for expression and purification. The SDS-PAGE electrophoresis results showed that the trimer of gB protein was larger than 300KDa, and the monomer size was about 110KDa. After denaturation treatment with mercaptoethanol, the disulfide bond at the furin cleavage site of the protein was opened, forming 2 subunits with size of 70KDa and 40KDa, respectively.

1.2 New Zealand rabbits

10-week-old female New Zealand rabbits were purchased from Songlian Experimental Animal Farm, Songjiang District, Shanghai.

1.3 Immunization of experimental rabbits

A standard in vivo immunization method as detailed in Ed Harlow et al., "antibody A Laboratory Manual", Cold Spring Harbor Laboratory 1988 was used. The brief procedure was as follows:
500 μg of the EBV gB ectodomain (SEQ ID NO: 24) purified in the above step 1.1 was mixed with equal volume of complete Freund's adjuvant (CFA) (purchased from SIGMA-ALDRICH, Item No.: F5881) to obtain 2 mL of emulsion, which was administrated to the experimental rabbits through multi-point injection at neck and back. On the 14th and 28th days after the first immunization, 500 μg of the EBV gB protein purified in the above step 1.1 was mixed with equal volume of incomplete Freund's adjuvant (IFA) (purchased from SIGMA-ALDRICH, Item No.: F5506) to obtain 2 mL of emulsion, which was administrated to the experimental rabbits through multiple point injection at neck and back. On the 42nd day after the first immunization, the whole blood was collected through the central artery of rabbit ear, the serum was separated, and the antibody titer against gB protein was determined.

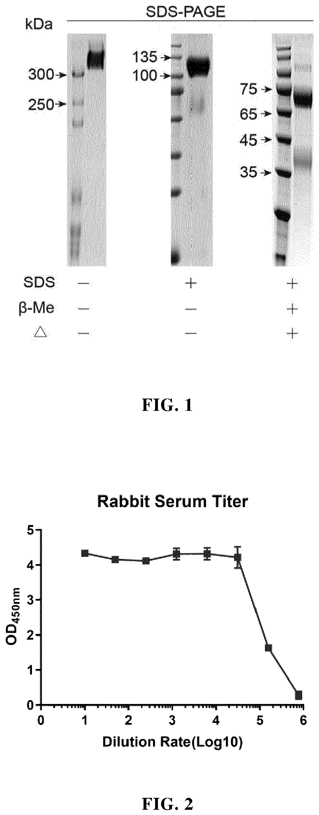

The results of rabbit serum antibody titer determination were shown in Fig. 2, and the results showed that the serum titer was above 10^6.

1.4 Preparation of rabbit peripheral blood mononuclear cells (PBMC)

The rabbit whole blood was diluted with serum-free RPMI1640 medium at a ratio of 1:1, peripheral blood mononuclear cells were separated by density gradient centrifugation using Ficoll reagent, Ficoll solution in a volume 1.5 times that of the rabbit whole blood was added to the bottom of the centrifuge tube, and then the diluted rabbit whole blood was added slowly, ramp-up centrifugation was performed at 800g, 30min, 4°C, after the centrifugation was completed, the suspended cells at the interface between Ficoll and RPMI1640 medium were collected as PBMC, and the PBMC cells were subjected to second centrifugation at 1500rpm, 5min, 4°C, and then collected.

1.5 Specific B cells screening for anti-EBV gB

The full-length gene sequence of rabbit CD40L was constructed into pLV CS2.0 lentiviral vector and subjected to lentivirus package, and used for infection of 293T cells, and a stable cell line 293T-rCD40L overexpressing rabbit CD40L was constructed as feeder cells for follow-up culturing of rabbit B cells in vitro.

The isolated rabbit PBMC cells were resuspended in 100 μL of sterile PBS solution, and the biotin-labeled gB protein (SEQ ID NO: 24) in corresponding amount was added according to the standard of 1 μg of the protein per 3 ml of rabbit whole blood PBMC, mixed gently by pipetting, and incubated at 4°C for 30min. Centrifugation was performed at 1500rpm for 3min at 4°C, the supernatant was discarded and the cells were retained, the cells were resuspended in PBS and washed 2-3 times, and 100μL of the staining system shown in the table below was added to each tube, and incubated at 4°C in the dark for 30 min.

Table 2:

| Dye | Addition amount |
|---|---|
| live/dead aqua (purchased from Molecular Probe, Item No.: L34957) | 1μL |
| CD4-FITC (purchased from Bio-Rad, Item No.: MCA799F) | 5μL |
| CD8-FITC (purchased from Bio-Rad, Item No.: MCA1576F) | 5μL |
| T Lymphocytes-FITC (purchased from Bio-Rad, Item No.: MCA800F) | 5μL |
| IgM-RPE (purchased from Bio-Rad, Item No.: MCA812PE) | 5μL |
| IgG-BV421 (purchased from Biolegend, Item No.: 406410) | 1μL |
| APC (purchased from eBioscience, Item No.: 17-4317-82) | 1μL |
| PBS | Supplement to 100μL |

In a 96-well cell culture plate, pre-screened 293T-rCD40L cells were spread, $2 \times 10^4$ cells/well, and each well contained 200 μL of RPMI1640 medium formulated with 10% FBS and 20 ng/mL human interleukin 2, 50 ng/mL human interleukin 21. The B cells were sorted into corresponding cell plates according to the standard of 2 cells per well. The culturing was carried out in an incubator at 37°C and 5% $CO_2$ for 7 days, and positive cell wells with specific reactivity to EBV-gB protein were screened by indirect ELISA method.

The cell supernatant of the positive cell wells was discarded, 300 μL of lysis solution was added to each well, the lysate was pipetted into a PCR plate to perform reverse transcription, and then the antibody light and heavy chain variable region sequences were amplified by nested PCR, respectively.

The PCR product of the paired heavy chain and light chain was picked out, recovered with a nucleic acid recovery kit, and sequenced. The sequencing results were sent to IMGT database (http://www.imgt.org/) for comparison to determine whether the obtained gene was an antibody gene, whether the gene was complete, whether it could successfully encode an antibody, and determine the family to which the antibody gene (V region and J region) belonged.

The cloning vector was digested, the restriction site of the heavy chain expression plasmid vector was EcoR I/BamH I, and the restriction site of the light chain expression plasmid vector was Nhe I/Sal I. The light and heavy chain variable region genes were constructed into the corresponding eukaryotic expression vectors pRVRCL and pRVRCH by Gibson assembly method to obtain IgK plasmid and IgH plasmid, respectively. Wherein, pRVRCH contained the nucleic acid sequence encoding the heavy chain constant region of the rabbit monoclonal antibody, and pRVRCL contained the nucleic acid sequence encoding the light chain constant region of the rabbit monoclonal antibody.

After the expression vector was constructed, transient transfection of HEK293T cells were performed by liposome method to express the rabbit monoclonal antibody against EBV-gB protein. 12 hours before the transfection, $10^4$ cells were inoculated into a 96-well cell culture plate; tube A: 0.2 μg of IgH plasmid and 0.2 μg of IgK plasmid were added to 10 μL of Opti-MEM; tube B: 0.4 μL of Novozyme ExFect®2000 Transfection Reagent was added to 10 μL of Opti-MEM. The contents of tube A and tube B were gently mixed, respectively, allowed to stand at room temperature for 5 min, then the diluted plasmid was added dropwise to the diluted transfection reagent, mixed gently, and incubated at room temperature for 10 min. The plasmid-transfection reagent complex was added dropwise to the cells and cultured in a cell incubator; after 48 hours, the cell supernatant was collected, centrifuged at 3000 rpm for 5 min at 4°C, the supernatant was taken, and the cell debris pellet was discarded. The reactivity of transfected antibody with gB protein was determined by indirect ELISA method.

1.6 Sequence determination of rabbit monoclonal antibody against EBV gB protein

Two rabbit monoclonal antibodies 3A3 and 3A5 specific to EBV-gB protein were obtained by the above method. After sequencing, the amino acid sequences of the heavy chain variable regions and light chain variable regions of 3A3 and 3A5 were determined as follows, and their CDR region sequences were analyzed through the IMGT database (http://www.imgt.org/). The amino acid sequences of the heavy chain variable regions and light chain variable regions of 3A3 and 3A5 and the corresponding CDR regions were shown in the table below.

Table 3: Variable regions and CDR sequences

| mAb | SEQ ID NO: | | | | | | | |
|---|---|---|---|---|---|---|---|---|
| | VH | VL | CDR-H1 | CDR-H2 | CDR-H3 | CDR-L1 | CDR-L2 | CDR-L3 |
| 3A3 | 1 | 2 | 3 | 4 | 5 | 6 | 7 | 8 |
| 3A5 | 9 | 10 | 11 | 12 | 13 | 14 | 15 | 16 |

1.7 Preparation of rabbit monoclonal antibody against EBV gB protein

For massive expression of 3A3 and 3A5 monoclonal antibodies, suspension cells 293F in logarithmic growth phase were prepared, placed on a cell shaker and cultured at 100rpm, 37°C, 5% $CO_2$ until the density was $1.5\times10^6$/mL, and the cell viability was >95%. 400 mL of the cells were taken and placed in a new cell culture flask as a transfection system. Tube A: 600 $\mu$g of plasmids expressing 3A3 and 3A5 light and heavy chains were separately added to 20 mL of suspension cell culture medium, shaken and mixed well; Tube B: 1.2 mg of PEI transfection reagent was added to 20 mL of suspension cell culture medium, shaken and mixed well. The solution in tube B was added to tube A, shaken and mixed well and incubated at room temperature for 15 min, then the mixed solution was added to a 400mL cell culture system, and placed in a cell shaker and cultured at 100rpm, 37°C, 5% $CO_2$ for antibody expression for 6 days. After the culture was completed, the cell supernatant was collected and incubated at 4°C at 4000 rpm for 10 min.

The above cell supernatant was filtrated with a 0.22 $\mu$m filter. AKTA instrument was turned on, the tube A and tube B were firstly washed with solution A (200mM disodium hydrogen phosphate dodecahydrate) and solution B (100mM citric acid monohydrate), respectively, and protein A column was installed. The protein A column was balanced with solution A at a flow rate of 8 mL/min for more than 15 min, and sample was loaded after the UV value, pH value and conductivity detected by the instrument were stable. The sample was loaded at a flow rate of 6-10mL/min, then the UV value would rise, and this peak was the breakthrough peak, and the column was continuously washed with solution A, and the breakthrough peak sample was collected for detection. After the pH value no longer changed, solution B was fed at a flow rate of 6-10mL/min, then the pH value dropped and the UV value rose, and this peak was the elution peak, and the antibody mainly existed in the elution peak, and the elution peak sample was collected for detection. The column was balanced with solution A, then the pipeline and protein A column were filled with 20% ethanol, the column was taken out, and stored at 4°C. SDS-PAGE identification was performed on the collected breakthrough peak and elution peak samples. The purified monoclonal antibody was dialyzed overnight with 20mM PBS buffer, measured by UV spectroscopy or BCA to determine concentration, subpackaged into 1.5 mL tubes, and stored at -20°C for later use.

Example 2: Reactivity of anti-EBV gB protein rabbit monoclonal antibody with gB protein

2.1 Preparation of reaction plate

The gB protein (SEQ ID NO: 24) was diluted with 50 mM CB buffer solution ($NaHCO_3$/$Na_2CO_3$ buffer solution, final concentration was 50 mM, pH value was 9.6) at pH 9.6 to prepare a coating solution having a gB protein final concentration of 2 $\mu$g/mL. 100 $\mu$L of the coating solution was added to each well of a 96-well microtiter plate, and coated at 37°C for 2 hours; washed once with PBST washing solution (20 mM PB7.4, 150 mM NaCl, 0.1% Tween20); then added with 200 $\mu$L of blocking solution (20 mM $Na_2HPO_4$/$NaH_2PO_4$ buffer solution containing 20% calf serum and 1% casein, pH 7.4) to each well, and blocked at 37°C for 2 hours. The blocking solution was discarded, the plate was dried and stored in an aluminum foil bag at 2-8°C for later use.

2.2 ELISA detection of rabbit monoclonal antibodies 3A3 and 3A5 with gB protein

The monoclonal antibodies 3A3 and 3A5 obtained in Example 1, and the reported monoclonal antibody AMMOS (Immunity, 2018, 48:799-811.e9) were taken, and diluted with 20 mM PBS buffer by 2-fold gradient dilution starting from 5 $\mu$g/mL as starting concentration, a total of 15 gradients. The microtiter plate coated with gB protein in the above step 2.1 was taken, added with 100 $\mu$L of diluted sample to each well, and place in a 37°C incubator to react for 30 min. The microtiter plate was washed 5 times with PBST washing solution (20mM PB7.4, 150mM NaCl, 0.1% Tween20), added with 100 $\mu$L of HRP-labeled goat anti-rabbit IgG reaction solution (purchased from Abcam, Item No.: ab6721) to each well, and placed in a 37°C incubator to react for 30 min. After the enzyme labeling reaction step was completed, the microplate plate was washed 5 times with PBST washing solution (20mM PB7.4, 150mM NaCl, 0.1%Tween20), added with 50 $\mu$L of TMB chromogenic reagent (purchased from Beijing Wantai Bio-Pharmaceutical Co., Ltd.) to each well, placed in a 37°C incubator to react for 15 min. After the chromogenic reaction step was completed, 50 $\mu$L of stop solution (purchased from Beijing Wantai Bio-Pharmaceutical Co., Ltd.) was added to each well of the microplate plate, and the OD450/630 value of each well was detected by a microplate reader.

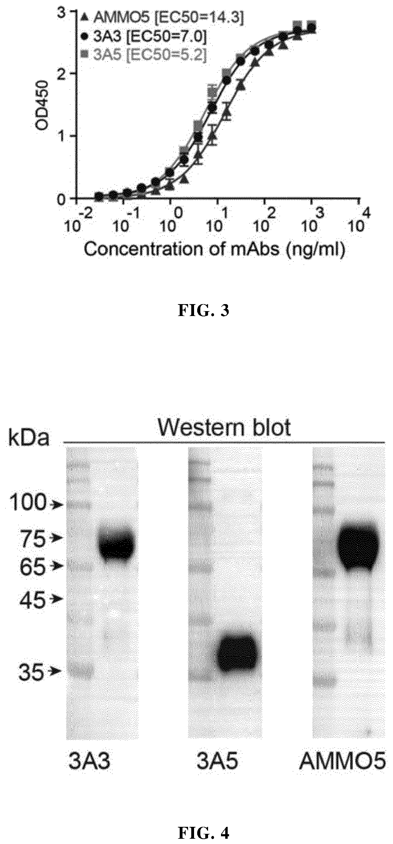

The results were shown in Fig. 3. The EC50 values of rabbit monoclonal antibodies 3A3, 3A5 to gB protein were 7.0 ng/mL and 5.2 ng/mL, respectively, and the EC50 value of human monoclonal antibody AMMOS to gB protein was 14.3 ng/mL. The results showed that the above antibodies all had good binding activity to the purified gB protein.

2.3 Western Blot detection of rabbit monoclonal antibodies 3A3 and 3A5 with gB protein

The gB protein (SEQ ID NO: 24) sample was added to a reducing buffer, heated at 100°C for 10 minutes to prepare the sample, and then subjected to SDS-PAGE protein electrophoresis.

Membrane transfer: The protein on protein glue was transferred to a nitrocellulose membrane using a membrane transfer device.

Blocking: The membrane was rinsed once with ultrapure water, added with commercial blocking solution-1 (purchased from Beijing Wantai Bio-Pharmaceutical Co., Ltd.), and incubated at room temperature for 2 hours.

Primary antibody incubation: The blocking solution was discarded, the antibody was diluted to 1 µg/mL with commercial enzyme dilute solution ED-13 (purchased from Beijing Wantai Bio-Pharmaceutical Co., Ltd.), added to the membrane, and gently shaken and incubated on a shaker at room temperature for 1 hour.

Secondary antibody incubation: The unbound primary antibody on the membrane was removed by washing with PBST washing solution (20 mM PB7.4, 150 mM NaCl, 0.1% Tween20), that was, the membrane was washed 3 times, 5 minutes each time, then the diluted HRP-labeled goat anti-rabbit IgG reaction solution (purchased from Abcam, Item No.: ab6721) was added, gently shaken and incubated on a shaker at room temperature for 1 hour.

Washing was carried out with PBST washing solution (20 mM PB7.4, 150 mM NaCl, 0.1% Tween20) 3 times, 5 minutes each time. An appropriate amount of chemiluminescence substrate mixture was added to cover the surface of the nitrocellulose membrane, and imaging and photographing were performed on a chemiluminescence imager. The results were shown in Fig. 4.

From the results in Fig. 4, it could be seen that after the denaturation treatment of gB protein ectodomain (24-683aa), the disulfide bond near the furin restriction site was broken, forming two protein fragments with molecular weights of about 70KDa and 40KDa, respectively. All of rabbit monoclonal antibodies 3A3, 3A5 and human monoclonal antibody AMMOS could be used for Western Blot detection with gB protein. The monoclonal antibody 3A3 and AMMOS could recognize the 70KDa fragment of the ectodomain of gB protein after denaturation treatment, and the monoclonal antibody 3A5 could recognize the 40KDa fragment of gB protein after denaturation treatment, indicating that the rabbit monoclonal antibodies 3A3 and 3A5 recognized different regions of gB protein.

Example 3: Detection of affinity constant of rabbit monoclonal antibody against EBV gB protein with gB protein

Kinetic analysis of the binding of monoclonal antibody to antigen was performed using the Biacore 8K system. All steps were carried out in PBS buffer, the matched Protein A chip of the company was used to capture the monoclonal antibody diluted to 1 µg/mL, and EBV-gB protein (SEQ ID NO: 24) was used as the detection antigen, and the antigen was diluted into 8 gradients: 200nM, 100nM, 50nM, 25nM, 12.5nM, 6.75nM, 3.125nM, 1.5625nM during the detection. The detection was carried out according to the following procedure: capture, 60s; analyte, 60s; dissociation, 600s; regeneration, 60s. The equilibrium dissociation constant of antibody was calculated using the data acquisition and analysis software supported by the instrument.

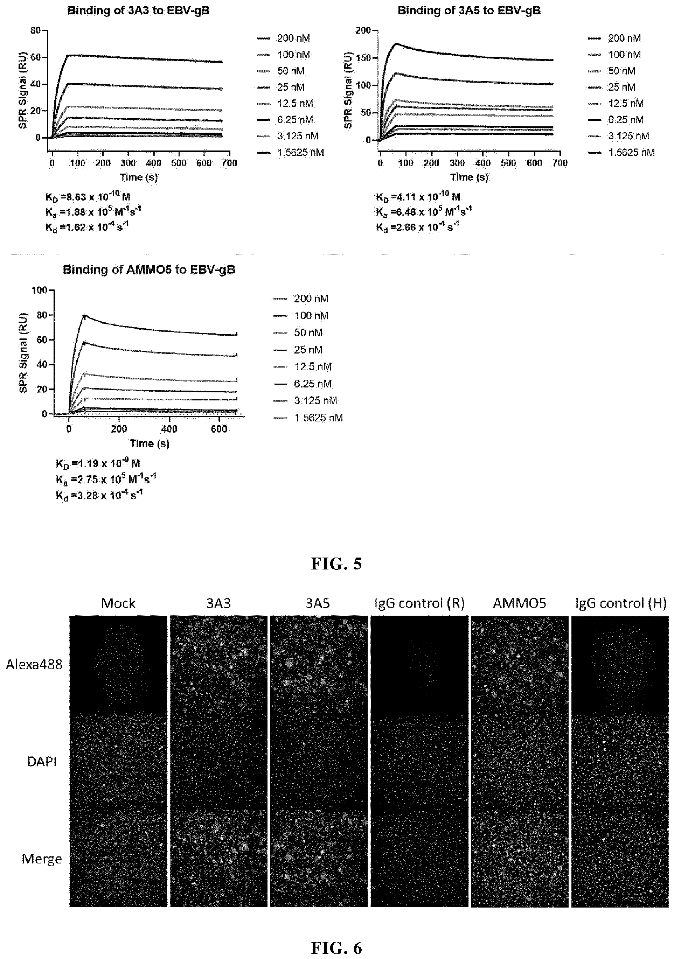

The results were shown in Fig. 5. The equilibrium dissociation constants (KD) of 3A3 and 3A5 for EBV-gB protein were 0.863nM and 0.411nM, respectively, and the KD value of the human monoclonal antibody AMMOS was 1.19nM, indicating that the rabbit monoclonal antibodies 3A3, 3A5 all have high affinity for gB protein.

Example 4: Detection of gB protein expressed by cells by using rabbit monoclonal antibody against EBV gB protein

MDCK (purchased from ATCC, Item No.: CCL-34) was inoculated into a 10 cm cell culture plate, and transfection was carried out when the cell confluency reached 60-80%. Tube A: 30 µg of mammalian expression plasmid comprising the full-length gene of wild-type EBV gB protein was added to 2mL of Opti-MEM; Tube B: 60 µL of Novozyme ExFect®2000 Transfection Reagent was added to 2mL of Opti-MEM. The contents of tube A and tube B were subjected to gentle mixing, respectively, allowed to stand at room temperature for 5 min, then the diluted plasmid was added dropwise to the diluted transfection reagent, mixed gently, and incubated at room temperature for 10 min. The plasmid-transfection reagent complex was added dropwise to the cells and cultured in a cell culture incubator. After 36 hours of transfection, the cells were digested with trypsin, inoculated into a 96-well cell culture plate at a ratio of 10^4 cells per well, and cultured in a cell culture incubator. 12 hours after the inoculation into the 96-well cell culture plate, the cell supernatant was discarded, washing was performed once by adding 100 µL of PBS to each well, 100 µL of 4% paraformaldehyde in PBS was added to each well to fix the cells in the dark for 15 minutes, and washing was performed 3 times by adding 100 µL of 20 mM PBS to each well. 100 µL of 3‰ Triton X-100 in PBS was added to each well to permeabilize the cells for 15 min, and washing was performed 3 times by adding 100 µL of PBS to each well. The monoclonal antibody was diluted to 1 µg/mL with PBS containing 2% BSA, added to a 96-well cell culture plate, 100 µL per well, incubated at room temperature for 30 min, and washed three times by adding 100 µL PBS to each well. Fluorescent secondary antibody Alexa Fluor® 488 Donkey Anti-Rabbit IgG (H+L) (purchased from Invitrogen, Item No.: A-21206) was diluted to 1 µg/mL with PBS containing 2% BSA, added to 96-well cell culture plate, 100 µL per well, incubated at room temperature for 30 min, and washed 3 times by adding 100 µL of PBS to each well. The cell nuclear dye DAPI was diluted at a ratio of 1:2000 with PBS containing 2% BSA, added to each well, 50 µL per well, incubated at room temperature for 5 min, and washed 3 times by adding 100 µL of PBS to each well. The 96-well cell culture plate was placed under a fluorescence microscope for observation and the results were photographed.

The results were shown in Fig. 6. The detection results of 3A3, 3A5 and AMMOS showed that the green fluorescence was distributed in the cell membrane and cytoplasm of MDCK cells expressing gB protein. The above results indicated that 3A3 and 3A5 had specific binding activity to gB protein expressed on cells, and 3A3 and 3A5 could recognize natural gB protein expressed on the cell membrane surface.

Example 5: Analysis of neutralizing activity of rabbit monoclonal antibody against EBV gB protein in virus infection model

B cell neutralization model of EBV:

The monoclonal antibodies 3A3, 3A5, the reported monoclonal antibody AMMOS and control rabbit monoclonal antibody 1E12 (non-neutralizing rabbit monoclonal antibody against gB protein, its nucleotide sequences of VH and VL were respectively as set forth in SEQ ID NOs: 28-29) were taken, diluted with RPMI1640 serum-free medium by 2-fold gradient dilution starting from 100 µg/mL as the initial concentration, in total of 15 gradients. 50 µL of the diluted antibody was taken and added to a 96-well cell plate, and 20 µL of the diluted solution of virus carrying GFP green fluorescent protein gene produced by CNE2 cells was added, mixed well, and cultured in a 37°C incubator for 2 hours. $1 \times 10^6$ AKATA cells (gifted by the Cancer Center of Sun Yat-sen University) were resuspended with 13mL of 10% FBS RPMI1640 medium, 130 µL of the cell suspension was added to the mixed solution of virus and antibody, and incubated in a 37°C incubator for 48 hours, the flow cytometer LSRFortessaX-20 of BD company was used to detect the virus infection rate of AKATA cells, the reduction ratio (%) of the number of GFP-positive cells in the antibody treatment group compared with the infection control group was calculated, and the inhibition rate (%) of the antibody on the B cell infection model was calculated.

Epithelial cell neutralization model of EBV:

HNE1 cells (gifted by the Cancer Center of Sun Yat-sen University) were plated into a 96-well plate in advance by using 10% FBS DMEM medium according to the standard of 160 µL in volume, 5000 cells/well, and cultured in a 37°C incubator for 24 hours. After the cells adhered to the wall, the monoclonal antibody was diluted with DMEM0 serum-free medium by 2-fold gradient dilution starting from 100 µg/mL as the initial concentration, in total of 15 gradients. 20 µL of the diluted antibody of different concentrations was pipetted and fully mixed with 20 µL of EBV virus suspension produced by AKATA cells, and incubated at 37°C for 3 hours, the mixed solution of antibody and virus was added to a 96-well plate coated with HNE1 cells, and cultured in a 37°C incubator for 48 hours, then the HNE1 cells in the 96-well plate were digested, the proportion of HNE1 cells expressing GFP green fluorescent protein was detected by the flow cytometer LSRFortessaX-20 of BD company, the reduction ratio (%) of the number of GFP-positive cells in the antibody treatment group compared with the infection control group was calculated, and the inhibition rate (%) of the antibody on the epithelial cell infection model was calculated.

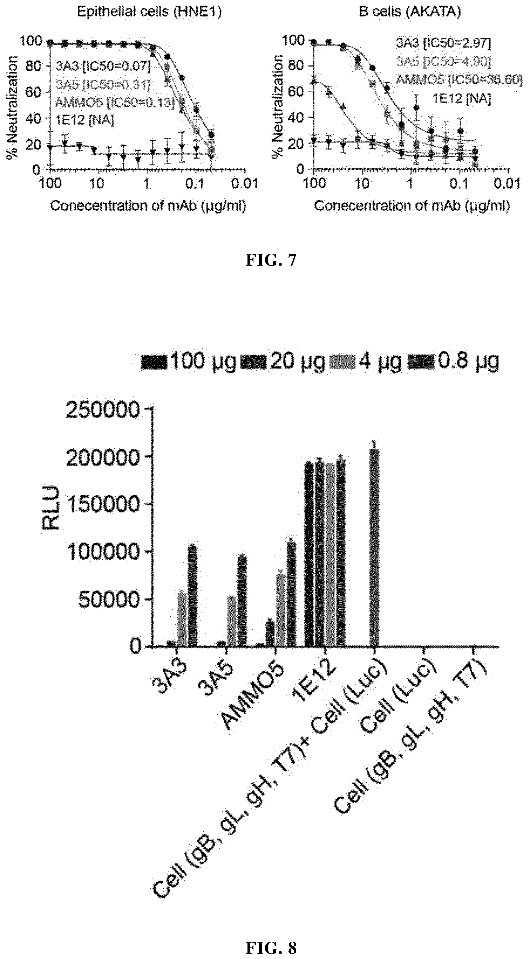

The results were shown in Fig. 7, the IC50 values of the monoclonal antibodies 3A3 and 3A5 on the epithelial cell infection model were 0.07 µg/mL and 0.31 µg/mL, respectively, and the IC50 value of AMMOS on the epithelial cell infection model was 0.13 µg/mL; the IC50 values of 3A3 and 3A5 on the B cell infection model were 2.97 µg/mL and 4.90 µg/mL, respectively, and the IC50 value of AMMOS on the B infection model was 36.60 µg/mL, indicating that the monoclonal antibodies 3A3 and 3A5 could achieve complete neutralization of viral infection on the two infection models.

Example 6: Analysis of blocking ability of rabbit monoclonal antibody against EBV gB protein on cell fusion model

293T cells were inoculated into a 10 cm cell culture plate, and transfected when the cell confluency reached 60-80%. There were set up: plate A: PEI was used as transfection reagent, eukaryotic expression plasmids carrying genes encoding full-length gB, gH, gL (see Genbank ID: KF373730.1) and T7 RNA polymerase in an amount of 2.5 µg were prepared respectively, and used for transfection; plate B: PEI was used as transfection reagent, and 10 µg of eukaryotic expression plasmid comprising Luciferase gene controlled by T7 promoter was transfected in the 293T cells.

24 hours after transfection, the 293T cells on the plate A were digested, and subpackaged into EP tubes at a ratio of $2 \times 10^5$ cells in each group, 200 µL of the antibody of different concentrations was added, and incubated in a 37°C incubator for 30 minutes, then transferred into a 24-well plate. The cells on the plate B were digested in advance, and $2 \times 10^5$ cells were added to each well of the above-mentioned 24-well plate, and cultured at 37°C for 24 hours.

100 µL of firefly luciferase substrate in the Dual-Glo Luciferase Assay System kit of Promega company was added to the 24-well plate, lysis was performed at room temperature for 20 minutes, and 80 µL of cell lysate supernatant was taken from each well for fluorescence quantitative detection. The reduction ratio of fluorescence readings in the antibody treatment group compared with the untreated control well was calculated, and the blocking efficiency (%) of the antibody on the cell fusion model was calculated.

The results were shown in Fig. 8. The results showed that the monoclonal antibodies 3A3 and 3A5 had a concentration-dependent blocking effect on the cell fusion model, indicating that they had a specific blocking effect on the cell membrane fusion, and under the same concentration conditions, the membrane fusion inhibition effects of 3A3 and 3A5 were better than that of AMMOS.

Example 7: Analysis of protective effect of rabbit monoclonal antibody against EBV gB protein on animal model

The EBV derived from AKATA cells was taken, mixed with 300 $\mu$g of antibody at 4°C according to the dose of 1000 TD50 (Transform unit dose), and incubated overnight at 4°C, the mixture was added to 10^7 peripheral blood mononuclear cells (PBMC) isolated from human cord blood, incubated at 37°C for 1 hour, and then injected into the abdominal cavity of 4-5 week-old NSG immunodeficient mice (purchased from Beijing IDMO Biotechnology Co., Ltd.). The orbital blood of mice was collected on the 0th, 14th, 28th and 42nd days after injection, and the virus copy number was detected. On the 7th day after PBMC injection, the OKT3 antibody targeting human T cells (purchased from Nobimpex, Item No.: B 104-0005) was intraperitoneally injected into mice to block the non-specific clearance of Epstein-Barr virus by human T cells in PBMCs. On the 65th day after PBMC injection, the NSG mice were euthanized, and the changes in the size of the spleen and the appearance of cancerous tissues were observed. EBV BARF5-specific primers (upstream primer: GGTCACAATCTCCACGCTGA; downstream primer: CAACGAGGCTGACCTGATCC) were used to identify the copy number of EBV DNA in the collected mouse peripheral blood by real-time fluorescent quantitative PCR. A commercial kit (purchased from Zhongshan Jinqiao, Cat. No.: ISH-7001) was used to perform EBER in situ hybridization detection on spleen tissue sections to identify whether there was virus in the spleen.

The results were shown in Fig. 9. Fig. 9A showed a schematic diagram of treatment process of animal model; Fig. 9B showed the survival rate of mice in each antibody treatment group; Fig. 9C showed the EBV DNA copy number in the peripheral blood of mice in each antibody treatment group; Fig. 9D showed the spleen size after euthanasia of mice in each antibody treatment group; Fig. 9E showed the results of EBER in situ hybridization detection of spleen tissue sections of mice in each antibody treatment group. It could be seen from the figures that the survival rates of mice treated with monoclonal antibodies 3A3 and 3A5 reached 100%, which were significantly better than those of the reported monoclonal antibody AMMOS and the control rabbit monoclonal antibody 1E12 (Fig. 9B). Over time, the EBV DNA copy numbers in the peripheral blood of mice treated with 3A3, 3A5 and AMMOS showed a downward trend. At the 6th week after antibody treatment, compared with the control antibody 1E12 treatment group, the DNA copy numbers of the 3A3, 3A5 and AMMOS treatment groups were at a lower level (Fig. 9C). At the same time, at the end of the experiment, after the mice were euthanized, the spleen of the control antibody group showed obvious enlargement and lesions (Fig. 9D). At the same time, the presence of EBER of EBV was detected by RNA in situ hybridization method (Fig. 9E), indicating the presence of virus in the spleen tissue. However, the spleens of mice treated with 3A3, 3A5, and AMMOS monoclonal antibody maintained normal shape and size (Fig. 9D), and no EBER was detected (Fig. 9E), indicating that the virus content in the spleen was low or there was no virus. The above results showed that 3A3 and 3A5 had a good effect of blocking virus infection in animals.

Example 8: Identification of epitope competition of rabbit monoclonal antibody against EBV gB protein with the reported neutralizing antibody against EBV gB protein

8.1 Preparation of horseradish peroxidase (HRP) conjugated antibody

1 mg of the target antibody at a concentration of 1 mg/mL was prepared, and dialyzed into CB buffer (NaHCO$_3$/Na$_2$CO$_3$ buffer, final concentration was 50 mM, pH was 9.6) at 4 °C, medium change was performed every 4 hours, in total of twice. HRP dry powder and NaIO$_4$ dry powder were dissolved in ultrapure water to 20 mg/mL, respectively, then the two solutions were mixed well at a volume ratio of 1:1, and HRP activation was performed by incubation at 4°C in the dark for 30 minutes. The HRP activation was stopped by adding ethylene glycol, in which ethylene glycol was added in a corresponding amount according to a standard that 20 $\mu$L of ethylene glycol was added to 20 mg of HRP, and incubation was performed in the dark for 30 min. 1mg of the HRP after the activation was stopped was added to the antibody solution, mixed well and then dialyzed to CB buffer at 4°C, medium change was performed once every 4 hours, in total of twice. Ultrapure water was used to prepare a NaBH$_4$ solution with a concentration of 20 mg/mL, and 10 $\mu$L of NaBH$_4$ solution was added to the above antibody solution to terminate the enzyme labeling reaction. Under the condition of 4°C, mixing was carried out once every 30 minutes, in total of 3 times. An equal volume of saturated ammonium sulfate solution was added to sediment the enzyme-labeled antibody, centrifugation was performed at 12,000rpm for 10min, the supernatant was discarded, and the antibody precipitated with saturated ammonium sulfate was resuspended and dissolved with the buffer solution that was prepared with PBS buffer and contained glycerol at a final concentration of 50% and 10% NBS (newborn bovine serum).

8.2 Preparation of reaction plate

The gB protein (SEQ ID NO: 24) was diluted with CB buffer (NaHCO$_3$/Na$_2$CO$_3$ buffer, final concentration was 50 mM, pH was 9.6), and the final concentration was 0.1 μg/mL. 100 μL of coating solution was added to each well of a 96-well microtiter plate, and coating was performed at 37°C for 2 hours. Washing was performed once with PBST washing solution (20 mM PB7.4, 150 mM NaCl, 0.1% Tween20). Then, 200 μL of blocking solution (20 mM Na$_2$HPO$_4$/NaH$_2$PO$_4$ buffer solution at pH 7.4 containing 20% calf serum and 1% casein) was added to each well, and allowed to stand at 37°C for 2 hours, and the blocking solution was discarded. After drying, it was packaged into an aluminum foil bag and stored at 2-8°C for later use.

8.3 Epitope competition detection of gB protein neutralizing antibody

The horseradish peroxidase (HRP)-conjugated antibody-labeled monoclonal antibodies 3A3, 3A5, and AMMOS as prepared in step 8.1 of this example were taken, and diluted with 20mM PBS buffer by 5-fold gradient dilution starting from a ratio of 1:100, in a total of 8 gradients. The microtiter plate coated with gB protein in step 8.2 above was taken, added with 100 μL of the diluted sample to each well, and placed in a 37°C incubator to react for 30 minutes. The microtiter plate was washed 5 times with PBST washing solution (20mM PB7.4, 150mM NaCl, 0.1% Tween20), and 50 μL of TMB chromogenic reagent (purchased from Beijing Wantai Bio-Pharmaceutical Co., Ltd.) was added to each well, and placed in a 37°C incubator to react for 15 minutes. After the chromogenic reaction step was completed, 50 μL of stop solution (purchased from Beijing Wantai Bio-Pharmaceutical Co., Ltd.) was added to each well of the microplate plate after the reaction, and the OD450/630 value of each well was detected on a microplate reader. The dilution factor of the HRP-labeled antibody corresponding to an OD value of about 1 was used as the dilution factor for subsequent use.
The monoclonal antibodies 3A3, 3A5, the reported antibodies 72A1 (mouse monoclonal antibody against EBV gp350 glycoprotein, Proc Natl Acad Sci USA, 1980,77:2979-83) and AMMOS were taken, and diluted to 10 μg/mL with 20 mM PBS buffer, the microtiter plate coated with gB protein was taken, added with 100 μL of the diluted antibody sample (named as "Primary antibody") to each well, and placed in a 37°C incubator to react for 30 minutes. The microtiter plate was washed with PBST washing solution (20mM PB7.4, 150mM NaCl, 0.1%Tween20) 5 times, added with 100μL of the diluted HRP-labeled antibody (named as "Secondary antibody") to each well, and placed in a 37°C incubator to react for 30 minutes. After completing the HRP-labeled antibody reaction step, the microtiter plate was washed 5 times with PBST washing solution (20mM PB7.4, 150mM NaCl, 0.1%Tween20), and added with 50 μL of TMB chromogenic reagent (purchased from Beijing Wantai Bio-Pharmaceutical Co., Ltd.) to each well, and placed in a 37°C incubator to react for 15 minutes. After the chromogenic reaction step was completed, 50 μL of stop solution (purchased from Beijing Wantai Bio-Pharmaceutical Co., Ltd.) was added to each well of the microplate plate after reaction, the OD450/630 value of each well was detected on a microplate reader, and the competition rate was calculated. The formula for calculating the competition rate of the antibody was:

$$\text{Competition rate \%} = [\text{OD}_{(\text{-Primary/+Secondary})} - \text{OD}_{(\text{+Primary/+Secondary})}] / \text{OD}_{(\text{-Primary/+Secondary})} \times 100.$$

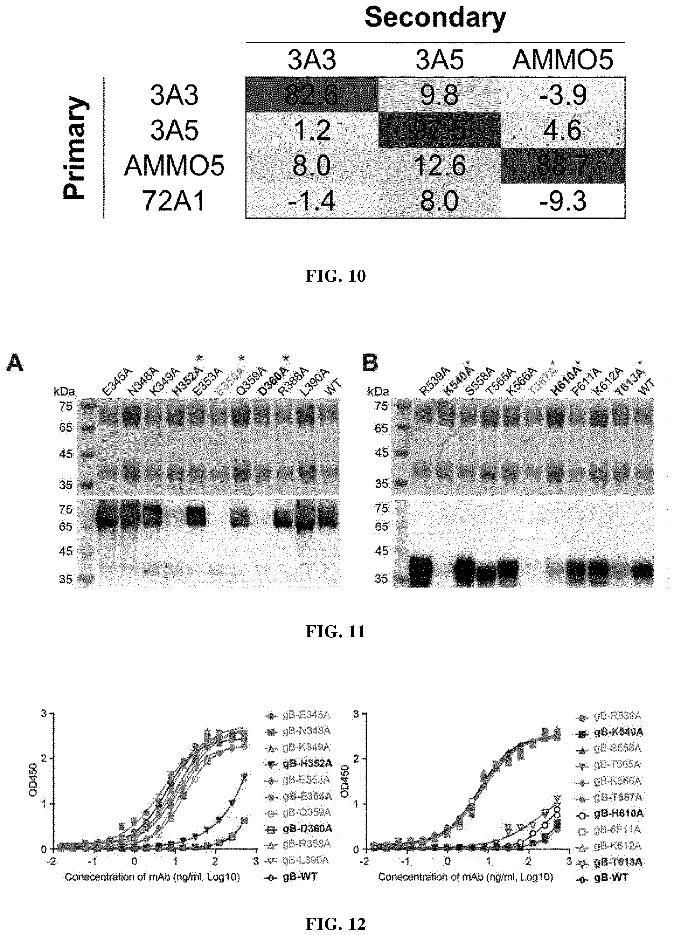

The competition rates among antibodies were shown in Fig. 10. The results showed that recognition epitopes of the screened and obtained rabbit monoclonal antibodies 3A3 and 3A5 were different from those of the reported gB neutralizing antibodies AMMOS and 72A1, and the rabbit monoclonal antibodies 3A3 and 3A5 recognized different epitopes, indicating that the antibodies 3A3 and 3A5 recognized new epitopes.

Example 9: Identification of key sites recognized by rabbit monoclonal antibody against EBV gB protein

9.1 Construction of gB ectodomain point mutant protein

In order to identify the key sites recognized by the rabbit neutralizing antibodies for gB protein, the cloning, expression and evaluation of gB protein ectodomain point mutation were carried out.
Primers were designed according to the instructions of the Mut Express II Fast Mutagenesis Kit V2 (purchased from Novizyme), and point mutation cloning was carried out to mutate the amino acid at the corresponding site of the gB protein ectodomain (SEQ ID NO: 24) into alanine, and whether the point mutation clone was constructed correctly was verified by sequencing. The correct point mutation clone was transiently transferred to 293F cells for eukaryotic expression, and the point mutant protein was purified by nickel column.

9.2 Identification of key sites for antibody recognition by Western Blot method

The wild-type (WT) or each point mutant gB protein sample obtained in the above step 9.1 was taken, added to a reducing buffer, heated at 100°C for 10min to prepare the sample, and then subjected to SDS-PAGE protein electrophoresis.

Membrane transfer: The protein on protein gel was transferred to the nitrocellulose membrane using a membrane transfer device.

Blocking: The membrane was rinsed once with ultrapure water, added with commercial blocking solution-1 (purchased from Beijing Wantai Bio-Pharmaceutical Co., Ltd.), and incubated at room temperature for 2 hours.

Primary antibody incubation: The blocking solution was discarded, the antibody was diluted to 1 μg/mL with commercial enzyme dilute solution ED-13 (purchased from Beijing Wantai Bio-Pharmaceutical Co., Ltd.), added to the membrane, and shaken gently and incubated on a shaker at room temperature for 1 hour.

Addition of secondary antibody: The unbound primary antibody on the membrane was washed off with PBST washing solution (20 mM PB7.4, 150 mM NaCl, 0.1% Tween20), the membrane was washed 3 times, 5 minutes each time, then added with the diluted HRP-labeled goat anti-rabbit IgG reaction solution (purchased from Abcam, Item No.: ab6721), and incubated on a shaker at room temperature for 1 hour.

The unbound secondary antibody on the membrane was washed off with PBST washing solution (20 mM PB7.4, 150 mM NaCl, 0.1% Tween20), 5 minutes each time. An appropriate amount of chemiluminescence substrate mixture was added to cover the surface of the nitrocellulose membrane, imaging and photographing were performed on a chemiluminescent imager, and the results were recorded.

The results of Western Blot were shown in Fig. 11, wherein Fig. 11A showed the result of Western Blot staining of protein samples by 3A3 antibody, and Fig. 11B showed the result of Western Blot staining of protein samples by 3A5 antibody. Fig. 11A showed that some point mutations affected the binding ability of the protein to the antibody. Wherein, the three amino acid positions 352, 356, and 360 were the key positions for the 3A3 antibody recognition, and any amino acid mutation at these positions could make the monoclonal antibody 3A3 not react or weakly react to the protein. Fig. 11B showed that the four amino acid positions 540, 567, 610, and 613 were the key positions for the 3A5 antibody recognition, and any amino acid mutation at these positions could make the monoclonal antibody 3A5 not react or weakly react to the protein.

9.3 Identification of reactivity of gB monoclonal antibody to point mutant protein by ELISA method

The wild-type (WT) or each point mutant gB protein sample obtained in step 9.1 above was taken and diluted with 50 mM CB buffer ($NaHCO_3/Na_2CO_3$ buffer, final concentration was 50 mM, pH was 9.6) at pH 9.6 to a final concentration of 2μg/mL. 100 μL of coating solution was added to each well of a 96-well microtiter plate, and coating was performed at 37°C for 2 hours. Washing was performed once with PBST washing solution (20 mM PB7.4, 150 mM NaCl, 0.1% Tween20). Then, 200 μL of blocking solution (20 mM $Na_2HPO_4/NaH_2PO_4$ buffer solution at pH 7.4 containing 20% calf serum and 1% casein) was added to each well, and blocking was performed at 37°C for 2 hours. The blocking solution was discarded. After drying, the plate was packaged into an aluminum foil bag and stored at 2-8°C for later use.

The monoclonal antibodies 3A3 and 3A5 obtained in Example 1 were diluted with 20 mM PBS buffer by 2-fold gradient dilution starting from 0.5 μg/mL as the initial concentration, in total of 15 gradients. The microtiter plate coated with gB protein was taken, added with 100 μL of the diluted sample to each well, and placed in a 37°C incubator to react for 30 minutes. The microtiter plate was washed 5 times with PBST washing solution (20mM PB7.4, 150mM NaCl, 0.1% Tween20), added with 100 μL of the HRP-labeled goat anti-rabbit IgG reaction solution to each well, and placed in a 37°C incubator to react for 30 minutes. After the enzyme labeling reaction step was completed, the microplate plate was washed 5 times with PBST washing solution (20mM PB7.4, 150mM NaCl, 0.1%Tween20), added with 50 μL of TMB chromogenic reagent (purchased from Beijing Wantai Bio-Pharmaceutical Co., Ltd.) to each well, and placed in a 37°C incubator to react for 15 minutes. After the chromogenic reaction step was completed, 50 μL of stop solution (purchased from Beijing Wantai Bio-Pharmaceutical Co., Ltd.) was added to each well of the microplate plate after reaction, and the OD450/630 value of each well was detected on a microplate reader.

The results of ELISA OD450 detection were shown in Fig. 12, wherein Fig. 12A showed the results of ELISA detection of protein samples by 3A3 antibody, and Fig. 12B showed the results of ELISA detection of protein samples by 3A5 antibody. The results showed that some point mutations affected the binding ability of the protein to the antibody. Wherein, the three amino acid positions 352, 356, and 360 were the key positions for the 3A3 antibody recognition, and any amino acid mutation at these positions could lead to a weakened reaction of the monoclonal antibody 3A3 to the protein. The four amino acid positions 540, 567, 610, and 613 were the key positions for the 3A5 antibody recognition, and any amino acid mutation at these position could lead to a weakened reaction of the monoclonal antibody 3A5 to the protein. The results were consistent with the results of Western Blot identification, which proved that the three amino acid positions 352, 356, and 360 were the key positions for the monoclonal antibody 3A3 recognition, and the four amino acid positions 540, 567, 610, and 613 were the key positions for the monoclonal antibody 3A5 recognition.

Example 10: Identification of epitope recognized by rabbit monoclonal antibody against EBV gB

10.1 Cloning and expression of gB protein segments

In order to identify the epitopes recognized by the two monoclonal antibodies, the pTO-T7-HBc149 vector was used to display the partial sequence of gB protein, and the reactivity of the antibody to different truncated epitopes was identified by Western Blot method. The nucleotide sequence and protein sequence of the HBc149/mut carrier protein were set forth in SEQ ID NOs: 27 and 21, respectively, wherein the bold and underlined parts were the nucleotide and amino acid sequences corresponding to the restriction sites BamH I and EcoR I. We inserted the target sequence into the middle of BamH I and EcoR I restriction sites, that was, inserted the gB fragment into the middle of SE...FG.

Different Domain sequences on the gB protein (sequence set forth in SEQ ID NO: 1) were truncated, mutual primers were designed and the gB protein particle was as template to obtain target fragments of different truncation lengths by PCR, and a gel recovery kit was used to recover the target fragments, the cloning vector was subjected to enzyme digestion, the restriction site was BamH I/EcoR I, and the Gibson assembly method was used to for construction on pTO-T7-HBc149 vector. After the clone was constructed, it was transformed into competent ER2566 (DE3) *Escherichia coli* (purchased from Shanghai Weidi Biotechnology Co., Ltd., Item No.: EC1060). A single clone colony was picked out and inoculated into LB liquid medium, expanded into 500 mL medium for culture, when the medium had an OD600 of 0.8, 0.5 mM IPTG as inducer was added to induce protein expression at 32°C. After induced expression was carried out for 8 hours, the cells were collected by centrifugation at 7000g for 10min, then sonicated, and centrifuged at 25000g for 10min to collect the supernatant after sonication, 30% volume of saturated ammonium sulfate solution was added to the supernatant after sonication to precipitate protein, the pellet was resuspended with PBS buffer and dialyzed into PBS solution, and finally purified by gel filtration using a prepacked Superdex 200 column.

10.2 Identification of antibody recognition epitope by Western Blot

The protein samples with different truncation lengths obtained in the above step 10.1 were taken, added with a reducing buffer, heated at 100°C for 10 minutes to prepare the samples, and then subjected to SDS-PAGE protein electrophoresis.

Membrane transfer: The protein on the protein glue was transferred to the nitrocellulose membrane using a membrane transfer device.

Blocking: The membrane was rinsed once with ultrapure water, added with commercial blocking solution-1 (purchased from Beijing Wantai Bio-Pharmaceutical Co., Ltd.), and incubated at room temperature for 2 hours.

Primary antibody incubation: The blocking solution was discarded, the antibody was diluted to 1 μg/mL with commercial enzyme dilute solution ED-13 (purchased from Beijing Wantai Bio-Pharmaceutical Co., Ltd.), added to the membrane, and shaken gently and incubated on a shaker at room temperature for 1 hour.

Addition of secondary antibody: The unbound primary antibody on the membrane was washed off with PBST washing solution (20 mM PB7.4, 150 mM NaCl, 0.1% Tween20), the membrane was washed 3 times, 5 minutes each time, then added with the diluted HRP-labeled goat anti-rabbit IgG reaction solution (purchased from Abcam, Item No.: ab6721), and incubated on a shaker at room temperature for 1 hour.

Washing was performed with PBST (20 mM PB7.4, 150 mM NaCl, 0.1% Tween20), 5 minutes each time. An appropriate amount of chemiluminescence substrate mixture was added to cover the surface of the nitrocellulose membrane, imaging and photographing were performed on a chemiluminescent imager, and the results were recorded.

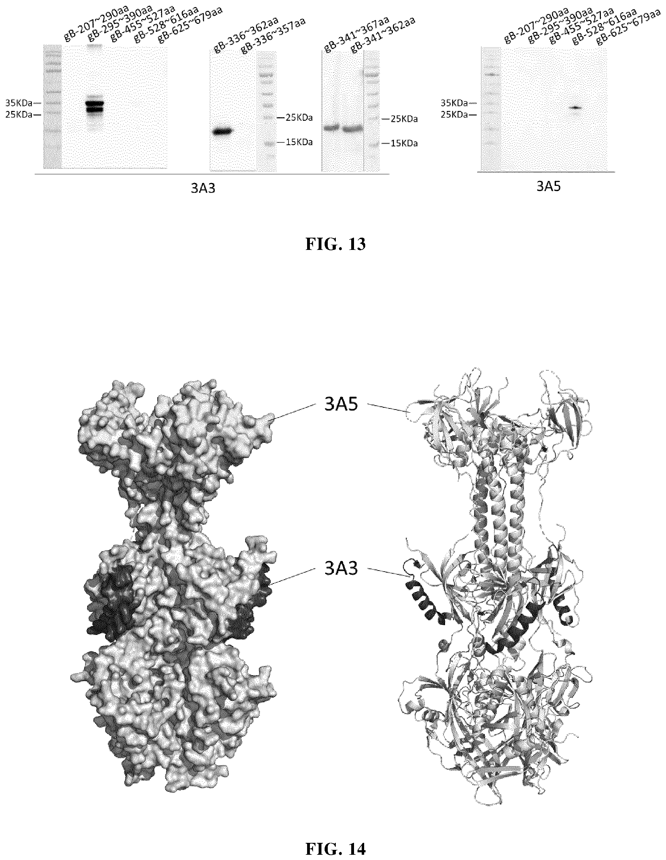

The results of Western Blot were shown in Fig. 13. It could be seen from the figure that the epitope recognized by 3A3 was the amino acid sequence at positions 341-362, the epitope recognized by 3A5 was the amino acid sequence at positions 528-616, and the locations of the epitopes recognized by the monoclonal antibodies 3A3 and 3A5 in the 3D structure of the protein were shown in Fig. 14 (drawn by pymol software, PDB ID of EBV gB: 3FVC).

Example 11: Preparation and immunogenicity evaluation of chimeric virus-like particle

11.1 Construction and identification of chimeric virus-like particle (cVLP) based on 3A3 recognition sequence

According to the structural analysis of gB protein (Fig. 14), it could be seen that 3A3 recognized a consecutive epitope. In order to explore whether this epitope had the feasibility of developing into an epitope vaccine, the sequence at positions 331-367 of the wild-type full-length gB protein (SEQ ID NO: 17) was selected, an expression clone was constructed according to the method described in Example 10.1, and the protein was expressed and purified. According to the method in Example 2.3, the activity detection of the HBc149 protein (SEQ ID NO: 22) chimerized with the gB-331~367aa epitope was performed by Western Blot.

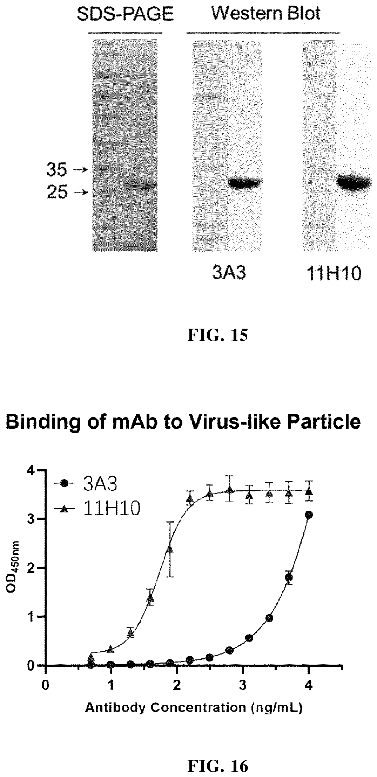

The detection results were shown in Fig. 15, which showed that gB-331-367aa epitope-specific rabbit monoclonal antibody 3A3 and HBC149 protein-specific mouse monoclonal antibody 11H10 (Theranostics. 2020 Apr 27;10(13):5704-5718) all could react to the HBc149 chimeric protein, indicating that the gB-331-367aa epitope was successfully expressed, and the activity of binding to 3A3 in Western Blot was maintained.

According to the method of Example 2.2, the activity detection of the HBc149 protein chimerized with the gB-331-367aa epitope was performed by ELISA.

The detection results were shown in Fig. 16. It could be seen from the figure that gB-331-367aa epitope-specific rabbit monoclonal antibody 3A3 and HBC149 protein-specific mouse monoclonal antibody 11H10 all could react to HBc149 chimeric protein, indicating that the gB-331-367aa epitope was displayed successfully, and the activity of binding to 3A3 in ELISA was maintained.

The purity of HBc149 virus-like particle (149-$\alpha$B) chimerized with gB-331-367aa epitope and wild-type HBc149 virus-like particle (149-WT) was identified by high performance liquid chromatography. Sample identification was performed using a TSKgel G5000PWxl column.

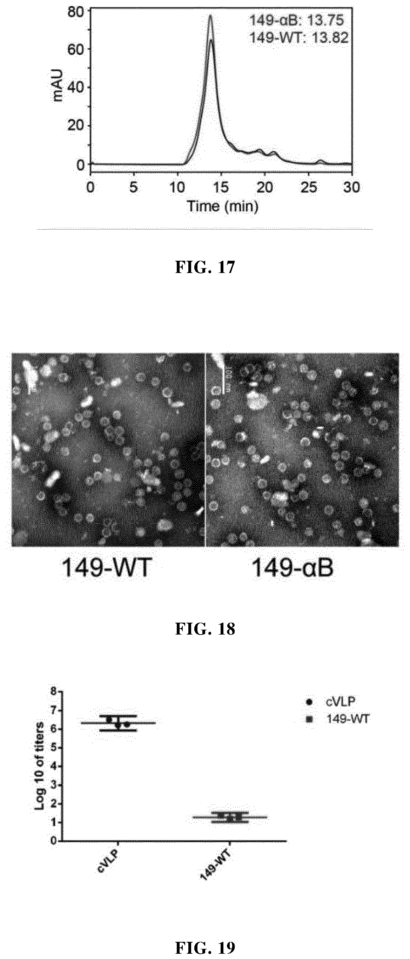

The HPLC identification results were shown in Fig. 17. The results showed that the virus-like particle gave single peaks with peak times of 13.75 minutes and 13.82 minutes, respectively, indicating that the virus-like particle had a high purity.

The chimeric virus-like particle was diluted to 0.5 mg/mL, added dropwise onto a 200-mesh carbon-coated copper grid, incubated for 5 min, then the excess solution was sucked off, washing was performed twice with double distilled water, and staining with 2% phosphotungstic acid was immediately performed for 30s. Photographing and data collection were performed using a FEI Tecnai T12 TEM transmission electron microscope.

The results of electron microscopy were shown in Fig. 18, and the results showed that the chimeric virus-like particle was successfully assembled and the sample was homogeneous.

11.2 Immunological evaluation of chimeric virus-like particle based on 3A3 recognition epitope

500 $\mu$g of the chimeric virus-like particle constructed in the above step 11.1 was mixed with an equal volume of complete Freund's adjuvant (CFA) to obtain 2 mL of emulsion, which was administrated to experimental rabbits by multiple point injection at neck and back. On the 14th and 28th days after the first immunization, the same dose of protein was mixed with an equal volume of incomplete Freund's adjuvant (IFA) to obtain 2 mL of emulsion, and administrated to experimental rabbits by multiple point injection at neck and back. On the 42nd day after the first immunization, the whole blood was collected through the central artery of rabbit ear, the serum was separated, and the immune serum binding titer was detected according to the ELISA method of Example 2.2, and the immune serum neutralization titer was evaluated according to the B cell and epithelial cell infection models of EBV of Example 5.

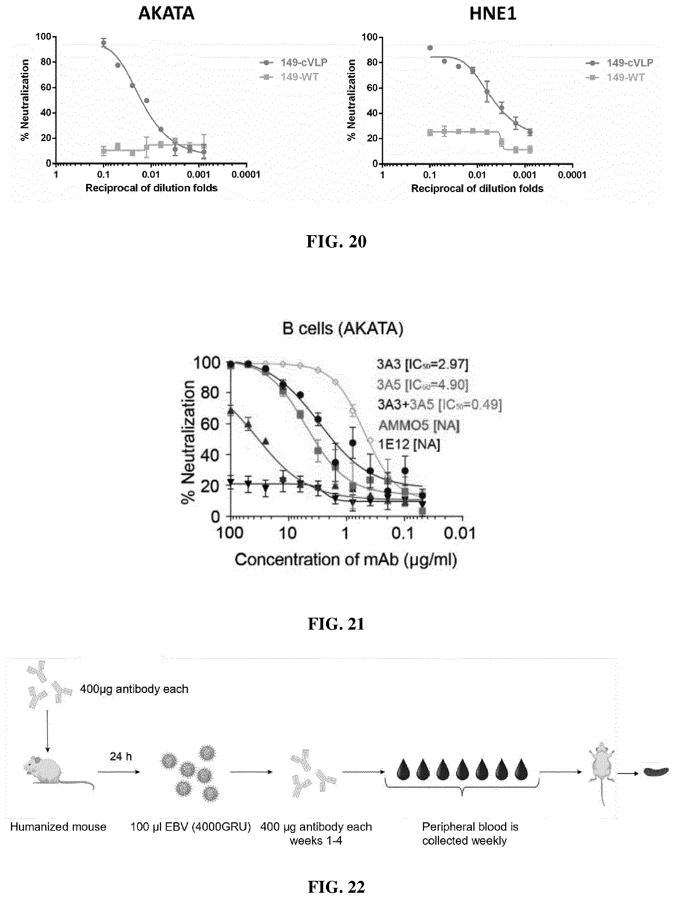

The ELISA detection results of the binding titers of rabbit sera immunized with HBc149 virus-like particle chimerized with gB-331-367aa epitope (149-cVLP) and wild-type HBc149 virus-like particle (149-WT) were shown in Fig. 19. The EBV neutralization titer detection results of virus-like particles 149-cVLP and 149-WT immunized rabbit sera on the B cell and epithelial cell models were shown in Fig. 20. The results showed that after immunizing experimental rabbits with chimeric virus-like particles based on the 3A3 recognition epitope, high-titer binding antibody (above 10^6) could be induced (Fig. 19). Immune sera could simultaneously neutralize virus infection on two cell models (Fig. 20), and the potential of developing into anepitope vaccines of the epitope had been shown.

Example 12: Analysis of synergistic neutralization activity of monoclonal antibodies 3A3 and 3A5 on virus infection model

Using the method shown in Example 5, the neutralizing activity of the monoclonal antibodies 3A3 and 3A5, the reported monoclonal antibody AMMOS, the control rabbit monoclonal antibody 1E12, and the mixed antibody 3A3+3A5 (wherein the molar concentration ratio of 3A3:3A5 was 1:1) on virus infection model was determined.

The results were shown in Fig. 21. The results showed that the antibodies 3A3 and 3A5 exhibited a synergistic neutralization effect (1+1>2) in the on vitro B cell neutralization model, and their neutralization titers were increased by nearly 10 times. This suggested the potential of using the antibodies 3A3 and 3A5 in combination in clinic.

Example 13: Animal protection effects of 3A3 chimeric antibody and 3A5 chimeric antibody on humanized mouse model

The constant region sequences of antibodies 3A3 and 3A5 were substituted with human heavy chain constant region sequence (SEQ ID NO: 30) and human light chain constant region sequence (SEQ ID NO: 31), respectively, to construct 3A3 chimeric antibody and 3A5 chimeric antibody.

The protection effects of the 3A3 chimeric antibody and 3A5 chimeric antibody, as well as the mixed antibody (3A3 chimeric antibody + 3A5 chimeric antibody, wherein the molar concentration ratio of 3A3 chimeric antibody to 3A5 chimeric antibody was 1:1) in CD34+ positive hematopoietic stem cell-reconstituted humanized mouse model were evaluated.

The treatment process (as shown in Fig. 22) of chimeric monoclonal antibody in the humanized mouse model comprised: a NOD.Cg-*Prkdc*em1IDMO*Il2rg*em2IDMO (NOD-*Prkdc*null *IL2Rγ*null, NPI®) humanized mouse model (provided by Beijing IDMO Biotechnology Co., Ltd.) was taken, wherein the mouse treatment method was as follows: CD34 positive cells derived from peripheral blood mononuclear cells (PBMC) in human umbilical cord blood were taken, and inoculated by tail vein injection into 4-5 week-old mice that were myeloablated with busulfan in advance, after 8 weeks of reconstitution of the human immune system, the orbital blood of the mice was collected to identify the proportion of humanized immune cells; when the proportion of human CD45-positive immune cells was 10% to 20%, after the NSG humanized mouse model was successfully constructed, the antibodies (3A3 chimeric antibody and 3A5 chimeric antibody, as well as the mixed antibody (3A3 Chimeric antibody+3A5 chimeric antibody)), and the control antibody VRC01 (which was HIV gp120-specific human monoclonal antibody, the VRC01 VH and VL amino acid sequences were set forth in SEQ ID NOs: 32 and 33, respectively, see: Rational design of envelope identifies broadly neutralizing human monoclonal antibody to HIV-1)) were injected into the abdominal cavity of mice of each group at a dose of 400 μg per mouse, respectively, 6 mice in each group; 24 hours after antibody injection, 100 μL of 25000 green Raji units (GRUs) dose of AKATA-derived Epstein-Barr virus (its preparation method was referred to the literature: Development of a robust, higher throughput green fluorescent protein (GFP)-based Epstein-Barr Virus (EBV) micro-neutralization assay) was injected through tail vein; after the virus was injected, the antibody was injected intraperitoneally into the mice at a dose of 20 mg/kg per mouse every other week, and the injection was continued for 4 weeks. Orbital blood of the mice was collected weekly to detect the changes in immune cells and virus copy numbers in peripheral blood, and to monitor body weight. The NSG mice were euthanized 7 weeks after the virus injection, and the changes in spleen size and the presence of cancerous tissue were observed. The EB virus DNA copy number in the collected mouse peripheral blood was determined by real-time fluorescent quantitative PCR using EB virus BALF5 gene-specific primers; and whether spleen cells or tumor cells were virus positive was determined by performing EBER in situ hybridization detection on mouse spleen tissue sections using commercial kits.

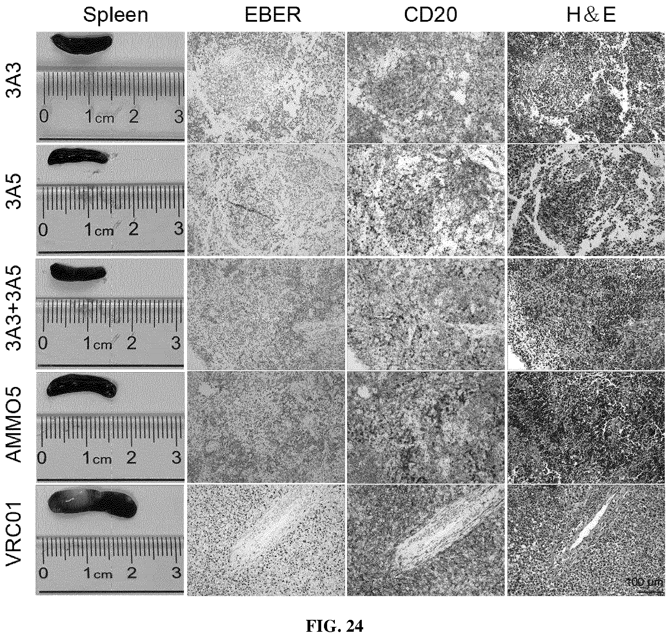

The results were shown in Figs. 23 and 24, wherein Fig. 23 showed the protection effects of 3A3 chimeric antibody, 3A5 chimeric antibody, the mixed antibody (3A3 chimeric antibody + 3A5 chimeric antibody), AMMOS and the control antibody VRC01 in the humanized mouse model. Wherein, Fig. 23A showed the body weight change of mice in each antibody treatment group; Fig. 23B showed the survival rate of mice in each antibody treatment group; Fig. 23C showed the changes in the proportion of hCD19+B cells in peripheral blood of mice in each antibody treatment group; Fig. 23D showed the changes in the proportion of hCD3+hhCD4+ double-positive T cells in peripheral blood of mice in each antibody treatment group; Fig. 23E showed the changes in the proportion of hCD3+hCD8+ double-positive T cells in peripheral blood of mice in each antibody treatment group; Fig. 23F showed the EBV DNA copy number in peripheral blood of mice in each antibody treatment group; Fig. 23G showed the proportion of hCD9+ B cells in spleen of mice after euthanasia in each antibody treatment group; Fig. 23H showed the proportion of hCD3+hCD4+ double-positive T cells in spleen of mice after euthanasia in each antibody treatment group; Fig. 23I showed the proportion of hCD3+hCD8+ double-positive T cells in spleen of mice after euthanasia in each antibody treatment group; Fig. 23J showed the EBV DNA copy number in spleen of mice after euthanasia in each antibody treatment group; and Fig. 23K showed the comparison of spleen weights of mice after euthanasia in each antibody treatment group.

Fig. 24 showed the results of EBER in situ hybridization detection, hCD20 staining and H&E staining of spleen tissue sections of mice after euthanasia in each antibody treatment group.

It could be seen from Figs. 23 and 24 that the 3A3 chimeric antibody, 3A5 chimeric antibody, and the mixed antibody (3A3 chimeric antibody + 3A5 chimeric antibody) could effectively prevent the viremia caused by virus replication in humanized mice, the changes in proportions of hCD19+ B cells and hCD3+hCD8+ double-positive T cells, and protect the mice from death caused by virus infection. The above results showed that the 3A3 chimeric antibody, 3A5 chimeric antibody, and the mixed antibody (3A3 chimeric antibody + 3A5 chimeric antibody) had a good effect of blocking virus infection in humanized mice.

Although the specific implementation of the present invention has been described in detail, those skilled in the art will understand that: according to all the teachings that have been disclosed, various modifications and changes can be made to the details, and these changes are all within the protection scope of the present invention. The full scope of the present invention is given by the claims appended hereto and any equivalents thereof.

**Claims**

1. An antibody or antigen-binding fragment thereof, which specifically binds to an epitope comprised in amino acid residues at positions 341-362 of an EBV gB protein, wherein the epitope comprises at least amino acid residues at positions 352, 356 and 360 of the EBV gB protein;

preferably, the epitope is a linear epitope, which consists of at least 3, at least 4, at least 5, at least 6, at least 7, at least 8, at least 9, at least 10, at least 15 or at least 20 consecutive amino acid residues located within the amino acid residues at positions 341-362 of EBV gB protein;

preferably, the epitope is a conformational epitope, which consists of at least 3, at least 4, at least 5, at least 6, at least 7, at least 8, at least 9, at least 10, at least 15 or at least 20 non-consecutive amino acid residues located within the amino acid residues at positions 341-362 of EBV gB protein, and comprises at least amino acid residues at positions 352, 356 and 360 of EBV gB protein;

preferably, the epitope consists of the amino acid residues at positions 341-362 of EBV gB protein.

2. An antibody or antigen-binding fragment thereof, which specifically binds to a gB protein of EBV, wherein the antibody or antigen-binding fragment thereof comprises:

(a) a heavy chain variable region (VH) comprising the following 3 complementarity determining regions (CDRs):

(i) a VH CDR1, which consists of the following sequence: SEQ ID NO: 3, or a sequence having a substitution, deletion or addition of one or several amino acids (e.g., a substitution, deletion or addition of 1, 2 or 3 amino acids) as compared thereto,
(ii) a VH CDR2, which consists of the following sequence: SEQ ID NO: 4, or a sequence having a substitution, deletion or addition of one or several amino acids (e.g., a substitution, deletion or addition of 1, 2 or 3 amino acids) as compared thereto, and
(iii) a VH CDR3, which consists of the following sequence: SEQ ID NO: 5, or a sequence having a substitution, deletion or addition of one or several amino acids (e.g., a substitution, deletion or addition of 1, 2 or 3 amino acids) as compared thereto;
and/or,

(b) a light chain variable region (VL) comprising the following 3 complementarity determining regions (CDRs):

(iv) a VL CDR1, which consists of the following sequence: SEQ ID NO: 6, or a sequence having a substitution, deletion or addition of one or several amino acids (e.g., a substitution, deletion or addition of 1, 2 or 3 amino acids) as compared thereto,
(v) a VL CDR2, which consists of the following sequence: SEQ ID NO: 7, or a sequence having a substitution, deletion or addition of one or several amino acids (e.g., a substitution, deletion or addition of 1, 2 or 3 amino acids) as compared thereto, and
(vi) a VL CDR3, which consists of the following sequence: SEQ ID NO: 8, or a sequence having a substitution, deletion or addition of one or several amino acids (e.g., a substitution, deletion or addition of 1, 2 or 3 amino acids) as compared thereto;
preferably, the CDR described in any one of (i) to (vi) is defined according to the IMGT numbering system;
preferably, the substitution described in any one of (i) to (vi) is a conservative substitution.

3. The antibody or antigen-binding fragment thereof according to claim 1 or 2, comprising:

(a) the following 3 heavy chain CDRs: a VH CDR1 having a sequence as set forth in SEQ ID NO: 3, a VH CDR2 having a sequence as set forth in SEQ ID NO: 4, a VH CDR3 having a sequence as set forth in SEQ ID NO: 5; and/or, the following 3 light chain CDRs: a VL CDR1 having a sequence set forth in SEQ ID NO: 6, a VL CDR2 having a sequence set forth in SEQ ID NO: 7, and a VL CDR3 having a sequence set forth in SEQ ID NO: 8; or,
(b) the 3 CDRs comprised in the heavy chain variable region (VH) as set forth in SEQ ID NO: 1; and/or, the 3 CDRs comprised in the light chain variable region (VL) as set forth in SEQ ID NO: 2; preferably, the 3 CDRs comprised in the VH and/or the 3 CDRs comprised in the VL are defined by the Kabat, IMGT or Chothia numbering system.

4. The antibody or antigen-binding fragment thereof according to any one of claims 1-3, comprising:

(a) a heavy chain variable region (VH), which comprises an amino acid sequence selected from the group consisting of:

(i) a sequence set forth in SEQ ID NO: 1;
(ii) a sequence having a substitution, deletion or addition of one or several amino acids (e.g., a substitution, deletion or addition of 1, 2, 3, 4 or 5 amino acids) as compared to the sequence as set forth in SEQ ID NO: 1; or
(iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence as set forth in SEQ ID NO: 1;
and

(b) a light chain variable region (VL), which comprises an amino acid sequence selected from the group consisting of:

(iv) a sequence as set forth in SEQ ID NO: 2;
(v) a sequence having a substitution, deletion or addition of one or several amino acids (e.g., a substitution, deletion or addition of 1, 2, 3, 4 or 5 amino acids) as compared to the sequence as set forth in SEQ ID NO: 2; or
(vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence as set forth in SEQ ID NO: 2;
preferably, the substitution described in (ii) or (v) is a conservative substitution;
preferably, the antibody or antigen-binding fragment thereof comprises: a VH comprising the sequence set forth in SEQ ID NO: 1 and a VL comprising the sequence set forth in SEQ ID NO: 2.

5. An antibody or antigen-binding fragment thereof, which specifically binds to an epitope comprised in amino acid residues at positions 528-616 of an EBV gB protein, wherein the epitope comprises at least amino acid residues at positions 540, 567, 610 and 613 of the EBV gB protein;

preferably, the epitope is a linear epitope, which consists of at least 3, at least 4, at least 5, at least 6, at least 7, at least 8, at least 9, at least 10, at least 15 or at least 20 consecutive amino acid residues located within the amino acid residues at positions 528-616 of EBV gB protein;
preferably, the epitope is a conformational epitope, which consists of at least 3, at least 4, at least 5, at least 6, at least 7, at least Consists of 8, at least 9, at least 10, at least 15 or at least 20 non-consecutive amino acid residues located within the amino acid residues at positions 528-616 of EBV gB protein, and comprises at least amino acid residues at positions 540, 567, 610 and 613 of EBV gB protein;
preferably, the epitope consists of amino acid residues at positions 528-616 of EBV gB protein.

6. An antibody or antigen-binding fragment thereof, which specifically binds to a gB protein of EBV, wherein the antibody or an antigen-binding fragment thereof comprises:

(a) a heavy chain variable region (VH) comprising the following 3 complementarity determining regions (CDRs):

(i) a VH CDR1, which consists of the following sequence: SEQ ID NO: 11, or a sequence having a substitution, deletion or addition of one or several amino acids (e.g., a substitution, deletion or addition of 1, 2 or 3 amino acids) as compared thereto,
(ii) a VH CDR2, which consists of the following sequence: SEQ ID NO: 12, or a sequence having a substitution, deletion or addition of one or several amino acids (e.g., a substitution, deletion or addition of 1, 2 or 3 amino acids) as compared thereto, and
(iii) a VH CDR3, which consists of the following sequence: SEQ ID NO: 13, or a sequence having a substitution, deletion or addition of one or several amino acids (e.g., a substitution, deletion or addition of 1, 2 or 3 amino acids) as compared thereto;
and/or,

(b) a light chain variable region (VL) comprising the following 3 complementarity determining regions (CDRs):

(iv) a VL CDR1, which consists of the following sequence: SEQ ID NO: 14, or a sequence having a substitution, deletion or addition of one or several amino acids (e.g., a substitution, deletion or addition of 1, 2 or 3 amino acids) as compared thereto,
(v) a VL CDR2, which consists of the following sequence: SEQ ID NO: 15, or a sequence having a substitution, deletion or addition of one or several amino acids (e.g., a substitution, deletion or addition of 1, 2 or 3 amino acids) as compared thereto, and
(vi) a VL CDR3, which consists of the following sequence: SEQ ID NO: 16, or a sequence having a substitution, deletion or addition of one or several amino acids (e.g., a substitution, deletion or addition of 1, 2 or 3 amino acids) as compared thereto;
preferably, the CDR described in any one of (i) to (vi) is defined according to the IMGT numbering system;
preferably, the substitution described in any one of (i) to (vi) is a conservative substitution.

7. The antibody or antigen-binding fragment thereof according to claim 5 or 6, comprising:

(a) the following 3 heavy chain CDRs: a VH CDR1 having a sequence as set forth in SEQ ID NO: 11, a VH CDR2 having a sequence as set forth in SEQ ID NO: 12, a VH CDR3 having a sequence as set forth in SEQ ID NO: 13; and/or, the following 3 light chain CDRs: a VL CDR1 having a sequence as set forth in SEQ ID NO: 14, a VL CDR2 having a sequence as set forth in SEQ ID NO: 15, and a VL CDR3 having a sequence as set forth in SEQ ID NO: 16;
or,
(b) the 3 CDRs comprised in the heavy chain variable region (VH) as set forth in SEQ ID NO: 9; and/or, the 3 CDRs comprised in the light chain variable region (VL) as set forth in SEQ ID NO: 10; preferably, the 3 CDRs comprised in the VH and/or the 3 CDRs comprised in the VL are defined by the Kabat, IMGT or Chothia numbering system.

8. The antibody or antigen-binding fragment thereof according to any one of claims 5-7, comprising:

(a) a heavy chain variable region (VH) comprising an amino acid sequence selected from the group consisting of:

(i) a sequence set forth in SEQ ID NO: 9;
(ii) a sequence having a substitution, deletion or addition of one or several amino acids (e.g., a substitution, deletion or addition of 1, 2, 3, 4 or 5 amino acids) as compared to the sequence as set forth in SEQ ID NO: 9; or
(iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence as set forth in SEQ ID NO: 9;
and

(b) a light chain variable region (VL) comprising an amino acid sequence selected from the group consisting of:

(iv) a sequence set forth in SEQ ID NO: 10;
(v) a sequence having a substitution, deletion or addition of one or several amino acids (e.g., a substitution, deletion or addition of 1, 2, 3, 4 or 5 amino acids) as compared to the sequence as set forth in SEQ ID NO: 10; or
(vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence as set forth in SEQ ID NO: 10;
preferably, the substitution described in (ii) or (v) is a conservative substitution;
preferably, the antibody or antigen-binding fragment thereof comprises: a VH comprising the sequence set forth in SEQ ID NO: 9 and a VL comprising the sequence set forth in SEQ ID NO: 10.

9. The antibody or antigen-binding fragment thereof according to any one of claims 1-8, which is humanized;

preferably, the antibody or antigen-binding fragment thereof comprises a framework region sequence derived from a human immunoglobulin;
preferably, the antibody or antigen-binding fragment thereof comprises: a heavy chain framework region sequence derived from a human heavy chain germline sequence, and a light chain framework region sequence derived from a human light chain germline sequence.

10. The antibody or antigen-binding fragment thereof according to any one of claims 1-9, which further comprises a constant region derived from a rabbit or human immunoglobulin;

preferably, the heavy chain of the antibody or antigen-binding fragment thereof comprises a heavy chain constant region derived from a human immunoglobulin (e.g., IgG1, IgG2, IgG3 or IgG4) and the light chain of the antibody or antigen-binding fragment thereof comprises a light chain constant region derived from a human immunoglobulin (e.g., κ or λ);
preferably, the heavy chain of the antibody or antigen-binding fragment thereof comprises a heavy chain constant region as set forth in SEQ ID NO: 30, and the light chain of the antibody or antigen-binding fragment thereof comprises a light chain constant region as set forth in SEQ ID NO: 31.

11. The antibody or antigen-binding fragment thereof according to any one of claims 1-10, wherein the antigen-binding fragment is selected from the group consisting of Fab, Fab', (Fab')$_2$, Fv, disulfide-bonded Fv, scFv, diabody and single domain antibody (sdAb); and/or, the antibody is a rabbit antibody, a chimeric antibody, a humanized antibody, a bispecific antibody or a multispecific antibody.

12. The antibody or antigen-binding fragment thereof according to any one of claims 1-11, wherein the antibody or antigen-binding fragment thereof has one or more of the following characteristics:

(a) neutralizing EBV in vitro or in a subject (e.g., a human);
(b) blocking or inhibiting EBV from fusion with a cell in vitro or in a subject (e.g., a human);
(c) preventing and/or treating an EBV infection or a disease associated with EBV infection.

13. An isolated nucleic acid molecule, which encodes the antibody or antigen-binding fragment thereof according to any one of claims 1-12, or a heavy chain variable region and/or a light chain variable region thereof.

14. A vector, which comprises the nucleic acid molecule according to claim 13; preferably, the vector is a cloning vector or an expression vector.

15. A host cell, which comprises the nucleic acid molecule according to claim 13 or the vector according to claim 14.

16. A method for preparing the antibody or antigen-binding fragment thereof according to any one of claims 1-12, comprising culturing the host cell according to claim 15 under conditions that allow the expression of the antibody or antigen-binding fragment thereof, and recovering the antibody or antigen-binding fragment thereof from a culture of the cultured host cell.

17. A composition, which comprises:

(i) the antibody or antigen-binding fragment thereof according to any one of claims 1-4, or a nucleic acid molecule, vector or host cell encoding the antibody or antigen-binding fragment thereof according to any one of claims 1-4; and,
(ii) the antibody or antigen-binding fragment thereof according to any one of claims 5-8, or a nucleic acid molecule, vector or host cell encoding the antibody or antigen-binding fragment thereof according to any one of claims 5-8;
preferably, the composition comprises the antibody or antigen-binding fragment thereof according to any one of claims 1-4, and the antibody or antigen-binding fragment thereof according to any one of claims 5-8;
preferably, the antibody in (i) is a chimeric antibody, and/or, the antibody in (ii) is a chimeric antibody.

18. A pharmaceutical composition, which comprises the antibody or antigen-binding fragment thereof according to any one of claims 1-12 or the composition according to claim 17, and optionally a pharmaceutically acceptable carrier and/or excipient.

19. Use of the antibody or antigen-binding fragment thereof according to any one of claims 1-12, the isolated nucleic acid molecule according to claim 13, the vector according to claim 14, the host cell according to claim 15, the composition according to claim 17, or the pharmaceutical composition according to claim 18 in the manufacture a medicament, wherein the medicament is used for neutralizing the virulence of EBV, or for inhibiting or blocking the fusion of EBV with a cell, or for preventing and/or treating an EBV infection or a disease associated with EBV infection in a subject;

preferably, the EBV infection is a chronic active EBV (CAEBV) infection or a primary EBV infection;
preferably, the disease associated with EBV infection is a mononucleosis or an EBV-associated cancer;
preferably, the EBV-associated cancer is selected from the group consisting of lymphoproliferative disorder (LPD) such as B-cell lymphoma including Burkitt lymphoma (BL), Hodgkin's lymphoma (HL), diffuse large B-cell lymphoma (DLBCL) or post-transplantation lymphoproliferative disorder (PTLD), or epithelial (nasopharyngeal, lung, breast) carcinoma, lymphoepithelioma, carcinoma with lymphoid stroma (GCLS, such as gastric cancer), or glioma;

preferably, the subject is a mammal, such as a human;
preferably, the antibody or antigen-binding fragment thereof is used alone, or in combination with an additional pharmaceutically active agent.

20. A method for preventing and/or treating an EBV infection or a disease associated with EBV infection in a subject (e.g., a human), comprising: administering to the subject in need thereof an effective amount of the antibody or antigen-binding fragment thereof according to any one of claims 1-12, the isolated nucleic acid molecule according to claim 13, the vector according to claim 14, the host cell according to claim 15, the composition according to claim 17, or the pharmaceutical composition according to claim 18.

21. A conjugate, which comprises the antibody or antigen-binding fragment thereof according to any one of claims 1-12, and a detectable label linked to the antibody or antigen-binding fragment thereof;
preferably, the detectable label is selected from the group consisting of enzyme (e.g., horseradish peroxidase or alkaline phosphatase), chemiluminescence reagent (e.g., acridinium ester, luminol and derivative thereof, or ruthenium derivative), fluorescent dye (e.g., fluorescein or fluorescent protein), radionuclide, or biotin.

22. A kit, which comprises the antibody or antigen-binding fragment thereof according to any one of claims 1-12 or the conjugate according to claim 21;

preferably, the kit comprises the conjugate according to claim 21;
preferably, the kit comprises the antibody or antigen-binding fragment thereof according to any one of claims 1-12, and a second antibody that specifically recognizes the antibody or antigen-binding fragment thereof;
optionally, the second antibody further comprises a detectable label such as enzyme (e.g., horseradish peroxidase or alkaline phosphatase), chemiluminescence reagent (e.g., acridinium ester, luminol and derivative thereof, or ruthenium derivative), fluorescent dye (e.g., fluorescein or fluorescent protein), radionuclide, or biotin.

23. A method for detecting the presence or level of EBV in a sample, which comprises using the antibody or antigen-binding fragment thereof according to any one of claims 1-12 or the conjugate according to claim 21;

preferably, the method is an immunological assay, such as western blot, enzyme immunoassay (e.g., ELISA), chemiluminescence immunoassay, fluorescence immunoassay or radioimmunoassay;
preferably, the method comprises using the conjugate according to claim 21;
preferably, the method comprises using the antibody or antigen-binding fragment thereof according to any one of claims 1-12, and the method further comprises using a second antibody carrying a detectable label (e.g., enzyme (e.g., horseradish peroxidase or alkaline phosphatase), chemiluminescence reagent (e.g., acridinium ester, luminol and derivative thereof, or ruthenium derivative), fluorescent dye (e.g., fluorescein or fluorescent protein), radionuclide or biotin) to detect the antibody or antigen-binding fragment thereof.

24. Use of the antibody or antigen-binding fragment thereof according to any one of claims 1-12 or the conjugate according to claim 21 in the manufacture of a detection reagent, wherein the detection reagent is used for detecting the presence or level of EBV in a sample, and/or for diagnosing whether a subject is infected with EBV;

preferably, the detection reagent detects the presence or level of EBV in the sample by the method according to claim 23;
preferably, the sample is a bodily fluid sample (e.g., whole blood, plasma, serum, salivary excretion or urine) from a subject (e.g., a mammal, preferably a human).

25. An isolated epitope peptide or variant thereof, wherein the epitope peptide comprises an epitope located within amino acid residues at positions 341-362 of an EBV gB protein, the epitope comprises at least amino acid residues at positions 352, 356 and 360 of EBV gB protein; the variant differs from the epitope peptide from which it is derived only by a mutation (e.g., substitution, addition or deletion) of one or several (e.g., 1, 2 or 3) amino acid residues, and does not comprise a mutation at the positions corresponding to amino acid positions 352, 356 and 360 of EBV gB protein, and retains a biological function of the epitope peptide from which it is derived;

preferably, the epitope peptide or variant thereof can be specifically bound by the antibody or antigen-binding fragment thereof according to any one of claims 1-4;
preferably, the amino acid residues at positions 341-362 of EBV gB protein are set forth in SEQ ID NO: 18;
preferably, the EBV gB protein has a sequence set forth in SEQ ID NO: 17.

**26.** The isolated epitope peptide or variant thereof according to claim 25, wherein the epitope is a linear epitope, which consists of at least 3, at least 4, at least 5, at least 6, at least 7, at least 8, at least 9, at least 10, at least 15 or at least 20 consecutive amino acid residues located within the amino acid residues at positions 341-362 of EBV gB protein;

preferably, the linear epitope consists of the amino acid residues at positions 341-362 of EBV gB protein;
preferably, the epitope peptide consists of 5-50 (e.g., 10-50, 10-40, 20-40; such as 20-25 or 35-40; such as 20, 21, 22, 23, 24, 25, 26, 27, 28, 30, 31, 32, 33, 34, 35, 36, 37, 38, 39, or 40) consecutive amino acid residues of EBV gB protein, and comprises the linear epitope;
preferably, the epitope peptide consists of the amino acid residues at positions 341-362 or amino acid residues at positions 331-367 of EBV gB protein;
preferably, the epitope peptide consists of the sequence set forth in SEQ ID NO:18 or 19.

**27.** The epitope peptide or variant thereof according to claim 25, wherein the epitope is a conformational epitope, which consists of at least 3, at least 4, at least 5, at least 6, at least 7, at least 8, at least 9, at least 10, at least 15 or at least 20 non-consecutive amino acid residues located within the amino acid residues at positions 341-362 of EBV gB protein, and comprises at least amino acid residues at positions 352, 356 and 360 of EBV gB protein;
preferably, the epitope peptide consists of 5-50 (e.g., 10-50, 10-40, 20-40; such as 20-25 or 35-40; such as 20, 21, 22, 23, 24, 25, 26, 27, 28, 30, 31, 32, 33, 34, 35, 36, 37, 38, 39, or 40) consecutive amino acid residues of EBV gB protein, and comprises the conformational epitope.

**28.** An isolated epitope peptide or variant thereof, wherein the epitope peptide comprises an epitope located within amino acid residues at positions 528-616 of an EBV gB protein, the epitope comprises at least amino acid residues at positions 540, 567, 610 and 613 of EBV gB protein; the variant differs from the epitope peptide from which it is derived only by a mutation (e.g., substitution, addition or deletion) of one or several (e.g., 1, 2 or 3) amino acid residues, and does not comprise a mutation at the positions corresponding to amino acid positions 540, 567, 610 and 613 of EBV gB protein, and retains a biological function of the epitope peptide from which it is derived;

preferably, the epitope peptide or variant thereof can be specifically bound by the antibody or antigen-binding fragment thereof according to any one of claims 5-8;
preferably, the amino acid residues at positions 528-616 of EBV gB protein are set forth in SEQ ID NO: 20;
preferably, the EBV gB protein has a sequence set forth in SEQ ID NO: 17.

**29.** The isolated epitope peptide or variant thereof according to claim 28, wherein the epitope is a linear epitope, which consists of at least 3, at least 4, at least 5, at least 6, at least 7, at least 8, at least 9, at least 10, at least 15 or at least 20 consecutive amino acid residues located within the amino acid residues at positions 528-616 of EBV gB protein;

preferably, the linear epitope consists of the amino acid residues at positions 528-616 of EBV gB protein;
preferably, the epitope peptide consists of 5-100 (e.g., 10-100, 10-90, 20-90; for example 5-10, 10-20, 20-30, 30-40, 40-50, 50-60, 60-70, 70-80, 80-90) consecutive amino acid residues of EBV gB protein, and comprises the linear epitope;
preferably, the epitope peptide consists of the amino acid residues at positions 528-616 of EBV gB protein or a fragment thereof;
preferably, the epitope peptide consists of the sequence set forth in SEQ ID NO: 20 or a fragment thereof.

**30.** The epitope peptide or variant thereof according to claim 28, wherein the epitope is a conformational epitope, which consists of at least 3, at least 4, at least 5, at least 6, at least 7, at least 8, at least 9, at least 10, at least 15 or at least 20 non-consecutive amino acid residues within the amino acid residues at positions 528-616 of EBV gB protein, and comprises at least amino acid residues at positions 540, 567, 610 and 613 of EBV gB protein;
preferably, the epitope peptide consists of 5-100 (e.g., 10-100, 10-90, 20-90; for example 5-10, 10-20, 20-30, 30-40, 40-50, 50-60, 60-70, 70-80, 80-90) consecutive amino acid residues of EBV gB protein, and comprises the conformational epitope.

**31.** A recombinant protein, which comprises the isolated epitope peptide or variant thereof according to any one of claims 25-30, and a carrier protein, wherein the recombinant protein is not a naturally occurring protein or fragment thereof,
preferably, the epitope peptide or variant thereof is linked to the carrier protein, optionally via a linker.

**32.** The recombinant protein according to claim 31, which is capable of displaying the isolated epitope peptide or variant thereof according to any one of claims 25-27, wherein the epitope peptide or variant thereof is capable of being specifically bound by the antibody or antigen-binding fragment thereof according to any one of claims 1-4.

**33.** The recombinant protein according to claim 31, which is capable of displaying the isolated epitope peptide or variant thereof according to any one of claims 28-30, wherein the epitope peptide or variant thereof is capable of being specifically bound by the antibody or antigen-binding fragment thereof according to any one of claims 5-8.

**34.** The recombinant protein according to any one of claims 31-33, which possesses one or more of the following characteristics:

(a) inducing an antiserum capable of neutralizing EBV, and/or blocking or inhibiting the fusion of EBV with a cell in a subject;
(b) inducing an antibody response effective in clearing EBV and an EBV-infected cell in vivo;
(c) preventing and/or treating an EBV infection or a disease associated with EBV infection in a subject.

**35.** An isolated nucleic acid molecule, which comprises a nucleotide sequence encoding the epitope peptide or variant thereof according to any one of claims 25-30, or the recombinant protein according to any one of claims 31-34.

**36.** A vector, which comprises the isolated nucleic acid molecule according to claim 35.

**37.** A host cell, which comprises the isolated nucleic acid molecule according to claim 35 or the vector according to claim 36.

**38.** A method for preparing the epitope peptide or variant thereof according to any one of claims 25-30 or the recombinant protein according to any one of claims 31-34, comprising culturing the host cell according to claim 37 under suitable conditions, and recovering the epitope peptide or variant thereof or the recombinant protein from a cell culture.

**39.** A particle, displaying on its surface the isolated epitope peptide or variant thereof according to any one of claims 25-30; preferably, the particle is a virus-like particle (VLP).

**40.** An immunogenic composition, which comprises the epitope peptide or variant thereof according to any one of claims 25-30, or the recombinant protein according to any one of claims 31-34 or the particle according to claim 39, and optionally a pharmaceutically acceptable carrier and/or excipient (e.g., adjuvant); preferably, the immunogenic composition is a vaccine.

**41.** Use of the epitope peptide or variant thereof according to any one of claims 25-30, or the recombinant protein according to any one of claims 31-34, or the isolated nucleic acid molecule according to claim 35, or the vector according to claim 36, or the host cell according to claim 37 or the particle according to claim 39, or the immunogenic composition according to claim 40 in the manufacture of an immunogenic composition, wherein the immunogenic composition is used for inducing an immune response against EBV in a subject and/or for preventing and/or treating an EBV infection or a disease associated with EBV infection in a subject;

preferably, the immunogenic composition is a vaccine;
preferably, the EBV infection is a chronic active EBV (CAEBV) infection or a primary EBV infection;
preferably, the disease associated with EBV infection is a mononucleosis or an EBV-associated cancer;
preferably, the EBV-associated cancer is selected from the group consisting of lymphoproliferative disorder (LPD) such as B-cell lymphoma including Burkitt lymphoma (BL), Hodgkin's lymphoma (HL), diffuse large B-cell lymphoma (DLBCL) or post-transplantation lymphoproliferative disorder (PTLD), or epithelial (nasopharyngeal, lung, breast) carcinoma, lymphoepithelioma, carcinoma with lymphoid stroma (GCLS, such as gastric cancer), or glioma;
preferably, the subject is a mammal, such as a human.

**42.** A method for inducing an immune response against EBV in a subject and/or for preventing and/or treating an EBV infection or a disease associated with EBV infection in a subject (e.g., a human), which comprises: administering to the subject in need an effective amount of the epitope peptide or variant thereof according to any one of claims 25-30, or the recombinant protein according to any one of claims 31-34, or the isolated nucleic acid molecule according to claim 35, or the vector according to claim 36, or the host cell according to claim 37 or the particle according to claim 39, or the immunogenic composition according to claim 40;

preferably, the EBV infection is a chronic active EBV (CAEBV) infection or a primary EBV infection;
preferably, the disease associated with EBV infection is a mononucleosis or an EBV-associated cancer;
preferably, the EBV-associated cancer is selected from the group consisting of lymphoproliferative disorder (LPD) such as B-cell lymphoma including Burkitt lymphoma (BL), Hodgkin's lymphoma (HL), diffuse large B-cell lymphoma (DLBCL) or post-transplantation lymphoproliferative disorder (PTLD), or epithelial (nasopharyngeal, lung, breast) carcinoma, lymphoepithelioma, carcinoma with lymphoid stroma (GCLS, such as gastric cancer), or glioma;
preferably, the subject is a mammal, such as a human.

**FIG. 1**

**FIG. 2**

**FIG. 3**

**FIG. 4**

**FIG. 5**

**FIG. 6**

Epithelial cells (HNE1)

3A3 [IC50=0.07]
3A5 [IC50=0.31]
AMMO5 [IC50=0.13]
1E12 [NA]

Conecentration of mAb (µg/ml)

B cells (AKATA)

3A3 [IC50=2.97]
3A5 [IC50=4.90]
AMMO5 [IC50=36.60]
1E12 [NA]

Conecentration of mAb (µg/ml)

**FIG. 7**

■100 µg ■20 µg ■4 µg ■0.8 µg

RLU

3A3
3A5
AMMO5
1E12
Cell (gB, gL, gH, T7)+ Cell (Luc)
Cell (Luc)
Cell (gB, gL, gH, T7)

**FIG. 8**

FIG. 9

**FIG. 10**

**FIG. 11**

**FIG. 12**

**FIG. 13**

**FIG. 14**

**FIG. 15**

## Binding of mAb to Virus-like Particle

**FIG. 16**

**FIG. 17**

149-WT        149-αB

**FIG. 18**

**FIG. 19**

**FIG. 20**

**FIG. 21**

**FIG. 22**

**FIG. 23**

**FIG. 24**

<div align="center">**INTERNATIONAL SEARCH REPORT**</div>

| International application No. |
| --- |
| **PCT/CN2022/074985** |

| A. | CLASSIFICATION OF SUBJECT MATTER |
| --- | --- |

C07K 14/05(2006.01)i; C07K 16/08(2006.01)i; A61K 39/395(2006.01)i; A61K 39/245(2006.01)i

According to International Patent Classification (IPC) or to both national classification and IPC

| B. | FIELDS SEARCHED |
| --- | --- |

Minimum documentation searched (classification system followed by classification symbols)

C07K; A61K

Documentation searched other than minimum documentation to the extent that such documents are included in the fields searched

Electronic data base consulted during the international search (name of data base and, where practicable, search terms used)

CNTXT, DWPI, CNKI, 万方, WANFANG, NCBI, ISI web of knowledge, 百度, BAIDU, 必应, Bing: 抗体, EB病毒, EBV, Epstein-Barr Virus, GB, gp125, gp110, BALF4, 表位, epitope, 包膜, 糖蛋白b, 疱疹病毒, hepesvirus, HSV

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | CN 111093691 A (NEON THERAPEUTICS INC.) 01 May 2020 (2020-05-01) claims 1-51, and description, p. 69, line 12, and p. 71, line 38 and line 40 | 1, 5, 9-42 |
| A | US 2020164059 A1 (FRED HUTCHINSON CANCER RESEARCH CENTER) 28 May 2020 (2020-05-28) figures 7-9 | 1-42 |
| X | CN 107163109 A (QINGDAO UNIVERSITY) 15 September 2017 (2017-09-15) claims 1-10, and embodiments 1 and 2 | 1, 5, 9-42 |
| X | 胡波等 (HU, Bo et al.). "EB病毒gp125蛋白的B细胞表位预测 (Prediction of B Cell Epitopes of the gp125 Protein in Epstein-Barr Virus)" 中国微生态学杂志 (Chinese Journal of Microecology), Vol. 26, No. 4, 30 April 2014 (2014-04-30), pp. 404-408 | 1, 5, 9-42 |
| X | 袁青等 (YUAN, Qing et al.). "EB病毒LMP-1、BALF4及BARF1片段的B细胞表位预测 (Prediction of the B-Cell Epitope for the LMP-1, BALF4 and BARF1 of Epstein-Barr Virus)" 中国卫生检验杂志 (Chinese Journal of Health Laboratory Technology), Vol. 22, No. 7, 31 July 2012 (2012-07-31), pp. 1567-1570 | 1, 5, 9-42 |

☑ Further documents are listed in the continuation of Box C. ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **07 April 2022** | **27 April 2022** |

| Name and mailing address of the ISA/CN | Authorized officer |
| --- | --- |
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT

| International application No. |
|---|
| **PCT/CN2022/074985** |

### C.     DOCUMENTS CONSIDERED TO BE RELEVANT

| Category* | Citation of document, with indication, where appropriate, of the relevant passages | Relevant to claim No. |
|---|---|---|
| X | MILOSEVIC, S. et al. "Identification of Major Histocompatibility Complex Class II-Restricted Antigens and Epitopes of the Epstein-Barr Virus by a Novel Bacterial Expression Cloning Approach" <br> *JOURNAL OF VIROLOGY*, Vol. 80, No. 21, 30 November 2006 (2006-11-30), <br> pp. 10357-10364 | 1, 5, 9-42 |
| A | CHEN, Jia et al. "The Epstein-Barr Virus (EBV) Glycoprotein B Cytoplasmic C-Terminal Tail Domain Regulates the Energy Requirement for EBV-Induced Membrane Fusion" <br> *JOURNAL OF VIROLOGY*, Vol. 88, No. 20, 31 October 2014 (2014-10-31), <br> pp. 11686-11695 | 1-42 |

Form PCT/ISA/210 (second sheet) (January 2015)

<table>
<tr><td>**INTERNATIONAL SEARCH REPORT**</td><td>International application No.<br>**PCT/CN2022/074985**</td></tr>
</table>

**Box No. I        Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

 a.  ☑ forming part of the international application as filed:

 ☑ in the form of an Annex C/ST.25 text file.

 ☐ on paper or in the form of an image file.

 b.  ☐ furnished together with the international application under PCT Rule 13*ter*.1(a) for the purposes of international search only in the form of an Annex C/ST.25 text file.

 c.  ☐ furnished subsequent to the international filing date for the purposes of international search only:

 ☐ in the form of an Annex C/ST.25 text file (Rule 13*ter*.1(a)).

 ☐ on paper or in the form of an image file (Rule 13*ter*.1(b) and Administrative Instructions, Section 713).

2.  ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

# EP 4 293 038 A1

**INTERNATIONAL SEARCH REPORT**

International application No.

**PCT/CN2022/074985**

| Box No. II | Observations where certain claims were found unsearchable (Continuation of item 2 of first sheet) |
|---|---|

This international search report has not been established in respect of certain claims under Article 17(2)(a) for the following reasons:

1. ☑ Claims Nos.: **20,42**
   because they relate to subject matter not required to be searched by this Authority, namely:

   [1] Claims 20 and 42 comprise a method for treating a living human or animal body, and therefore do not comply with PCT Rule 39.1(iv). The present report is formed on the basis of a pharmaceutical use of the antibody or antigen-binding fragment thereof, a nucleic acid molecule, a vector, a host cell, a composition, or a pharmaceutical composition, an epitope peptide or a variant thereof, a recombinant protein, an ion, etc. in claims 20 and 42.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (January 2015)

**INTERNATIONAL SEARCH REPORT**
Information on patent family members

International application No.

**PCT/CN2022/074985**

| Patent document cited in search report | | | Publication date (day/month/year) | Patent family member(s) | | | Publication date (day/month/year) |
|---|---|---|---|---|---|---|---|
| CN | 111093691 | A | 01 May 2020 | JP | 2020515640 | A | 28 May 2020 |
| | | | | CA | 3058807 | A1 | 11 October 2018 |
| | | | | EP | 3606550 | A2 | 12 February 2020 |
| | | | | WO | 2018187356 | A2 | 11 October 2018 |
| US | 2020164059 | A1 | 28 May 2020 | WO | 2018209125 | A1 | 15 November 2018 |
| CN | 107163109 | A | 15 September 2017 | | None | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4816567 P, Cabilly
- US 4816567 A, Cabilly
- US 5225539 A, Winter
- US 5530101 A, Queen
- US 5585089 A
- US 5693762 A
- US 6180370 B

### Non-patent literature cited in the description

- Construction of Peptide Display Vector Based on HBV Core Protein. **YANG HAIJIE et al.** Journal of Xiamen University. Natural Science, May 2004, vol. 43
- **MORIMOTO et al.** *J. Biochem. Biophys. Methods,* 1992, vol. 24, 107-117
- **BRENNAN et al.** *Science,* 1985, vol. 229 (81
- **HUDSON.** *Curr. Opin. Immunol.,* 1999, vol. 11, 548-557
- **LITTLE et al.** *Immunol. Today,* 2000, vol. 21, 364-370
- **CARTER et al.** *Bio/Technology,* 1992, vol. 10, 163-167
- **BRENNAN et al.** *Science,* 1985, vol. 229, 81
- **SLUPETZKY, K. et al.** *J Gen Virol,* 2001, vol. 82, 2799-2804
- **VARSANI, A. et al.** *J Virol,* 2003, vol. 77, 8386-8393
- **KOLETZKI, D. et al.** *Biol Chem,* 1999, vol. 380, 325-333
- **KÖNIG.** *J. Virol.,* 1998, vol. 72 (6), 4997
- **KABAT.** Sequences of Proteins of Immunological Interest. National Institutes of Health, 1987
- **CHOTHIA ; LESK.** *J. Mol. Biol.,* 1987, vol. 196, 901-917
- **CHOTHIA et al.** *Nature,* 1989, vol. 342, 878-883
- **KABAT et al.** Sequences of Proteins of Immunological Interest. Public Health Service, National Institutes of Health, 1991
- **LEFRANC et al.** *Dev. Comparat. Immunol.,* 2003, vol. 27, 55-77
- **PAUL, W.** Fundamental Immunology. Raven Press, 1989
- **HOLLIGER et al.** *Nat Biotechnol,* 2005, vol. 23, 1126-1136
- **WARD et al.** *Nature,* 1989, vol. 341, 544-546
- **BIRD et al.** *Science,* 1988, vol. 242, 423-426
- **HUSTON et al.** *Proc. Natl. Acad. Sci. USA,* 1988, vol. 85, 5879-5883
- **PLUCKTHUN.** The Pharmacology of Monoclonal antibody. Springer-Verlag, 1994, vol. 113, 269-315
- **HOLLIGER et al.** *Proc. Natl. Acad. Sci. USA,* 1993, vol. 90, 6444-6448
- **ALFTHAN et al.** *Protein Eng.,* 1995, vol. 8, 725-731
- **CHOI et al.** *Eur. J. Immunol.,* 2001, vol. 31, 94-106
- **HU et al.** *Cancer Res.,* 1996, vol. 56, 3055-3061
- **KIPRIYANOV et al.** *J. Mol. Biol.,* 1999, vol. 293, 41-56
- **ROOVERS et al.** *Cancer Immunol,* 2001
- **HOLLIGER P. et al.** *Proc. Natl. Acad. Sci. USA,* 1993, vol. 90, 6444-6448
- **POLJAK R. J. et al.** *Structure,* 1994, vol. 2, 1121-1123
- **MORRISON et al.** *Proc. Natl. Acad. Sci. USA,* 1984, vol. 81, 6851-6855
- **KABAT, E.A. et al.** Sequences of Proteins of Immunological Interest. NIH Publication, 1991, 3242
- Antibody Engineering: Methods and Protocols. Humana Press, 2004, vol. 248
- **NEEDLEMAN et al.** *J. Mol. Biol.,* 1970, vol. 48, 443-453
- **E. MEYERS ; W. MILLER.** *Comput. Appl Biosci.,* 1988, vol. 4, 11-17
- **NEEDLEMAN ; WUNSCH.** *J Mol Biol.,* 1970, vol. 48, 444-453
- **BRUMMELL et al.** *Biochem.,* 1993, vol. 32, 1180-1187
- **KOBAYASHI et al.** *Protein Eng.,* 1999, vol. 12 (10), 879-884
- **BURKS et al.** *Proc. Natl Acad. Set USA,* 1997, vol. 94, 412-417
- Immunology-A Synthesis. Sinauer Associates, 1991
- Remington's Pharmaceutical Sciences. Pennsylvania: Mack Publishing Company, 1995

- **J. SAMBROOK et al.** Molecular Cloning: A Laboratory Manual. Cold Spring Harbor Laboratory Press, 1989
- **F. M. AUSUBEL et al.** Molecular Biology Experimental Guide. John Wiley & Sons, Inc, 1995
- **ED HARLOW et al.** antibody A Laboratory Manual. Cold Spring Harbor Laboratory, 1988
- *Immunity,* 2018, vol. 48, 799-811.e9
- *Proc Natl Acad Sci USA,* 1980, vol. 77, 2979-83
- *Theranostics.,* 27 April 2020, vol. 10 (13), 5704-5718